# EUROPEAN PATENT SPECIFICATION

(11) **EP 2 707 021 B1**
(45) Date of publication and mention of the grant of the patent: **21.02.2024**
(21) Application number: 12782984.4
(22) Date of filing: 08.05.2012
(51) Int. Cl.: C07K 14/475, C12N 15/00, C12N 15/62, A61K 38/18

(54) **GENETICALLY ENGINEERED GROWTH FACTOR VARIANTS**
GENETISCH MANIPULIERTE WACHSTUMSFAKTORVARIANTEN
VARIANTS DE FACTEUR DE CROISSANCE GÉNÉTIQUEMENT MODIFIÉS

(30) Priority: 09.05.2011 US 201161484052 P
(43) Date of publication of application: 19.03.2014
(73) Proprietor: Minerva Biotechnologies Corporation, Waltham, MA 02451 (US)
(72) Inventor: BAMDAD, Cynthia, Boston, MA 02115 (US); SMAGGHE, Benoit, J., Honolulu, HI 96814 (US)
(74) Representative: Mewburn Ellis LLP
(86) International application number: PCT/US2012/036975
(87) International publication number: WO 2012/154759

(56) References cited:
- WO-A1-02/36626
- WO-A1-2010/144887
- WO-A2-02/077199
- WO-A2-2008/070171
- US-A1- 2005 112 607
- US-A1- 2006 228 357
- US-A1- 2007 009 918
- US-A1- 2009 075 926
- US-A1- 2009 175 867
- US-A1- 2010 093 092
- NEUHOLD L A ET AL: "HLH forced dimers: Tethering MyoD to E47 generates a dominant positive myogenic factor insulated from negative regulation by Id", CELL, CELL PRESS, US, vol. 74, no. 6, 24 September 1993 (1993-09-24), pages 1033-1042, XP027461974, ISSN: 0092-8674, DOI: 10.1016/0092-8674(93)90725-6 [retrieved on 1993-09-24]
- KIM Y-I ET AL: "Point mutations affecting the oligomeric structure of Nm23-H1 abrogates its inhibitory activity on colonization and invasion of prostate cancer cells", BIOCHEMICAL AND BIOPHYSICAL RESEARCH COMMUNICATIONS, ACADEMIC PRESS INC. ORLANDO, FL, US, vol. 307, no. 2, 25 July 2003 (2003-07-25) , pages 281-289, XP004435298, ISSN: 0006-291X, DOI: 10.1016/S0006-291X(03)01195-1
- EDITH H POSTEL ET AL: "Structure-based mutational and functional analysis identify human NM23-H2 as a multifunctional enzyme", BIOCHEMISTRY, vol. 41, no. 20, 27 April 2002 (2002-04-27), pages 6330-6337, XP055154108, ISSN: 0006-2960, DOI: 10.1021/bi025606+
- SUHN-KEE CHAE ET AL: "Transactivation potential of the C-terminus of human Nm23-H1", FEBS LETTERS, vol. 423, no. 2, 3 March 1998 (1998-03-03) , pages 235-238, XP055154144, ISSN: 0014-5793, DOI: 10.1016/S0014-5793(98)00100-8
- CHANG CHRISTINA L ET AL: "A nucleoside diphosphate kinase A (nm23-H1) serine 120 fwdarw glycine substitution in advanced stage neuroblastoma affects enzyme stability and alters protein-protein interaction", ONCOGENE, NATURE PUBLISHING GROUP, GB, vol. 12, no. 3, February 1996 (1996-02), pages 659-667, XP008173437, ISSN: 0950-9232
- POSTEL E H ET AL: "Mutational analysis of NM23-H2/NDP kinase identifies the structural domains critical to recognition of a c-myc regulatory element", PROCEEDINGS OF THE NATIONAL ACADEMY OF SCIENCES, NATIONAL ACADEMY OF SCIENCES, US, vol. 93, no. 14, July 1996 (1996-07), pages 6892-6897, XP002329794, ISSN: 0027-8424, DOI: 10.1073/PNAS.93.14.6892
- L. MILON: "The Human nm23-H4 Gene Product Is a Mitochondrial Nucleoside Diphosphate Kinase", JOURNAL OF BIOLOGICAL CHEMISTRY, vol. 275, no. 19, 12 May 2000 (2000-05-12) , pages 14264-14272, XP055154038, ISSN: 0021-9258, DOI: 10.1074/jbc.275.19.14264
- J. WANG ET AL: "Requirement of Nanog dimerization for stem cell self-renewal and pluripotency", PROCEEDINGS OF THE NATIONAL ACADEMY OF SCIENCES, vol. 105, no. 17, 29 April 2008 (2008-04-29), pages 6326-6331, XP055154155, ISSN: 0027-8424, DOI: 10.1073/pnas.0802288105
- PLUCKTHUN A ET AL: "New protein engineering approaches to multivalent and bispecific antibody fragments", IMMUNOTECHNOLOGY, ELSEVIER SCIENCE PUBLISHERS BV, NL, vol. 3, no. 2, June 1997 (1997-06), pages 83-105, XP004126672, ISSN: 1380-2933, DOI: 10.1016/S1380-2933(97)00067-5
- Masanao Murakami ET AL: "The suppressor of metastasis Nm23-H1 interacts with the Cdc42 Rho family member and the pleckstrin homology domain of oncoprotein Dbl-1 to suppress cell migration", Cancer biology & therapy, vol. 7, no. 5, 27 May 2008 (2008-05-27), pages 677-688, XP55378542, US ISSN: 1538-4047, DOI: 10.4161/cbt.7.5.5665
- VALENTIJN L J ET AL: "Read-through transcript from NM23-H1 into the neighboring NM23-H2 gene encodes a novel protein, NM23-LV", GENOMICS, ACADEMIC PRESS, SAN DIEGO, US, vol. 87, no. 4, 25 January 2006 (2006-01-25), pages 483-489, XP024929548, ISSN: 0888-7543, DOI: 10.1016/J.YGENO.2005.11.004 [retrieved on 2006-04-01]

## Description

### BACKGROUND OF THE INVENTION

### 1. Field of the Invention:

The present application relates to the field of growth factor variants and methods for controlling the multimerization of such growth factors.

### 2. General Background and State of the Art

In biological systems, proteins often make up complicated signaling cascades that direct the cell to behave in a particular way. For example, a common way that cells are directed to begin the process of dividing is that a protein (ligand) binds to the extra cellular domain of a transmembrane protein receptor wherein binding of the ligand to the extra cellular domain confers a change in the conformation of the receptor. The ligand-induced conformational change can take place in the extra cellular domain, the intra cellular domain or both and results in a change in which proteins or molecules are able to bind to the receptor. This outside to inside signaling is a common mechanism that is used to signal cells to divide, initiate programmed cell death and many other processes.

One commonly used mechanism that regulates the activity of growth factor receptors is ligand-induced dimerization of the receptor's extra cellular domain which in turn brings the intracellular tails close together which makes a good docking site for modifying proteins such as kinases that initiate a signaling cascade that eventuates in a signal to the cell's nucleus that causes the cell to divide.

Ligand-induced dimerization of the extra cellular domain of growth factor receptors is often accomplished through the binding of ligand dimers; that is two ligands non-covalently bind to each other to form homo- or hetero-dimers which then bind to two receptors that are either the same (homo) or different (hetero).

An important example of ligand-induced receptor dimerization is NM23 dimers binding to and dimerizing the extra cellular domain of MUC1*, which is the truncated form of the MUC1 transmembrane protein that is tumor and stem cell specific. Whether or not the ligand is a monomer, dimer or a higher order multimer is a function of, among other things, its concentration. For many growth factor receptors, only the dimeric form of the ligand activates the growth factor receptor. Additionally, in many biological systems, there are feedback loops wherein the higher order multimers turn off the function that is promoted by the dimer. For example, the NM23 dimer activates pluripotent growth but the NM23 hexamer turns off pluripotent growth and initiates differentiation. Similarly, the CI protein of Phage lambda turns on transcription of one set of genes when it is bound to DNA as a tetramer but turns off transcription of those genes when, as a function of increased concentration, the CI protein becomes an octamer. In many cases, it is desirable to constitutively activate a growth factor receptor, or increase some activity that is mediated by a specific multimerization state of a protein. The problem is that it is very difficult to express and isolate a specific multimer and even more difficult to maintain that multimerization state when it is added to a biological system or expressed within a biological system. Therefore, it would be advantageous to be able to generate ligands that exist exclusively in a specific multimerization state, or prefer that multimerization state, such as dimers or that prefer dimerization.

WO 2008/070171 describes methods for proliferating cells by adding NM23. Although NM23 mutants have been reported that prefer dimer formation, the portion that exists as the active dimer relative to the inactive hexamer varies greatly, particularly when expressed as the recombinant protein, depending on the cell that is expressing it, concentration, and a number protein expression conditions that are difficult or impossible to control. Therefore, it would be beneficial to develop methods, including recombinant methods, which would result in a higher percentage of or more stable populations of dimeric forms of NM23 or NM23 mutants.

### SUMMARY OF THE INVENTION

The invention which is defined in the appended claims overcomes the above-mentioned problems

### BRIEF DESCRIPTION OF THE DRAWINGS

The present invention will become more fully understood from the detailed description given herein below, and the accompanying drawings which are given by way of illustration only, and thus are not limitative of the present invention, and wherein;

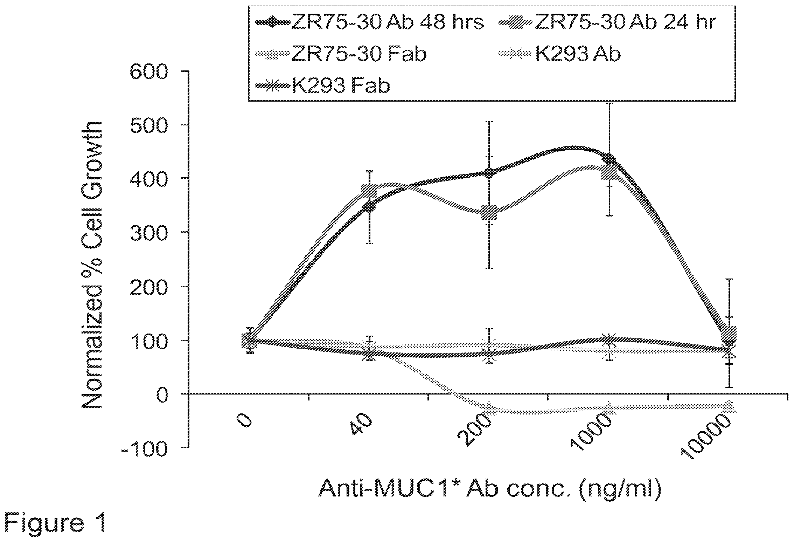

Figure 1 is a graph of cancer cell growth measured as a function of bivalent or monovalent antibody concentration, showing that it is dimerization of the MUC1* receptor that stimulates growth. The growth of MUC1-positive breast cancer cells, ZR-75-30, was stimulated by the addition of bivalent (Ab) Anti-MUC1* and inhibited by the addition of the monovalent Fab. The addition of bivalent antibody produces the characteristic bell-shaped growth curve indicative of growth factor receptor dimerization. The growth of MUC1-negative HEK 293 cells was not impacted by either the bivalent or monovalent Fab Anti-MUC1*. When the bivalent antibody was added in excess, there is one bivalent antibody bound to each receptor rather than one bivalent antibody dimerizing every two receptors and thus inhibits growth.

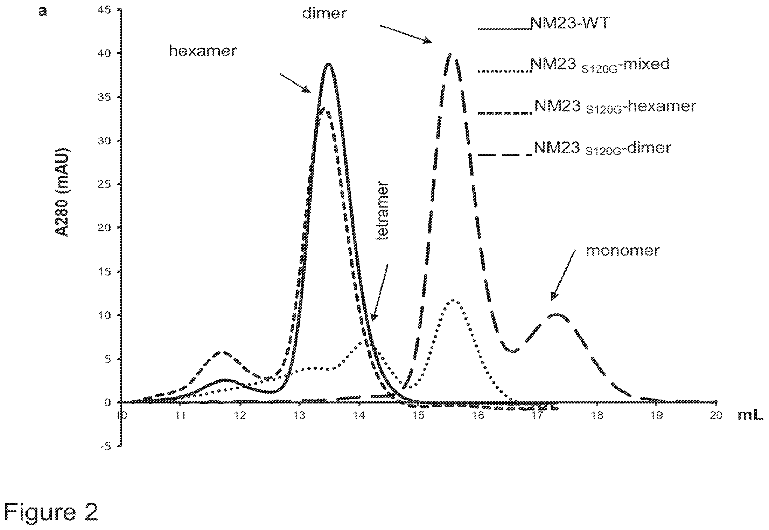

Figure 2 shows an overlay of FPLC traces that characterize the different multimerization states of either wild type NM23 (WT) or three different preparations of mutant NM23-S120G. The wild type NM23 shows a single peak that corresponds to the molecular weight of the hexamer and a shoulder corresponding to higher order multimers. One preparation of NM23-S120G (labeled "mixed") that was not refolded, has the dominant peak that corresponds to the dimer and a lesser peak of tetramers. Another preparation of NM23-S120G ("hexamer") that was also not refolded has the major peak of hexamers with shoulder of higher order multimers. A refolded preparation of NM23-S120G ("dimer") is comprised mostly of dimers.

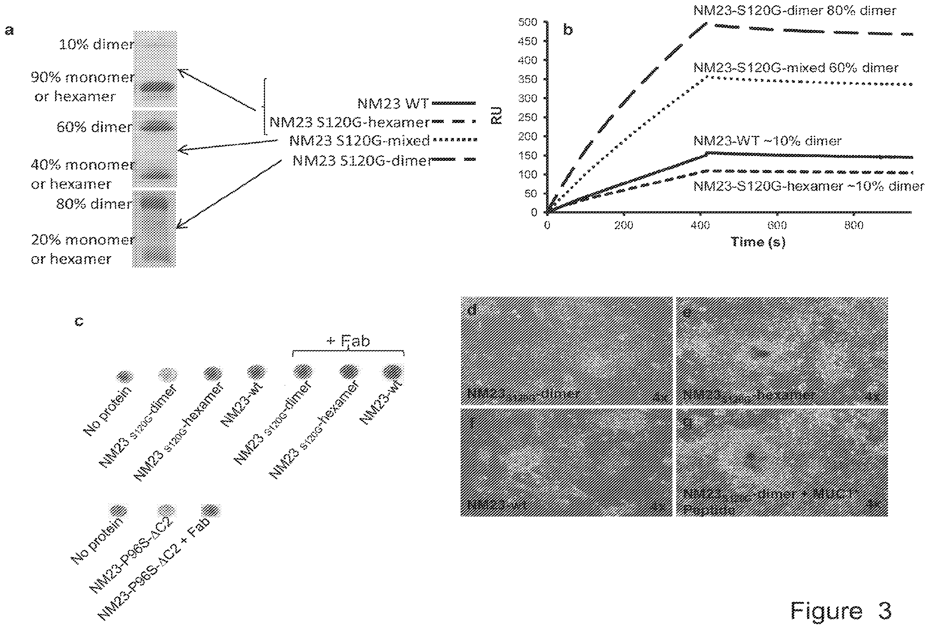

Figure 3a) shows photographs of non-reducing gels of NM23-WT, NM23-S120G-mixed, NM23-S120G-hexamer and NM23-S120G-dimer, which show the multimerization state of the wild type protein and the three different preparations of the S120G mutant. b) shows an overlay of Surface Plasmon Resonance (SPR) measurements showing the ability of the four different NM23s to bind to a MUC1* extra cellular domain peptide (PSMGFR) attached to the SPR chip surface. Results show that the amount of binding of NM23 to its cognate receptor, MUC1*, is a function of how much dimer is present in the sample. SPR measures protein mass at the chip-solution interface, so if the hexamer bound to the MUC1* peptide surface, it would yield an SPR signal 3-times greater than if a dimer bound. c) shows photograph of a nanoparticle experiment that shows that only NM23 dimers bind to the cognate receptor MUC1*. MUC1* extra cellular domain peptide was immobilized onto gold nanoparticles. To each aliquot of nanoparticles, either NM23-WT, NM23-S120G-dimer or NM23-S120G-hexamer was added. If the NM23 bound to the nanoparticle immobilized MUC1* peptide, it would cause the nanoparticles to become drawn close together which causes the solution to change from pink to blue. The experiment shows that only the NM23-S120G-dimer bound to the MUC1* peptide. The addition of an anti-MUC1* Fab competitively inhibited binding of NM23-S120G-dimers in solution to the MUC1* peptide on the nanoparticles. (d-g) shows different NM23 multimers tested for their ability to support pluripotent stem cell growth. Human ES (embryonic stem) cells were cultured in either (d) NM23-S120G-dimer, (e) NM23-S120G-hexamer, (f) NM23-WT, or (g) NM23-S120G-dimer plus the MUC1* extra cellular domain peptide (PSMGFR) to competitively inhibit binding of the NM23 dimer to the MUC1* receptor on the stem cell surface. Induction of differentiation is readily observed (colony thickening, darkening) in (g), (e), and (f) in that order, showing that inhibition of the NM23-dimer-MUC1* interaction induces differentiation as does culturing the cell in NM23 hexamers that do not bind to MUC1*. Only the dimer preparation of NM23-S120G (d) was able to support undifferentiated stem cell growth.

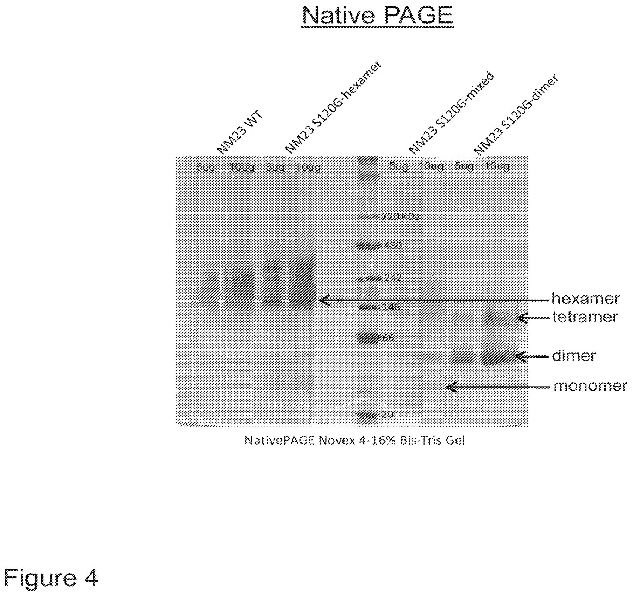

Figure 4 shows a native, non-denaturing gel that shows the multimerization state of NM23-WT versus three different preparations of recombinant NM23-S120G.

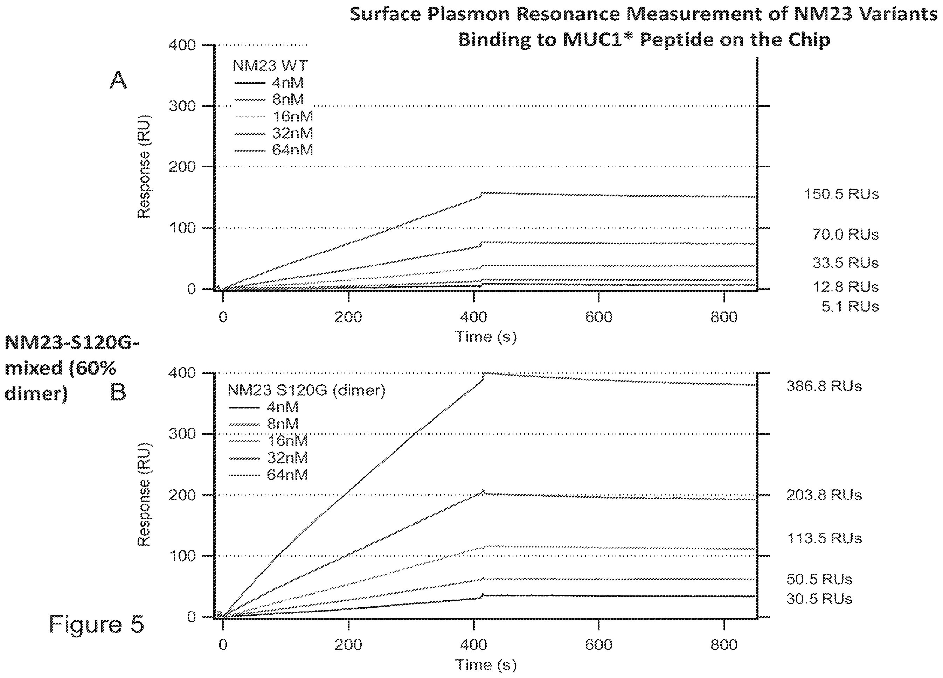

Figure 5 shows SPR measurements of A) NM23 wild type (WT) and B) a preparation of NM23-S 120G- "mixed" that produced 60% dimer. Protein was injected at five different concentrations. Results show that 8-times more NM23-S120G-mixed protein bound to a MUC1* extra cellular domain peptide surface than NM23-WT. Because the wild type protein is a hexamer, the number of RUs must be divided by 3 to compare to the amount of dimer that bound. Although both wild type and S120G-dimer show a concentration dependence in binding, the amount of wild type hexamer that bound is so small that it may still be within the noise range of the system.

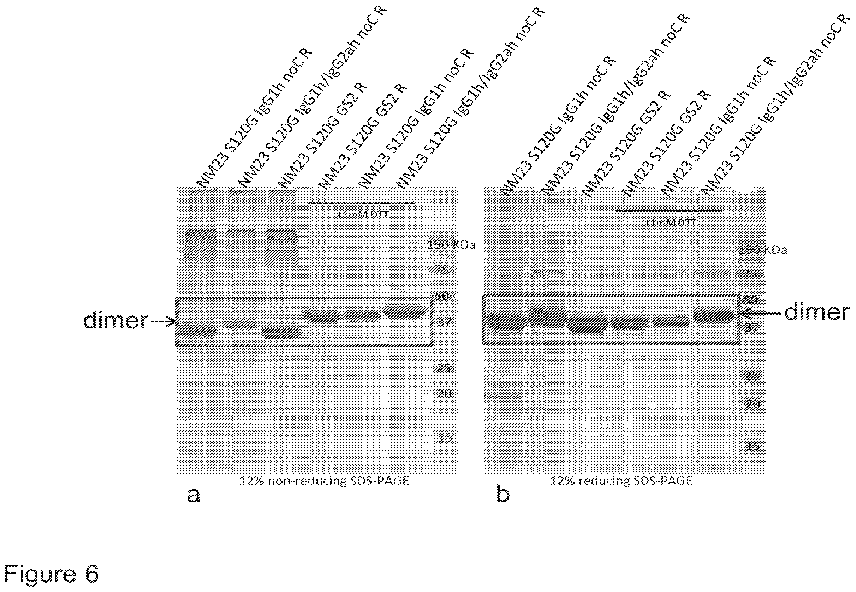

Figure 6(a) shows a photograph of a non-reducing gel loaded with NM23 single chain variants in the presence or absence of added DTT (dithiothreitol) to reduce disulfide bonds. Dimers are indicated by red boxes. In the absence of DTT, there are some higher molecular weight species, which may be because the protein was loaded onto the gel at roughly 1000-times the concentration that is used for culturing cells, (b) shows a photograph of a reducing gel shows that the NM23 single chain variants migrate through the gel with the expected molecular weight of dimers.

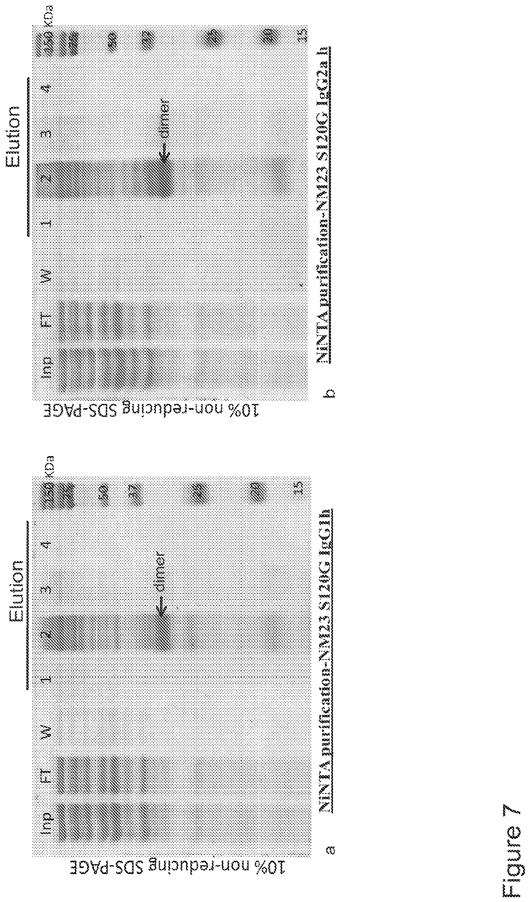

Figure 7 shows PAGE characterization of the purification of single chain variants NM23 S120G IgG1h and NM23 S120G IgG2ah using a non-reducing gel to avoid disruption of disulfide bonds.

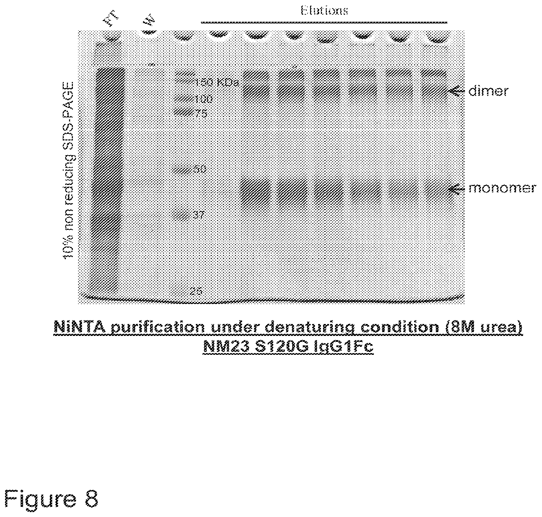

Figure 8 shows PAGE characterization of the purification of the fusion chimera variant NM23 S120G IgG1Fc using a non-reducing gel to avoid disruption of disulfide bonds.

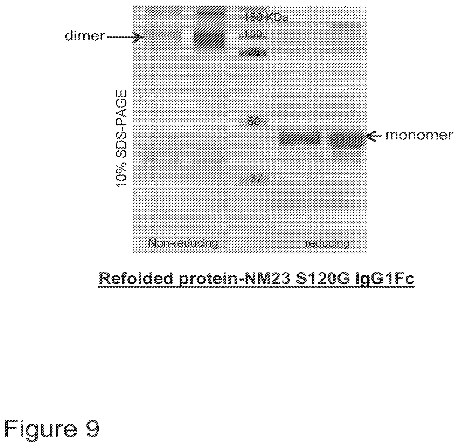

Figure 9 shows PAGE characterization of refolded fusion chimera variant NM23 S120G IgG1Fc using a non-reducing gel to avoid disruption of disulfide bonds, where the variant runs with the molecular weight of a dimer and also using a reducing gel which shows that in the absence of the disulfide binds, the fusion variant runs with the apparent molecular weight of a monomer.

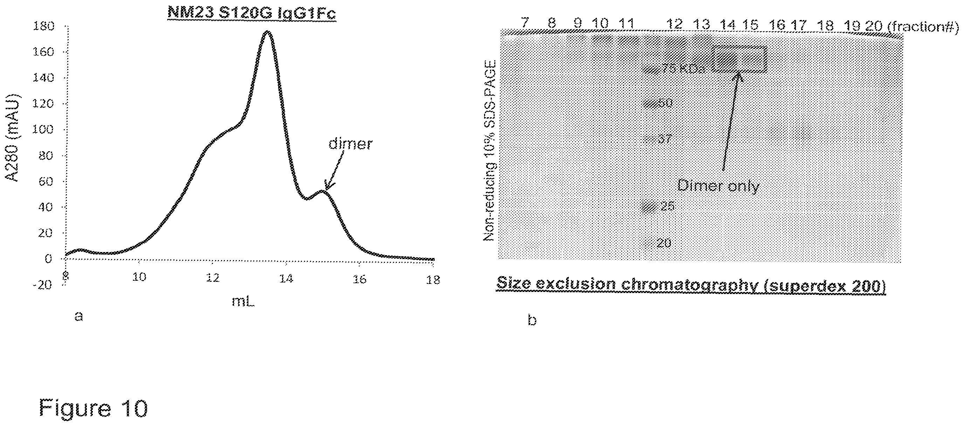

Figure 10 shows FPLC (a) and non-reducing SDS-PAGE (b) characterization of the fusion chimera variant NM23-S120G-IgG1Fc.

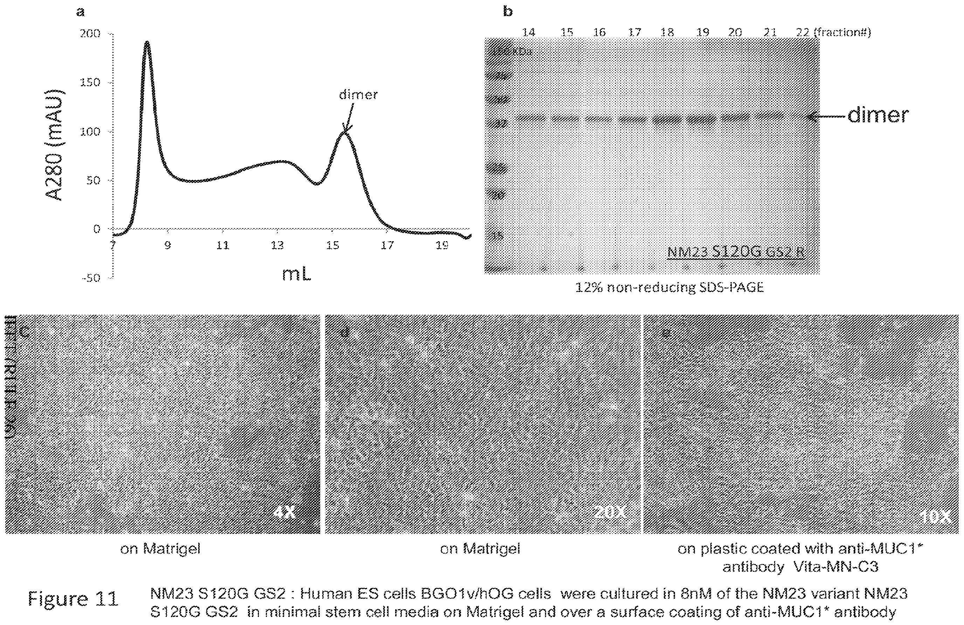

Figure 11 shows FPLC (a) and non-reducing SDS-PAGE (b) of refolded NM23-S120G-GS2 that shows major populations of dimer. (c-e) show photographs of human ES cells, BGO1v/hOG line, that were cultured in 8nM of the NM23 variant NM23-S120G-GS2, which was not refolded or purified, in minimal stem cell media on Matrigel (c and d) and on a cell culture plate coated with anti-MUC1* antibody, MN-C3 (e). These data show that NM23-S120G-GS2 does not need to be refolded or purified before use.

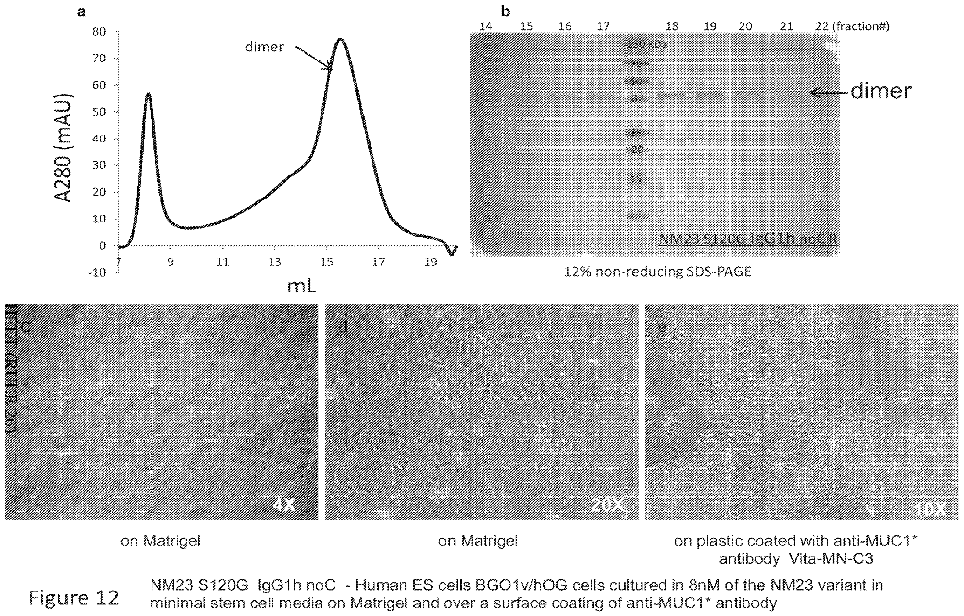

Figure 12 shows FPLC (a) and non-reducing SDS-PAGE (b) of refolded NM23-S120G -IgG1h noC that shows major populations of dimer. (c-e) shows photographs of human ES cells, BGO1v/hOG line, that were cultured in 8nM of the NM23 variant NM23-S120G -IgG1h noC, which was not refolded or purified, in minimal stem cell media on Matrigel (c and d) and on a cell culture plate coated with anti-MUC1* antibody, MN-C3 (e). These data show that this variant does not need to be refolded or purified before use.

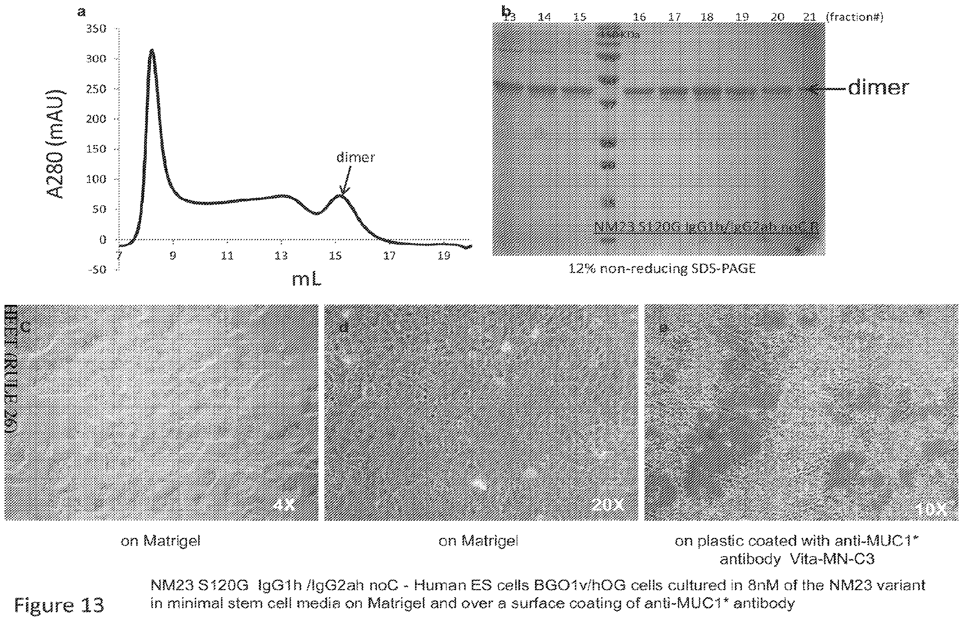

Figure 13 shows FPLC (a) and non-reducing SDS-PAGE (b) of refolded NM23-S120G -IgG1h /IgG2ah noC that shows major populations of dimer. (c-e) shows photographs of human ES cells, BGO1v/hOG line, that were cultured in 8nM of the NM23 variant NM23-S120G -IgG1h /IgG2ah noC, which was not refolded or purified, in minimal stem cell media on Matrigel (c and d) and on a cell culture plate coated with anti-MUC1* antibody, MN-C3 (e). These data show this variant does not need to be refolded or purified before use.

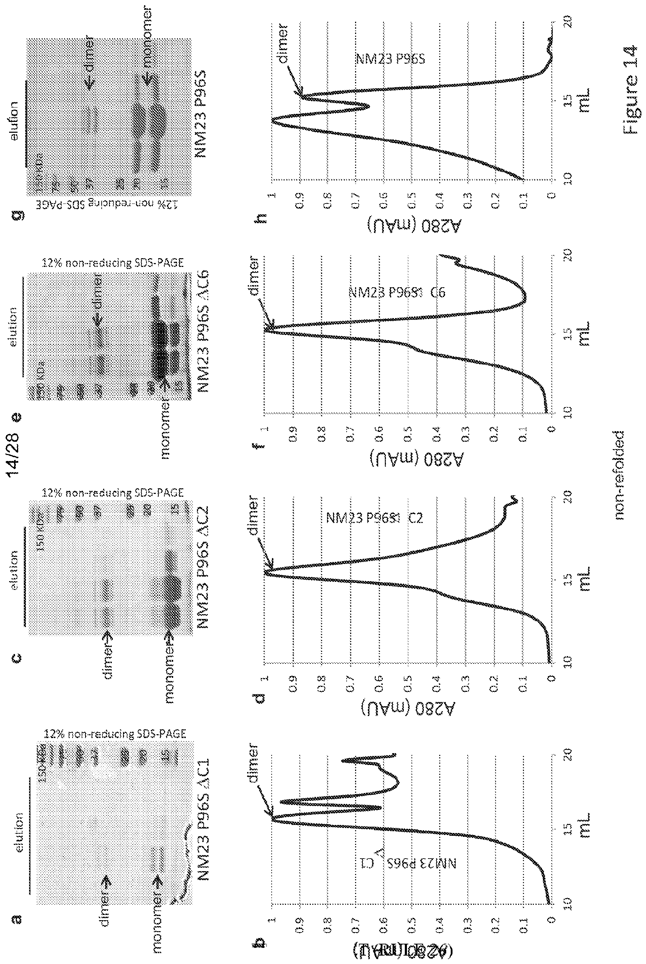

Figures 14(a) to 14(h) show SDS-PAGE characterization using non-reducing gels and corresponding FPLC traces for NM23 single chain variants NM23-P96SΔC1 (a, b), NM23-P96SΔC2 (c, d), NM23-P96SΔC6 (e, f) and NM23-P96S (g, h), which were not refolded or purified. As can be seen from comparison of the FPLC traces, NM23-P96SΔC2 and NM23-P96SΔC6 have their major peaks in the dimer range and thus are preferred. These data show that these variants do not need to be refolded or purified before use.

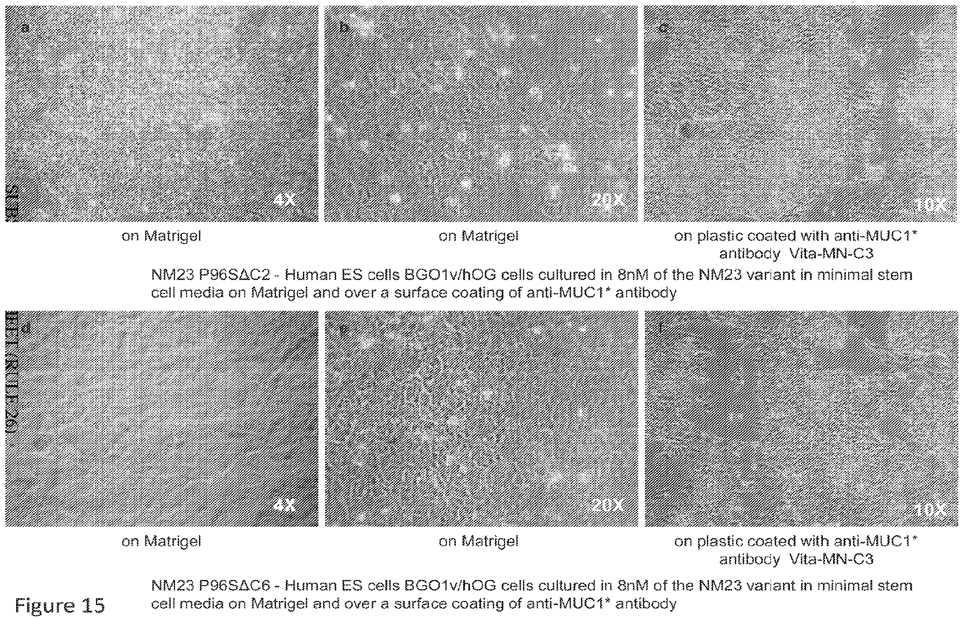

Figure 15 shows photographs of human ES cells, BGO1v/hOG line, that were cultured in 8nM of an NM23 variant in minimal stem cell media on Matrigel (a, b, d, e) and on a cell culture plate coated with anti-MUC1* antibody, MN-C3 (c, f); (a-c) variant is P96SΔC2; (d-f) variant is P96SΔC6. These variants were not refolded or purified, showing that they do not need to be refolded or purified before use.

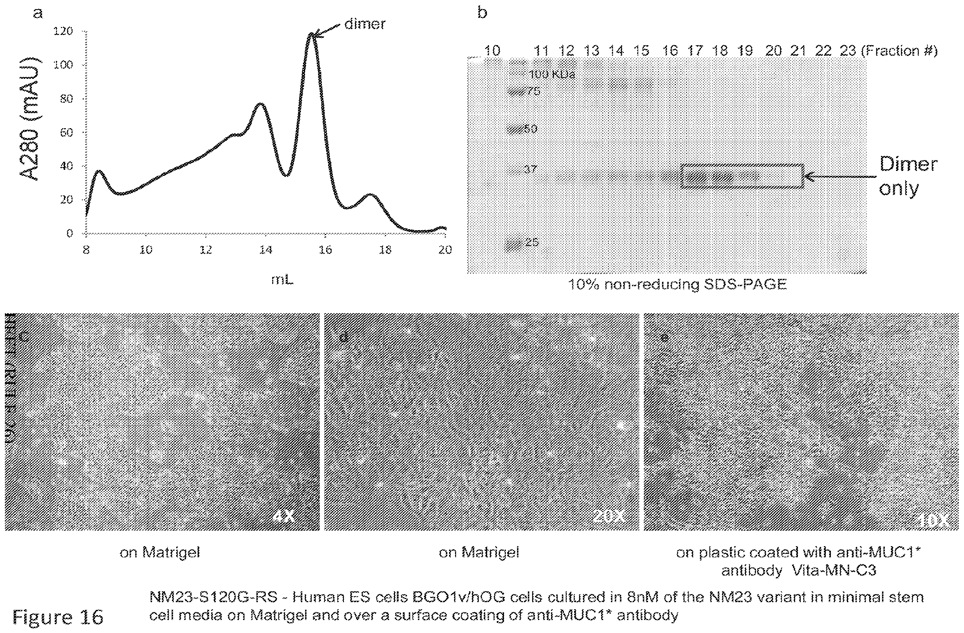

Figure 16 shows that the major population of NM23-S 120G (refolded,"RS") exists as a dimer as shown in the FPLC trace (a) and verified by a non-reducing PAGE (b). Dimer only fractions purified by FPLC were pooled and used at 8nM in minimal stem cell media to grow human ES cells, BGO1v/hOG line, (c-e) photographs of the human stem cells show that cultured in 8nM of the NM23 variant produces pluripotent stem cells whether on Matrigel (c, d) or on a cell culture plate coated with anti-MUC1* antibody, MN-C3 (e).

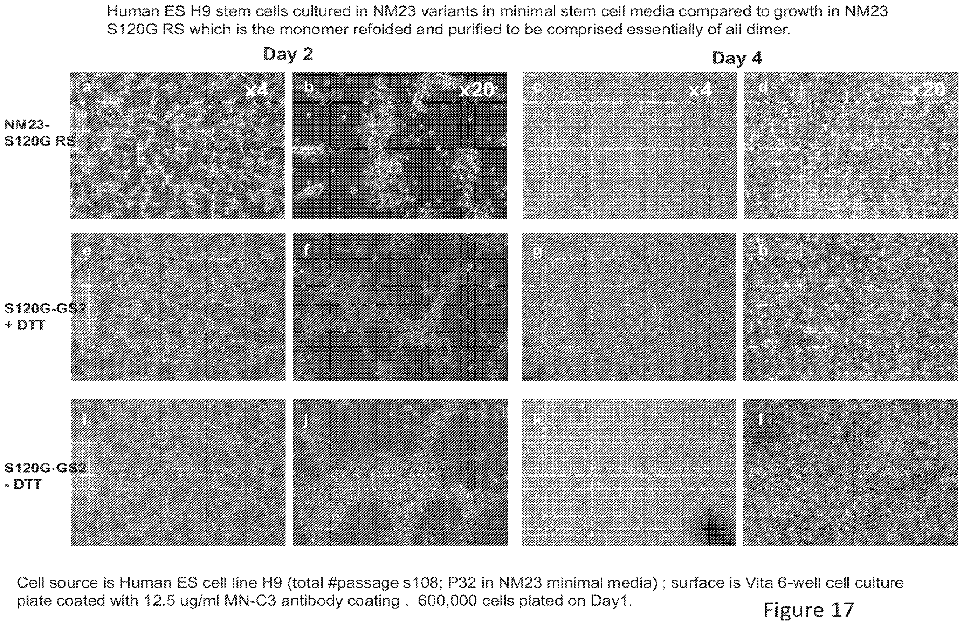

Figure 17 shows photographs of human ES cells (H9 line) cultured in minimal stem cell media plus either refolded and purified NM23-S120G RS (a-d) or the single chain construct S120G-GS2 that is expressed as the dimer and does not need to be refolded and purified. S120G-GS2 was added either in the presence (e-h) or absence (i-1) of added DTT. The images show that stem cells cultured in the single chain variant grow as well as they do in the dimer fraction of the refolded and purified NM23-S120G-RS. Cells are undifferentiated and pluripotent stem cells as evidenced by the lack of thickening and darkening of the cell masses.

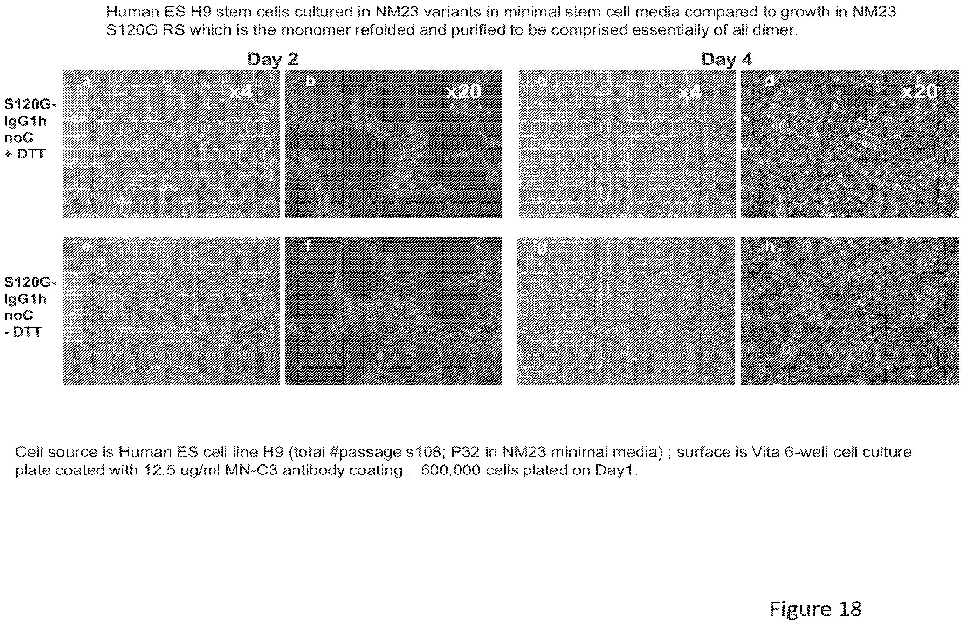

Figure 18 shows photographs of human ES cells (H9 line) cultured in NM23-S120G-IgG1h noC in minimal stem cell media. This NM23 variant is expressed as the dimer and does not require further processing or refolding to ensure that it is in the dimer state. S120G-IgG1h noC was added either in the presence (a-d) or absence (e-h) of added DTT. The images show that stem cells cultured in the single chain variant grow as well as they do in the dimer fraction of the refolded and purified S120G RS (compare to Figure 17a-17d). Cells are undifferentiated and pluripotent stem cells as evidenced by the lack of thickening and darkening of the cell masses.

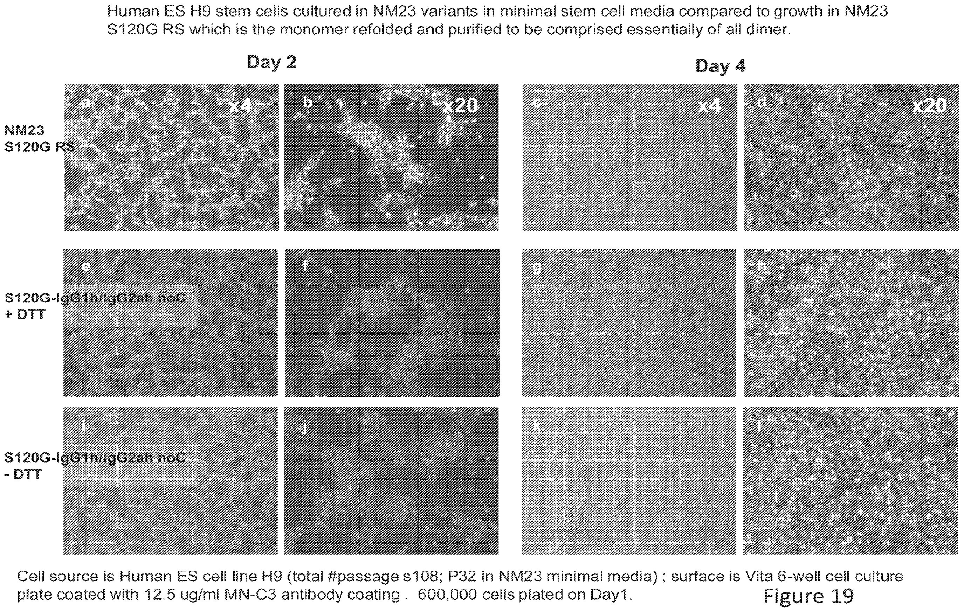

Figure 19 shows photographs of human ES cells (H9 line) cultured in minimal stem cell media plus either refolded and purified NM23-S120G RS (a-d) or the single chain construct (NM23) S120G-IgG1h/IgG2ah noC that is expressed as the dimer and does not need to be refolded and purified. S120G-IgG1h/IgG2ah noC was added either in the presence (e-h) or absence (i-1) of added DTT. The images show that stem cells cultured in the single chain variant grow as well as they do in the dimer fraction of the refolded and purified NM23-S120G RS (compare to Figure 17a-17d). Cells are undifferentiated and pluripotent stem cells as evidenced by the lack of thickening and darkening of the cell masses.

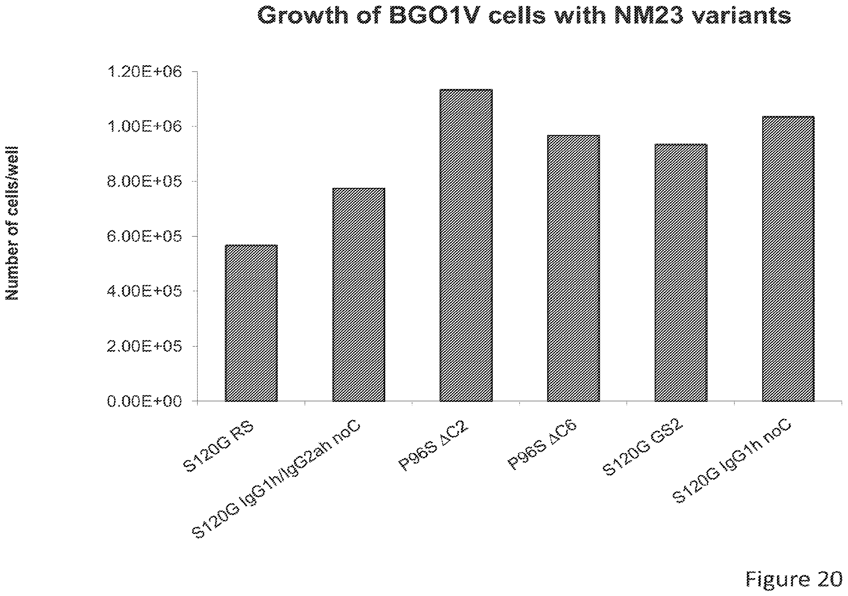

Figure 20 shows a graph that plots the growth of human stem cells cultured in either the refolded and purified dimer only fraction of NM23-S120G or NM23 variants that are designed to spontaneously form dimers or are single chain constructs comprised of two NM23 monomers. In each case, 200,000 cells were plated and cells were counted 4 days later. The graph shows that the variants that do not require refolding or further purification of dimer population cause stem cells to proliferate as well or better than NM23-S120G-RS that has been refolded to form a majority of dimers and further purified by FPLC (compare to Figure 17a-17d).

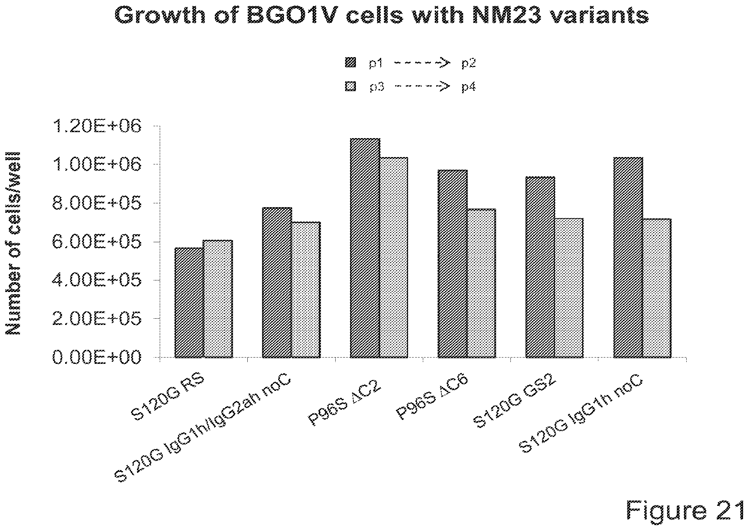

Figure 21 shows a graph that plots the growth of human stem cells cultured in either the refolded and purified dimer only fraction of NM23-S120G-RS or NM23 variants that are designed to spontaneously form dimers or are single chain constructs comprised of two NM23 monomers. The graph shows that the variants cause stem cells to proliferate as well or better than NM23-S120G-RS and that the effect persists over several passages.

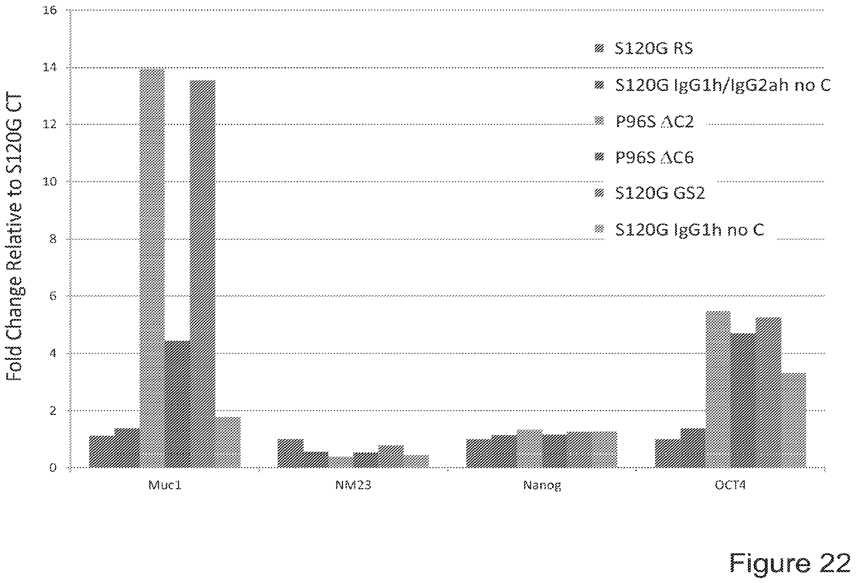

Figure 22 is a graph of RT-PCR measurements of gene expression of the NM23 variants compared to the refolded and purified NM23-S120G-RS. Gene expression analysis shows that culturing cells in the NM23 variants designed to spontaneously form dimers, express the pluripotency genes to the same or higher levels as the refolded and purified dimer only populations of NM23.

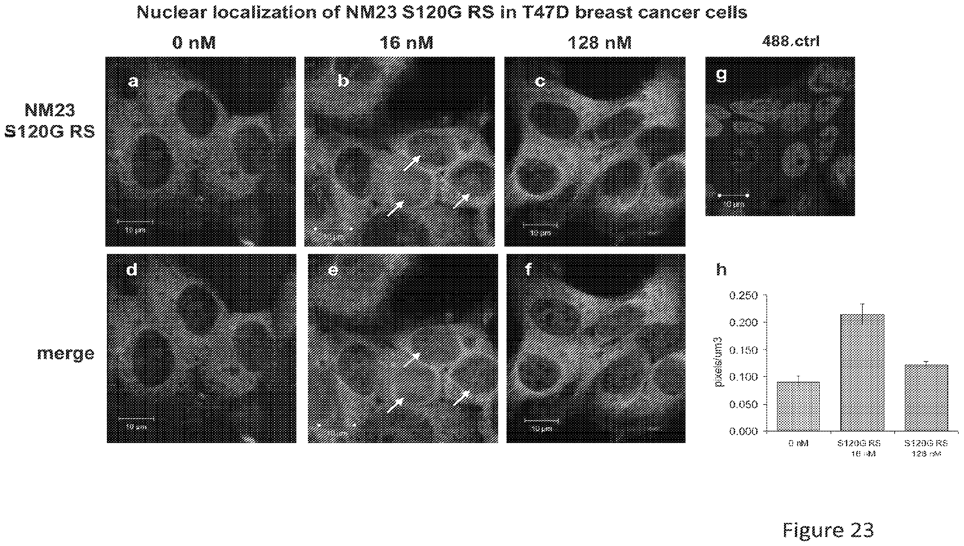

Figure 23 shows photographs of the nuclear localization of NM23-S120G-RS which is the single point mutation that favors dimerization but must be refolded and purified to obtain a population of mostly dimer. a) no exogenous NM23-S120G-RS added; b) 16nM of NM23-S120G RS; c) 128nM of NM23-S120G RS. (a-c) cells are stained with fluorescently labeled anti-NM23 antibody; (d-f) overlay of DAPI nuclear stain and the fluorescently labeled NM23. White arrows indicate presence of NM23 in the nucleus. (g) is the 488 fluorescent control. (h) is a bar graph showing the quantification of NM23 in the nucleus for conditions a-c and shows that adding exogenous NM23-S120G RS to the culture media causes internalization of the NM23 and translocation to the nucleus. Note that there is more NM23 in the nucleus when added at 16nM than when added at 128nM, which is consistent with our findings that at too high a concentration, the NM23 dimers bind to every MUC1* receptor, rather than the NM23 dimers binding to and dimerizing two MUC1* receptors.

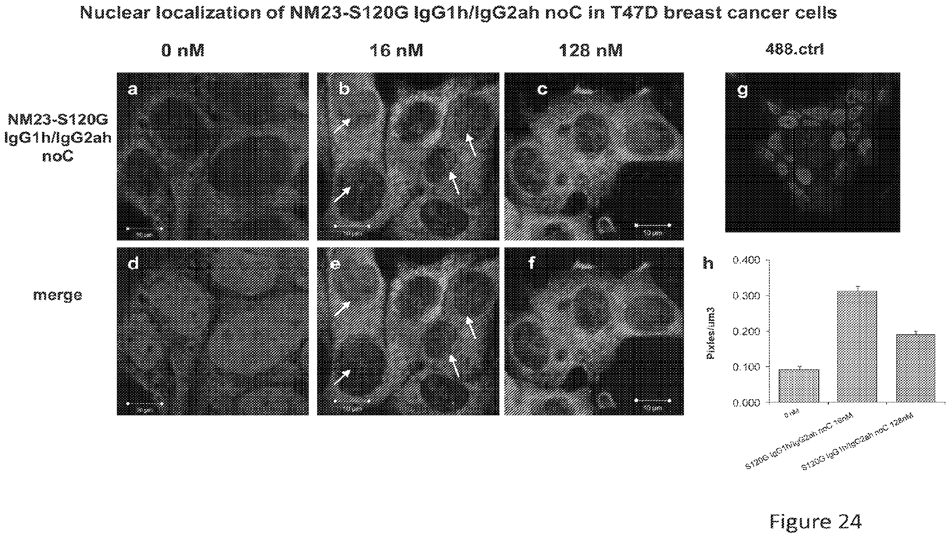

Figure 24 shows photographs of the nuclear localization of NM23 variant NM23-S120G IgG1h /IgG2ah noC. a) no exogenous NM23 variant added; b) 16nM of the NM23 variant; c) 128nM of the NM23 variant. (a-c) cells are stained with fluorescently labeled anti-NM23 antibody; (d-f) overlay of DAPI nuclear stain and the fluorescently labeled NM23. White arrows indicate presence of NM23 in the nucleus. (g) is the 488 fluorescent control. (h) is a bar graph showing the quantification of NM23 in the nucleus for conditions a-c and shows that adding exogenous NM23 S120G IgG1h /IgG2ah noC variant to the culture media causes internalization of the NM23 and translocation to the nucleus. Note that there is more of the NM23 variant in the nucleus when added at 16nM than when added at 128nM, which is consistent with our findings that at too high a concentration, the NM23 dimers bind to every MUC1* receptor, rather than the NM23 dimers binding to and dimerizing two MUC1* receptors.

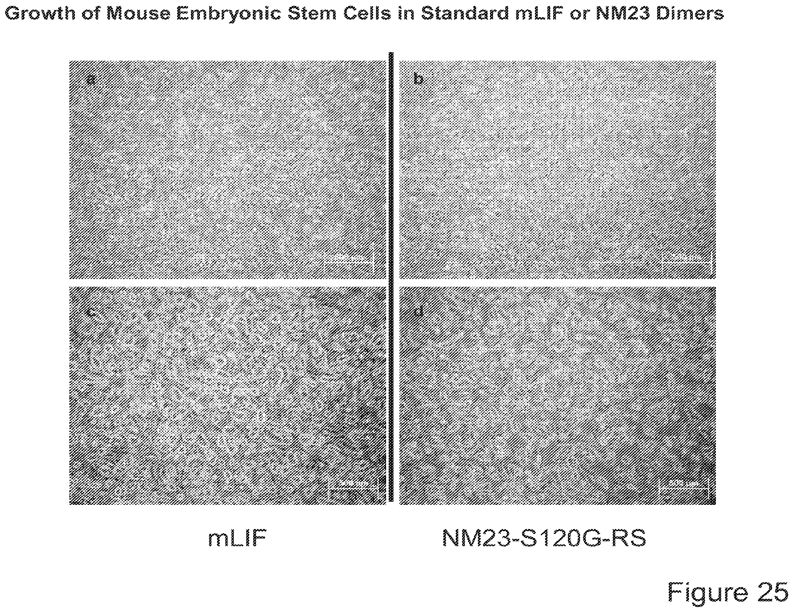

Figure 25 shows photographs of mouse embryonic stem (ES) cells that have been cultured in on inactivated MEF feeder cell layers for two days in mouse ES cell minimal medium supplemented with either mLIF or NM23-S120G-RS. The images show that mouse ES cells grow as well using NM23 dimers as the only growth factor as they do in the standard mouse stem cell media with m LIF as the basic growth factor.

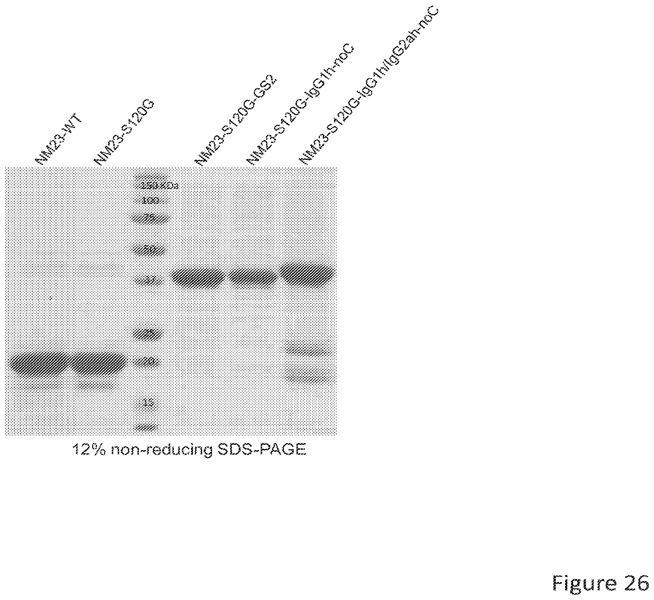

Figure 26 shows photographs of non-reducing SDS-PAGE characterization of NM23 variants NM23-S120G-GS2, NM23-S120G-IgG1h noC and NM23-S120G-IgG1h/IgG2ah noC that have not been refolded, compared to NM23-WT and NM23-S120G (non-refolded). a) shows that on a non-reducing gel, variants NM23-S120G-GS2, NM23-S120G-IgG1h noC and NM23-S120G-IgG1h/IgG2ah noC that have not been refolded or purified exist as the dimer in contrast to the wild type protein that runs with an apparent molecular weight of a monomer. However, recall that NM23 hexamers run at the molecular weight of monomers on non-reducing gels because higher order NM23 multimers do not depend on disulfide bonds, while the dimers do.

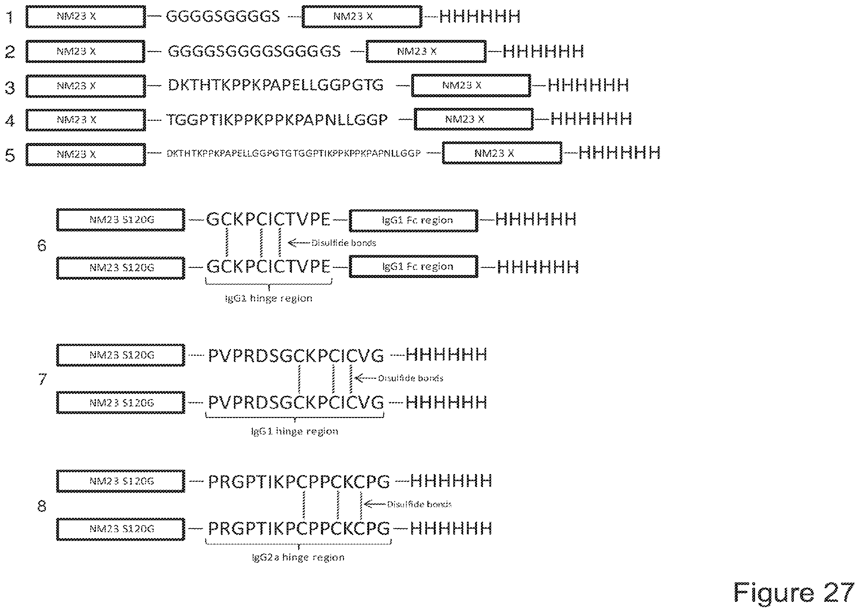

Figure 27 shows examples of recombinantly produced NM23 variants. It is noted that affinity tags, such as (Histidine)₆, Strep TagII, and so forth are optional elements.

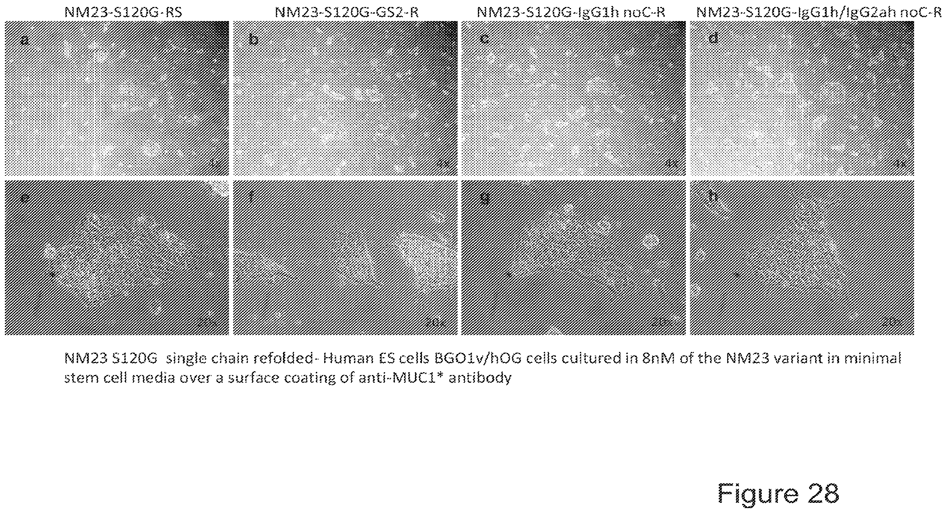

Figure 28 shows photographs of human stem cells cultured in NM23 variants that had been refolded (denoted by the "R" in figure labels). The NM23 variants used at 8nM were NM23-S120G-RS (a,e), NM23-S120G-GS2 (b,f), NM23-S120G-IgG1h noC (c,g), and NM23-S120G-IgG1h/IgG2ah noC (d,h). The cell morphology is consistent with pluripotent stem cells, as they are round and not fibroblast in shape and they are a single layer devoid of thickening or darkening which are indicative of cell differentiation.

### DETAILED DESCRIPTION OF THE PREFERRED EMBODIMENTS

In the present application, "a" and "an" are used to refer to both single and a plurality of objects.

As used herein, "multimer" refers to a plurality of monomers that are covalently linked together or non-covalently fused to each other.

As used herein, "higher order multimer" refers to a plurality of monomers that are covalently linked together or non-covalently fused to each other, which is greater than a dimer.

### Sequence Listing Free Text

As regards the use of nucleotide symbols other than a, g, c, t, they follow the convention set forth in WIPO Standard ST.25, Appendix 2, Table 1, wherein k represents t or g; n represents a, c, t or g; m represents a or c; r represents a or g; s represents c or g; w represents a or t and y represents c or t.

### Human NM23 H1

(DNA) 
atggccaactgtgagcgtaccttcattgcgatcaaaccagatggggtccagcggggtcttgtgggagagattatcaag
cgttttgagcagaaaggattccgccttgttggtctgaaattcatgcaagcttccgaagatcttctcaaggaacactacgttgacctgaagg
accgtccattctttgccggcctggtgaaatacatgcactcagggccggtagttgccatggtctgggaggggctgaatgtggtgaagac
gggccgagtcatgctcggggagaccaaccctgcagactccaagcctgggaccatccgtggagacttctgcatacaagttggcagga
acattatacatggcagtgattctgtggagagtgcagagaaggagatcggcttgtggtttcaccctgaggaactggtagattacacgagc
tgtgctcagaactggatctatgaatga (SEQ ID NO:97)
(amino acids)

MANCERTFIAIKPDGVQRGLVGEIIKRFEQKGFRLVGLKFMQASEDLLKEH
YVDLKDRPFFAGLVKYMHSGPVVAMVWEGLNVVKTGRVMLGETNPADSKPGTIRG
DFCIQVGRNIIHGSDSVESAEKEIGLWFHPEELVDYTSCAQNWIYE- (SEQ ID NO:98)

### Mouse NM23 H1

(DNA) 
atggccaacagtgagcgcaccttcattgccatcaagcctgatggggtccagcgggggctggtgggcgagatcatca
agcggttcgagcagaaggggttccgccttgttggtctgaagtttctgcaggcttcagaggaccttctcaaggagcactacactgacctg
aaggaccgccccttctttactggcctggtgaaatacatgcactcaggaccagtggttgctatggtctgggagggtctgaatgtggtgaa
gacaggccgcgtgatgcttggagagaccaaccccgcagactctaagcctgggaccatacgaggagacttctgcatccaagttggca
ggaacatcattcatggcagcgattctgtaaagagcgcagagaaggagatcagcttgtggtttcagcctgaggagctggtggagtacaa
gagctgtgcgcagaactggatctatgagtga (SEQ ID NO:99)
(amino acids)

MANSERTFIAIKPDGVQRGLVGEIIKRFEQKGFRLVGLKFLQASEDLLKEH
YTDLKDRPFFTGLVKYMHSGPVVAMVWEGLNVVKTGRVMLGETNPADSKPGTIRG
DFCIQVGRNIIHGSDSVKSAEKEISLWFQPEELVEYKSCAQNWIYE- (SEQ ID NO:100)

### Human NM23 H2

(DNA) 
atggccaacctggagcgcaccttcatcgccatcaagccggacggcgtgcagcgcggcctggtgggcgagatcatc
aagcgcttcgagcagaagggattccgcctcgtggccatgaagttcctccgggcctctgaagaacacctgaagcagcactacattgac
ctgaaagaccgaccattcttccctgggctggtgaagtacatgaactcagggccggttgtggccatggtctgggaggggctgaacgtg
gtgaagacaggccgagtgatgcttggggagaccaatccagcagattcaaagccaggcaccattcgtggggacttctgcattcaggtt
ggcaggaacatcattcatggcagtgattcagtaaaaagtgctgaaaaagaaatcagcctatggtttaagcctgaagaactggttgacta
caagtcttgtgctcatgactgggtctatgaataa (SEQ ID NO:101)
(amino acids)

MANLERTFIAIKPDGVQRGLVGEIIKRFEQKGFRLVAMKFLRASEEHLKQH
YIDLKDRPFFPGLVKYMNSGPVVAMVWEGLNVVKTGRVMLGETNPADSKPGTIRG
DFCIQVGRNIIHGSDSVKSAEKEISLWFKPEELVDYKSCAHDWVYE- (SEQ ID
NO:102)

### Mouse NM23 H2

(DNA) 
atggccaacctcgagcgtaccttcattgccatcaagccagatggcgtgcagcgcggcctggtgggcgagatcatca
aacggttcgagcagaaggggttccgcctggtggccatgaagttccttcgggcctctgaagaacacctgaagcagcattacatcgacct
gaaagaccgtcctttcttcccggggctggtgaagtacatgaactcggggcccgtggtggccatggtctgggaggggctcaatgtggt
 
gaaaacgggccgagtgatgctgggggagaccaatccagctgattcaaaaccaggcaccatccgtggggatttctgcattcaagttgg
caggaacatcattcatggcagtgattcagtggagagtgctgagaaagagatccatctgtggtttaagcccgaagaactgatcgactaca
agtcttgtgcccatgactgggtgtacgagtag (SEQ ID NO:103)
(amino acids)

MANLERTFIAIKPDGVQRGLVGEIIKRFEQKGFRLVAMKFLRASEEHLKQH
YIDLKDRPFFPGLVKYMNSGPVVAMVWEGLNVVKTGRVMLGETNPADSKPGTIRG
DFCIQVGRNIIHGSDSVESAEKEIHLWFKPEELIDYKSCAHDWVYE- (SEQ ID NO:104)

### NM23 S120G (cloned between NdeI and XhoI) tagged with histidine cluster at the C-terminus

(DNA) 
atggccaactgtgagcgtaccttcattgcgatcaaaccagatggggtccagcggggtcttgtgggagagattatcaag
cgttttgagcagaaaggattccgccttgttggtctgaaattcatgcaagcttccgaagatcttctcaaggaacactacgttgacctgaagg
accgtccattctttgccggcctggtgaaatacatgcactcagggccggtagttgccatggtctgggaggggctgaatgtggtgaagac
gggccgagtcatgctcggggagaccaaccctgcagactccaagcctgggaccatccgtggagacttctgcatacaagttggcagga
acattatacatggcggtgattctgtggagagtgcagagaaggagatcggcttgtggtttcaccctgaggaactggtagattacacgagc
tgtgctcagaactggatctatgaactcgagcaccaccaccaccaccactga (SEQ ID NO:61)
(amino acid)

MANCERTFIAIKPDGVQRGLVGEIIKRFEQKGFRLVGLKFMQASEDLLKEH
YVDLKDRPFFAGLVKYMHSGPVVAMVWEGLNVVKTGRVMLGETNPADSKPGTIRG

DFCIQVGRNIIHGGDSVESAEKEIGLWFHPEELVDYTSCAQNWIYELEHHHHHH-
(SEQ ID NO:62)

### NM23 S120G (cloned between NdeI and AgeI) tagged with histidine cluster at the C-terminus

atggccaactgtgagcgtaccttcattgcgatcaaaccagatggggtccagcggggtcttgtgggagagattatcaag
cgttttgagcagaaaggattccgccttgttggtctgaaattcatgcaagcttccgaagatcttctcaaggaacactacgttgacctgaagg
accgtccattctttgccggcctggtgaaatacatgcactcagggccggtagttgccatggtctgggaggggctgaatgtggtgaagac
gggccgagtcatgctcggggagaccaaccctgcagactccaagcctgggaccatccgtggagacttctgcatacaagttggcagga
acattatacatggcggtgattctgtggagagtgcagagaaggagatcggcttgtggtttcaccctgaggaactggtagattacacgagc
tgtgctcagaactggatctatgaaaccggtcaccaccaccaccaccactga (SEQ ID NO:63)

MANCERTFIAIKPDGVQRGLVGEIIKRFEQKGFRLVGLKFMQASEDLLKEH
YVDLKDRPFFAGLVKYMHSGPVVAMVWEGLNVVKTGRVMLGETNPADSKPGTIRG
DFCIQVGRNIIHGGDSVESAEKEIGLWFHPEELVDYTSCAQNWIYETGHHHHHH-
(SEQ ID NO:64)

### NM23 P96S (cloned between NdeI and AgeI) tagged with histidine cluster at the C-terminus

(DNA) 
atggccaactgtgagcgtaccttcattgcgatcaaaccagatggggtccagcggggtcttgtgggagagattatcaag
cgttttgagcagaaaggattccgccttgttggtctgaaattcatgcaagcttccgaagatcttctcaaggaacactacgttgacctgaagg
accgtccattctttgccggcctggtgaaatacatgcactcagggccggtagttgccatggtctgggaggggctgaatgtggtgaagac
gggccgagtcatgctcggggagaccaactctgcagactccaagcctgggaccatccgtggagacttctgcatacaagttggcagga
acattatacatggcagtgattctgtggagagtgcagagaaggagatcggcttgtggtttcaccctgaggaactggtagattacacgagc
tgtgctcagaactggatctatgaaaccggtcaccaccaccaccaccactga (SEQ ID NO:65)
(amino acid)

MANCERTFIAIKPDGVQRGLVGEIIKRFEQKGFRLVGLKFMQASEDLLKEH
YVDLKDRPFFAGLVKYMHSGPVVAMVWEGLNVVKTGRVMLGETNSADSKPGTIRG
DFCIQVGRNIIHGSDSVESAEKEIGLWFHPEELVDYTSCAQNWIYETGHHHHHH-
(SEQ ID NO:66)

### NM23 P96S (cloned between NdeI and XhoI) tagged with histidine cluster at the C-terminus

(DNA) 
atggccaactgtgagcgtaccttcattgcgatcaaaccagatggggtccagcggggtcttgtgggagagattatcaag
cgttttgagcagaaaggattccgccttgttggtctgaaattcatgcaagcttccgaagatcttctcaaggaacactacgttgacctgaagg
 
accgtccattctttgccggcctggtgaaatacatgcactcagggccggtagttgccatggtctgggaggggctgaatgtggtgaagac
gggccgagtcatgctcggggagaccaactctgcagactccaagcctgggaccatccgtggagacttctgcatacaagttggcagga
acattatacatggcagtgattctgtggagagtgcagagaaggagatcggcttgtggtttcaccctgaggaactggtagattacacgagc
tgtgctcagaactggatctatgaactcgagcaccaccaccaccaccactga (SEQ ID NO:115)
(amino acids)

MANCERTFIAIKPDGVQRGLVGEIIKRFEQKGFRLVGLKFMQASEDLLKEH
YVDLKDRPFFAGLVKYMHSGPVVAMVWEGLNVVKTGRVMLGETNSADSKPGTIRG
DFCIQVGRNIIHGSDSVESAEKEIGLWFHPEELVDYTSCAQNWIYELEHHHHHH-
(SEQ ID NO:116)

### NM23 P96S/S120G (cloned between NdeI and AgeI) tagged with histidine cluster at the C-terminus

(DNA) 
atggccaactgtgagcgtaccttcattgcgatcaaaccagatggggtccagcggggtcttgtgggagagattatcaag
cgttttgagcagaaaggattccgccttgttggtctgaaattcatgcaagcttccgaagatcttctcaaggaacactacgttgacctgaagg
accgtccattctttgccggcctggtgaaatacatgcactcagggccggtagttgccatggtctgggaggggctgaatgtggtgaagac
gggccgagtcatgctcggggagaccaactctgcagactccaagcctgggaccatccgtggagacttctgcatacaagttggcagga
acattatacatggcggtgattctgtggagagtgcagagaaggagatcggcttgtggtttcaccctgaggaactggtagattacacgagc
tgtgctcagaactggatctatgaaaccggtcaccaccaccaccaccactga (SEQ ID NO:67)
(amino acid)

MANCERTFIAIKPDGVQRGLVGEIIKRFEQKGFRLVGLKFMQASEDLLKEH
YVDLKDRPFFAGLVKYMHSGPVVAMVWEGLNVVKTGRVMLGETNSADSKPGTIRG
DFCIQVGRNIIHGGDSVESAEKEIGLWFHPEELVDYTSCAQNWIYETGHHHHHH-
(SEQ ID NO:68)

### NM23 P96S/S120G (cloned between NdeI and XhoI) tagged with histidine cluster at the C-terminus

(DNA) 
atggccaactgtgagcgtaccttcattgcgatcaaaccagatggggtccagcggggtcttgtgggagagattatcaag
cgttttgagcagaaaggattccgccttgttggtctgaaattcatgcaagcttccgaagatcttctcaaggaacactacgttgacctgaagg
accgtccattctttgccggcctggtgaaatacatgcactcagggccggtagttgccatggtctgggaggggctgaatgtggtgaagac
gggccgagtcatgctcggggagaccaactctgcagactccaagcctgggaccatccgtggagacttctgcatacaagttggcagga
acattatacatggcggtgattctgtggagagtgcagagaaggagatcggcttgtggtttcaccctgaggaactggtagattacacgagc
tgtgctcagaactggatctatgaactcgagcaccaccaccaccaccactga (SEQ ID NO:117)
(amino acids)

MANCERTFIAIKPDGVQRGLVGEIIKRFEQKGFRLVGLKFMQASEDLLKEH
YVDLKDRPFFAGLVKYMHSGPVVAMVWEGLNVVKTGRVMLGETNSADSKPGTIRG
DFCIQVGRNIIHGGDSVESAEKEIGLWFHPEELVDYTSCAQNWIYELEHHHHHH-
(SEQ ID NO:118)

### NM23 P96SΔC1 (cloned between NdeI and AgeI) tagged with histidine cluster at the C-terminus

(DNA) 
atggccaactgtgagcgtaccttcattgcgatcaaaccagatggggtccagcggggtcttgtgggagagattatcaag
cgttttgagcagaaaggattccgccttgttggtctgaaattcatgcaagcttccgaagatcttctcaaggaacactacgttgacctgaagg
accgtccattctttgccggcctggtgaaatacatgcactcagggccggtagttgccatggtctgggaggggctgaatgtggtgaagac
gggccgagtcatgctcggggagaccaactctgcagactccaagcctgggaccatccgtggagacttctgcatacaagttggcagga
acattatacatggcagtgattctgtggagagtgcagagaaggagatcggcttgtggtttcaccctgaggaactggtagattacacgagc
tgtgctcagaactggatctataccggtcaccaccaccaccaccactga (SEQ ID NO:69)
(amino acid)

MANCERTFIAIKPDGVQRGLVGEIIKRFEQKGFRLVGLKFMQASEDLLKEH
YVDLKDRPFFAGLVKYMHSGPVVAMVWEGLNVVKTGRVMLGETNSADSKPGTIRG
DFCIQVGRNIIHGSDSVESAEKEIGLWFHPEELVDYTSCAQNWIYTGHHHHHH- (SEQ
ID NO:70)

### NM23 P96SΔC1 (cloned between NdeI and XhoI) tagged with histidine cluster at the C-terminus

(DNA) 
atggccaactgtgagcgtaccttcattgcgatcaaaccagatggggtccagcggggtcttgtgggagagattatcaag
cgttttgagcagaaaggattccgccttgttggtctgaaattcatgcaagcttccgaagatcttctcaaggaacactacgttgacctgaagg
accgtccattctttgccggcctggtgaaatacatgcactcagggccggtagttgccatggtctgggaggggctgaatgtggtgaagac
gggccgagtcatgctcggggagaccaactctgcagactccaagcctgggaccatccgtggagacttctgcatacaagttggcagga
acattatacatggcagtgattctgtggagagtgcagagaaggagatcggcttgtggtttcaccctgaggaactggtagattacacgagc
tgtgctcagaactggatctatctcgagcaccaccaccaccaccactga (SEQ ID NO:119)
(amino acids)

MANCERTFIAIKPDGVQRGLVGEIIKRFEQKGFRLVGLKFMQASEDLLKEH
YVDLKDRPFFAGLVKYMHSGPVVAMVWEGLNVVKTGRVMLGETNSADSKPGTIRG
DFCIQVGRNIIHGSDSVESAEKEIGLWFHPEELVDYTSCAQNWIYLEHHHHHH- (SEQ
ID NO:120)

### NM23 P96SΔC2 (cloned between NdeI and AgeI) tagged with histidine cluster at the C-terminus

(DNA) 
atggccaactgtgagcgtaccttcattgcgatcaaaccagatggggtccagcggggtcttgtgggagagattatcaag
cgttttgagcagaaaggattccgccttgttggtctgaaattcatgcaagcttccgaagatcttctcaaggaacactacgttgacctgaagg
accgtccattctttgccggcctggtgaaatacatgcactcagggccggtagttgccatggtctgggaggggctgaatgtggtgaagac
gggccgagtcatgctcggggagaccaactctgcagactccaagcctgggaccatccgtggagacttctgcatacaagttggcagga
acattatacatggcagtgattctgtggagagtgcagagaaggagatcggcttgtggtttcaccctgaggaactggtagattacacgagc
tgtgctcagaactggatcaccggtcaccaccaccaccaccactga (SEQ ID NO:71)
(amino acid)

MANCERTFIAIKPDGVQRGLVGEIIKRFEQKGFRLVGLKFMQASEDLLKEH
YVDLKDRPFFAGLVKYMHSGPVVAMVWEGLNVVKTGRVMLGETNSADSKPGTIRG
DFCIQVGRNIIHGSDSVESAEKEIGLWFHPEELVDYTSCAQNWITGHHHHHH- (SEQ
ID NO:72)

### NM23 P96SΔC2 (cloned between NdeI and XhoI) tagged with histidine cluster at the C-terminus

(DNA) 
atggccaactgtgagcgtaccttcattgcgatcaaaccagatggggtccagcggggtcttgtgggagagattatcaag
cgttttgagcagaaaggattccgccttgttggtctgaaattcatgcaagcttccgaagatcttctcaaggaacactacgttgacctgaagg
accgtccattctttgccggcctggtgaaatacatgcactcagggccggtagttgccatggtctgggaggggctgaatgtggtgaagac
gggccgagtcatgctcggggagaccaactctgcagactccaagcctgggaccatccgtggagacttctgcatacaagttggcagga
acattatacatggcagtgattctgtggagagtgcagagaaggagatcggcttgtggtttcaccctgaggaactggtagattacacgagc
tgtgctcagaactggatcctcgagcaccaccaccaccaccactga (SEQ ID NO:121)
(amino acids)

MANCERTFIAIKPDGVQRGLVGEIIKRFEQKGFRLVGLKFMQASEDLLKEH
YVDLKDRPFFAGLVKYMHSGPVVAMVWEGLNVVKTGRVMLGETNSADSKPGTIRG
DFCIQVGRNIIHGSDSVESAEKEIGLWFHPEELVDYTSCAQNWILEHHHHHH- (SEQ
ID NO:122)

### NM23 P96SΔC6 (cloned between NdeI and AgeI) tagged with histidine cluster at the C-terminus

(DNA) 
atggccaactgtgagcgtaccttcattgcgatcaaaccagatggggtccagcggggtcttgtgggagagattatcaag
cgttttgagcagaaaggattccgccttgttggtctgaaattcatgcaagcttccgaagatcttctcaaggaacactacgttgacctgaagg
 
accgtccattctttgccggcctggtgaaatacatgcactcagggccggtagttgccatggtctgggaggggctgaatgtggtgaagac
gggccgagtcatgctcggggagaccaactctgcagactccaagcctgggaccatccgtggagacttctgcatacaagttggcagga
acattatacatggcagtgattctgtggagagtgcagagaaggagatcggcttgtggtttcaccctgaggaactggtagattacacgagc
tgtgctaccggtcaccaccaccaccaccactga (SEQ ID NO:73)
(amino acid)

MANCERTFIAIKPDGVQRGLVGEIIKRFEQKGFRLVGLKFMQASEDLLKEH
YVDLKDRPFFAGLVKYMHSGPVVAMVWEGLNVVKTGRVMLGETNSADSKPGTIRG
DFCIQVGRNIIHGSDSVESAEKEIGLWFHPEELVDYTSCATGHHHHHH- (SEQ ID
NO:74)

### NM23 P96SΔC6 (cloned between NdeI and XhoI) tagged with histidine cluster at the C-terminus

(DNA) 
atggccaactgtgagcgtaccttcattgcgatcaaaccagatggggtccagcggggtcttgtgggagagattatcaag
cgttttgagcagaaaggattccgccttgttggtctgaaattcatgcaagcttccgaagatcttctcaaggaacactacgttgacctgaagg
accgtccattctttgccggcctggtgaaatacatgcactcagggccggtagttgccatggtctgggaggggctgaatgtggtgaagac
gggccgagtcatgctcggggagaccaactctgcagactccaagcctgggaccatccgtggagacttctgcatacaagttggcagga
acattatacatggcagtgattctgtggagagtgcagagaaggagatcggcttgtggtttcaccctgaggaactggtagattacacgagc
tgtgctctcgagcaccaccaccaccaccactga (SEQ ID NO:123)
(amino acids)

MANCERTFIAIKPDGVQRGLVGEIIKRFEQKGFRLVGLKFMQASEDLLKEH
YVDLKDRPFFAGLVKYMHSGPVVAMVWEGLNVVKTGRVMLGETNSADSKPGTIRG
DFCIQVGRNIIHGSDSVESAEKEIGLWFHPEELVDYTSCALEHHHHHH- (SEQ ID
NO:124)

### NM23 P96SΔC1 /S120G (cloned between NdeI and AgeI) tagged with histidine cluster at the C-terminus

(DNA) 
atggccaactgtgagcgtaccttcattgcgatcaaaccagatggggtccagcggggtcttgtgggagagattatcaag
cgttttgagcagaaaggattccgccttgttggtctgaaattcatgcaagcttccgaagatcttctcaaggaacactacgttgacctgaagg
accgtccattctttgccggcctggtgaaatacatgcactcagggccggtagttgccatggtctgggaggggctgaatgtggtgaagac
gggccgagtcatgctcggggagaccaactctgcagactccaagcctgggaccatccgtggagacttctgcatacaagttggcagga
acattatacatggcggtgattctgtggagagtgcagagaaggagatcggcttgtggtttcaccctgaggaactggtagattacacgagc
tgtgctcagaactggatctataccggtcaccaccaccaccaccactga (SEQ ID NO:75)
(amino acid)

MANCERTFIAIKPDGVQRGLVGEIIKRFEQKGFRLVGLKFMQASEDLLKEH
YVDLKDRPFFAGLVKYMHSGPVVAMVWEGLNVVKTGRVMLGETNSADSKPGTIRG
DFCIQVGRNIIHGGDSVESAEKEIGLWFHPEELVDYTSCAQNWIYTGHHHHHH- (SEQ
ID NO:76)

### NM23 P96SΔC1 /S120G (cloned between NdeI and XhoI) tagged with histidine cluster at the C-terminus

(DNA) 
atggccaactgtgagcgtaccttcattgcgatcaaaccagatggggtccagcggggtcttgtgggagagattatcaag
cgttttgagcagaaaggattccgccttgttggtctgaaattcatgcaagcttccgaagatcttctcaaggaacactacgttgacctgaagg
accgtccattctttgccggcctggtgaaatacatgcactcagggccggtagttgccatggtctgggaggggctgaatgtggtgaagac
gggccgagtcatgctcggggagaccaactctgcagactccaagcctgggaccatccgtggagacttctgcatacaagttggcagga
acattatacatggcggtgattctgtggagagtgcagagaaggagatcggcttgtggtttcaccctgaggaactggtagattacacgagc
tgtgctcagaactggatctatctcgagcaccaccaccaccaccactga (SEQ ID NO:125)
(amino acids)

MANCERTFIAIKPDGVQRGLVGEIIKRFEQKGFRLVGLKFMQASEDLLKEH
YVDLKDRPFFAGLVKYMHSGPVVAMVWEGLNVVKTGRVMLGETNSADSKPGTIRG
DFCIQVGRNIIHGGDSVESAEKEIGLWFHPEELVDYTSCAQNWIYLEHHHHHH- (SEQ
ID NO:126)

### NM23 P96SΔC2 /S120G (cloned between NdeI and AgeI) tagged with histidine cluster at the C-terminus

(DNA) 
atggccaactgtgagcgtaccttcattgcgatcaaaccagatggggtccagcggggtcttgtgggagagattatcaag
cgttttgagcagaaaggattccgccttgttggtctgaaattcatgcaagcttccgaagatcttctcaaggaacactacgttgacctgaagg
accgtccattctttgccggcctggtgaaatacatgcactcagggccggtagttgccatggtctgggaggggctgaatgtggtgaagac
gggccgagtcatgctcggggagaccaactctgcagactccaagcctgggaccatccgtggagacttctgcatacaagttggcagga
acattatacatggcggtgattctgtggagagtgcagagaaggagatcggcttgtggtttcaccctgaggaactggtagattacacgagc
tgtgctcagaactggatcaccggtcaccaccaccaccaccactga (SEQ ID NO:77)
(amino acid)

MANCERTFIAIKPDGVQRGLVGEIIKRFEQKGFRLVGLKFMQASEDLLKEH
YVDLKDRPFFAGLVKYMHSGPVVAMVWEGLNVVKTGRVMLGETNSADSKPGTIRG
DFCIQVGRNIIHGGDSVESAEKEIGLWFHPEELVDYTSCAQNWITGHHHHHH- (SEQ
ID NO:78)

### NM23 P96SΔC2 /S120G (cloned between NdeI and XhoI) tagged with histidine cluster at the C-terminus

(DNA) 
atggccaactgtgagcgtaccttcattgcgatcaaaccagatggggtccagcggggtcttgtgggagagattatcaag
cgttttgagcagaaaggattccgccttgttggtctgaaattcatgcaagcttccgaagatcttctcaaggaacactacgttgacctgaagg
accgtccattctttgccggcctggtgaaatacatgcactcagggccggtagttgccatggtctgggaggggctgaatgtggtgaagac
gggccgagtcatgctcggggagaccaactctgcagactccaagcctgggaccatccgtggagacttctgcatacaagttggcagga
acattatacatggcggtgattctgtggagagtgcagagaaggagatcggcttgtggtttcaccctgaggaactggtagattacacgagc
tgtgctcagaactggatcctcgagcaccaccaccaccaccactga (SEQ ID NO:127)
(amino acids)

MANCERTFIAIKPDGVQRGLVGEIIKRFEQKGFRLVGLKFMQASEDLLKEH
YVDLKDRPFFAGLVKYMHSGPVVAMVWEGLNVVKTGRVMLGETNSADSKPGTIRG
DFCIQVGRNIIHGGDSVESAEKEIGLWFHPEELVDYTSCAQNWILEHHHHHH- (SEQ
ID NO:128)

### NM23 P96SΔC6 /S120G (cloned between NdeI and AgeI) tagged with histidine cluster at the C-terminus

(DNA) 
atggccaactgtgagcgtaccttcattgcgatcaaaccagatggggtccagcggggtcttgtgggagagattatcaag
cgttttgagcagaaaggattccgccttgttggtctgaaattcatgcaagcttccgaagatcttctcaaggaacactacgttgacctgaagg
accgtccattctttgccggcctggtgaaatacatgcactcagggccggtagttgccatggtctgggaggggctgaatgtggtgaagac
gggccgagtcatgctcggggagaccaactctgcagactccaagcctgggaccatccgtggagacttctgcatacaagttggcagga
acattatacatggcggtgattctgtggagagtgcagagaaggagatcggcttgtggtttcaccctgaggaactggtagattacacgagc
tgtgctaccggtcaccaccaccaccaccactga (SEQ ID NO:79)
(amino acid)

MANCERTFIAIKPDGVQRGLVGEIIKRFEQKGFRLVGLKFMQASEDLLKEH
YVDLKDRPFFAGLVKYMHSGPVVAMVWEGLNVVKTGRVMLGETNSADSKPGTIRG
DFCIQVGRNIIHGGDSVESAEKEIGLWFHPEELVDYTSCATGHHHHHH- (SEQ ID
NO:80)

### NM23 P96SΔC6 /S120G (cloned between NdeI and XhoI) tagged with histidine cluster at the C-terminus

(DNA) 
atggccaactgtgagcgtaccttcattgcgatcaaaccagatggggtccagcggggtcttgtgggagagattatcaag
cgttttgagcagaaaggattccgccttgttggtctgaaattcatgcaagcttccgaagatcttctcaaggaacactacgttgacctgaagg
 
accgtccattctttgccggcctggtgaaatacatgcactcagggccggtagttgccatggtctgggaggggctgaatgtggtgaagac
gggccgagtcatgctcggggagaccaactctgcagactccaagcctgggaccatccgtggagacttctgcatacaagttggcagga
acattatacatggcggtgattctgtggagagtgcagagaaggagatcggcttgtggtttcaccctgaggaactggtagattacacgagc
tgtgctctcgagcaccaccaccaccaccactga (SEQ ID NO:129)
(amino acids)

MANCERTFIAIKPDGVQRGLVGEIIKRFEQKGFRLVGLKFMQASEDLLKEH
YVDLKDRPFFAGLVKYMHSGPVVAMVWEGLNVVKTGRVMLGETNSADSKPGTIRG
DFCIQVGRNIIHGGDSVESAEKEIGLWFHPEELVDYTSCALEHHHHHH- (SEQ ID
NO:130)

### Linker Sequences:

**GS2 linker**
   5'-ggcggtggcggatccggcggtggcggatcc-3' (SEQ ID NO:81)
   GGGGSGGGGS (SEQ ID NO:82)
**GS3 linker**
   5'-ggcggtggcggatccggcggtggcggatccggcggtggcggatcc-3' (SEQ ID NO:83)
   GGGGSGGGGSGGGGS (SEQ ID NO:84)
**IgGlh no C linker (modified hinge portion of an Fc region of an antibody)**
   5'-gataaaacccatactaaaccgccaaaaccggcgccggaactgctgggtggtcctggtaccggt-3' (SEQ ID NO:85)
   DKTHTKPPKPAPELLGGPGTG (SEQ ID NO:86)
**IgG2ah no C linker (modified hinge portion of an Fc region of an antibody)**
   5'-actggtggtccgactattaaacctccgaaacctccgaaacctgctccgaacctgctgggtggtccg-3' (SEQ ID NO:87)
   TGGPTIKPPKPPKPAPNLLGGP (SEQ ID NO:88)
**IgG1h/IgG2ah no C linker (combined hinge portions of two Fc regions of two different isotype antibodies** 
5'gataaaacccatactaaaccgccaaaaccggcgccggaactgctgggtggtcctggtaccggtactggtggtcc
gactattaaacctccgaaacctccgaaacctgctccgaacctgctgggtggtccg-3' (SEQ ID NO:89)
 DKTHTKPPKPAPELLGGPGTGTGGPTIKPPKPPKPAPNLLGGP (SEQ ID NO:90)
**Other Examples of Sequences are as Follows:**
   (DNA) (ggtggttctggt)n (n=1 to 3) (other DNA sequences are possible depending on the codon used for each amino acid) (SEQ ID NO:131)
   (corresponding amino acid sequence) (GGSG)n (n=1 to 3) (SEQ ID NO: 132)
   (DNA) gct(gaagctgctgctaaa)nGCT (other DNA sequences are possible depending on the codon used for each amino acid) (SEQ ID NO:133)
   (corresponding amino acid sequence) A(EAAAK)*ₙ*A (*n* = 2-5) (SEQ ID NO:134)
   (DNA) ggtgctggtggtgctggtggtgctggtgctggtggtgctggtgctggtgctggt (other DNA sequences are possible depending on the codon used for each amino acid) (SEQ ID NO: 135)
   (corresponding amino acid sequence) GAGGAGGAGAGGAGAGAG (SEQ ID NO:136)
   (DNA)
      ggtgctggtggtgctggtggtgctggtgctggtggtgctggtgctggtgctggtgaacttggtgctggtggtgctggtggtgctggtgct ggtggtgctggtgctggtgctggt (other DNA sequences are possible depending on the codon used for each amino acid) (SEQ ID NO:137)
   (corresponding amino acid sequence) GAGGAGGAGAGGAGAGAGELGAGGAGGAGAGGAGAGAG (SEQ ID NO:138)
   (DNA) ggtggtgctggtgctggtgctggt (other DNA sequences are possible depending on the codon used for each amino acid) (SEQ ID NO:139)
   (corresponding amino acid sequence) GGAGAGAG (SEQ ID NO :140)
   (DNA) ggttctggtggtggtggttctggtggtggtggttctggt (other DNA sequences are possible depending on the codon used for each amino acid) (SEQ ID NO:141)
   (corresponding amino acid sequence) GSGGGGSGGGGSG (SEQ ID NO:142)
   (DNA) cttgctgctgct (other DNA sequences are possible depending on the codon used for each amino acid) (SEQ ID NO:143)
   (corresponding amino acid sequence) LAAA (SEQ ID NO:144)
   (DNA) cttggtggtggtggttctggtggtggtggttctggtggtggtggttctgctgctgct (other DNA sequences are possible depending on the codon used for each amino acid) (SEQ ID NO: 145
   (corresponding amino acid sequence) LGGGGSGGGGSGGGGSAAA (SEQ ID NO: 146)
   (DNA)
   ctttctggtggtggtggttctggtggtggtggttctggtggtggtggttctggtggtggtggttctgctgctgct (other DNA sequences are possible depending on the codon used for each amino acid) (SEQ ID NO: 147)
   (corresponding amino acid sequence) LSGGGGSGGGGSGGGGSGGGGSAAA (SEQ ID NO:148)
   (DNA) cttgct(gaagctgctgctaaa)ngctgctgct (n=1 to 5) (other DNA sequences are possible depending on the codon used for each amino acid) (SEQ ID NO:149)
   (corresponding amino acid sequence) LA(EAAAK)nAAA (n=1 to 5) (SEQ ID NO: 150)
   (DNA)
      ctttttaataaagaacaacaaaatgctttttatgaaattcttcatcttcctaatcttaatgaagaacaacgtaatggttttattcaatctcttaaag atgatccttctcaatctgctaat (other DNA sequences are possible depending on the codon used for each amino acid) (SEQ ID NO:151)
   (corresponding amino acid sequence) LFNKEQQNAFYEILHLPNLNEEQRNGFIQSLKDDPSQSANLLAEAKKLNDAQAAA (SEQ ID NO:152)

### Sample Chimera Sequences:

**NM23 S120G IgGlFc (NM23-X connected to the Fc region of an antibody)** 
atggccaactgtgagcgtaccttcattgcgatcaaaccagatggggtccagcggggtcttgtgggagagattatcaag
cgttttgagcagaaaggattccgccttgttggtctgaaattcatgcaagcttccgaagatcttctcaaggaacactacgttgacctgaagg
accgtccattctttgccggcctggtgaaatacatgcactcagggccggtagttgccatggtctgggaggggctgaatgtggtgaagac
gggccgagtcatgctcggggagaccaaccctgcagactccaagcctgggaccatccgtggagacttctgcatacaagttggcagga
acattatacatggcggtgattctgtggagagtgcagagaaggagatcggcttgtggtttcaccctgaggaactggtagattacacgagc
tgtgctcagaactggatctatgaactcgagggttgtaagccttgcatatgtacagtcccagaagtatcatctgtcttcatcttccccccaaa
gcccaaggatgtgctcaccattactctgactcctaaggtcacgtgtgttgtggtagacatcagcaaggatgatcccgaggtccagttca
gctggtttgtagatgatgtggaggtgcacacagctcagacgcaaccccgggaggagcagttcaacagcactttccgctcagtcagtga
acttcccatcatgcaccaggactggctcaatggcaaggagttcaaatgcagggtcaacagtgcagctttccctgcccccatcgagaaa
accatctccaaaaccaaaggcagaccgaaggctccacaggtgtacaccattccacctcccaaggagcagatggccaaggataaagt
cagtctgacctgcatgataacagacttcttccctgaagacattactgtggagtggcagtggaatgggcagccagcggagaactacaag
aacactcagcccatcatggacacagatggctcttacttcgtctacagcaagctcaatgtgcagaagagcaactgggaggcaggaaata
ctttcacctgctctgtgttacatgagggcctgcacaaccaccatactgagaagagcctctcccactctcctggtaaactcgagcaccacc
accaccaccactga (SEQ ID NO:91)
 
MANCERTFIAIKPDGVQRGLVGEIIKRFEQKGFRLVGLKFMQASEDLLKEHYVDLKD
RPFFAGLVKYMHSGPVVAMVWEGLNVVKTGRVMLGETNPADSKPGTIRGDFCIQV
GRNIIHGGDSVESAEKEIGLWFHPEELVDYTSCAQNWIYELEGCKPCICTVPEVSSVFI
FPPKPKDVLTITLTPKVTCVVVDISKDDPEVQFSWFVDDVEVHTAQTQPREEQFNSTF
RSVSELPIMHQDWLNGKEFKCRVNSAAFPAPIEKTISKTKGRPKAPQVYTIPPPKEQM
 
AKDKVSLTCMITDFFPEDITVEWQWNGQPAENYKNTQPIMDTDGSYFVYSKLNVQK
SNWEAGNTFTCSVLHEGLHNHHTEKSLSHSPGKLEHHHHHH- (SEQ ID NO:92)
**NM23 S120G IgGlh (NM23-X connected to only modified hinge portion of the Fc region of an antibody)** 
atggccaactgtgagcgtaccttcattgcgatcaaaccagatggggtccagcggggtcttgtgggagagattatcaag
cgttttgagcagaaaggattccgccttgttggtctgaaattcatgcaagcttccgaagatcttctcaaggaacactacgttgacctgaagg
accgtccattctttgccggcctggtgaaatacatgcactcagggccggtagttgccatggtctgggaggggctgaatgtggtgaagac
gggccgagtcatgctcggggagaccaaccctgcagactccaagcctgggaccatccgtggagacttctgcatacaagttggcagga
acattatacatggcggtgattctgtggagagtgcagagaaggagatcggcttgtggtttcaccctgaggaactggtagattacacgagc
tgtgctcagaactggatctatgaaccggtgccacgtgattctggttgtaaaccgtgtatttgtgttggtctcgagcaccaccaccaccacc
actga (SEQ ID NO:93)
 
MANCERTFIAIKPDGVQRGLVGEIIKRFEQKGFRLVGLKFMQASEDLLKEH
YVDLKDRPFFAGLVKYMHSGPVVAMVWEGLNVVKTGRVMLGETNPADSKPGTIRG
DFCIQVGRNIIHGGDSVESAEKEIGLWFHPEELVDYTSCAQNWIYEPVPRDSGCKPCI
CVGLEHHHHHH (SEQ ID NO:94)
**NM23 S120G IgG2ah (NM23-X connected to only modified hinge portion of the Fc region of an antibody)**

atggccaactgtgagcgtaccttcattgcgatcaaaccagatggggtccagcggggtcttgtgggagagattatcaag
cgttttgagcagaaaggattccgccttgttggtctgaaattcatgcaagcttccgaagatcttctcaaggaacactacgttgacctgaagg
accgtccattctttgccggcctggtgaaatacatgcactcagggccggtagttgccatggtctgggaggggctgaatgtggtgaagac
gggccgagtcatgctcggggagaccaaccctgcagactccaagcctgggaccatccgtggagacttctgcatacaagttggcagga
acattatacatggcggtgattctgtggagagtgcagagaaggagatcggcttgtggtttcaccctgaggaactggtagattacacgagc
tgtgctcagaactggatctatgaaccgcgtggtccgaccattaaaccgtgtccaccgtgtaaatgtccaggtctcgagcaccaccacca
ccaccactga (SEQ ID NO:95)

MANCERTFIAIKPDGVQRGLVGEIIKRFEQKGFRLVGLKFMQASEDLLKEH
YVDLKDRPFFAGLVKYMHSGPVVAMVWEGLNVVKTGRVMLGETNPADSKPGTIRG
DFCIQVGRNIIHGGDSVESAEKEIGLWFHPEELVDYTSCAQNWIYEPRGPTIKPCPPCK
CPGLEHHHHHH- (SEQ ID NO:96)

The present disclosure is of methods for using proteins that preferentially form specific multimer(s), wherein the specific multimer has a desired biological function that the monomer or some other multimer of the protein does not have. For example, many growth factors exert their growth promoting function only when they are in the dimeric form. These may be homo or hetero-dimers. In these cases the methods described herein are used to enhance growth by increasing the amount of dimers of the growth factors. Conversely, if the desired biological function is to inhibit growth, for example, of cancer, then the growth factors would be engineered or mutants selected that resist dimer formation.

Methods that generate proteins or variants of the native protein that are more likely to exist in the desired multimeric form, that which results in a desired biological function, but which function is not conferred by the monomer or other multimeric form, can be used *in vitro, ex vivo* or *in vivo. In vitro,* the variant that prefers a specific multimerization state can be expressed and optionally purified, then used for cell culture, particularly for the culture of stem and progenitor cells, including hematopoietic stem and progenitor cells and other immature cells of the bone marrow or other *in vitro* uses. **In the invention** the parent protein is NM23 and the multimer-specific variant prefers dimer formation, optionally inhibits formation of the tetramer or hexamer and otherwise increases the amount of dimer that is present over a wide range of protein concentrations. In some cases, it may be desirable to recombinantly synthesize the multimer-specific variant with a leader sequence that increases the proteins entrance into the cell.

Alternatively, nucleic acids encoding the multimer-specific variant can be introduced into a cell, using any one of a variety of methods known to those skilled in the art, such as homologous recombination, stable or transient transfection or transduction, viral transduction, such as by using lentiviruses or retroviruses, including self-inactivating vectors, including self- inactivating lentiviral vectors and self-inactivating retroviral vectors. In one embodiment, the cell is a stem cell or an iPS cell, which may be derived from the patient or from a donor. The resultant cells may be transplanted into a patient before or after *in vitro* expansion. In one case, nucleic acid manipulations are performed to correct a genetic abnormality in the cell that has also been transfected or transduced to express the multimer-specific variant.

In the invention**,** the protein is NM23 and the multimer-specific variant prefers dimerization and inhibits the formation of higher order multimers, with the net effect that the population of the expressed protein has an increased amount of dimer, over a wide range of concentrations, compared to the parent protein. In this way, the NM23 dimers, which are growth factors, will drive the expansion of the cell even after transplantation into the patient. The growth factor variant can be inserted into the cell so that it is only transiently expressed, for example by using a self-inactivating viral vector. Alternatively, the multimer-specific variant, i.e. an NM23 variant that prefers dimer formation, can be permanently inserted into the genome. Optionally, the cell can also be transfected or transduced to express MUC1, or a fragment of MUC1, including MUC1 chimeras, or MUC1*, wherein the extra cellular domain has been truncated to included essentially most or all of the PSMGFR sequence.

In some cases, including those in which cells are transfected with nucleic acids encoding a multimer-specific variant, it is desirable to modify the nucleic acid of the variant at the 5' end to include a leader sequence that causes the cell to secrete the expressed protein. Such sequences are known to those skilled in the art and commonly include sequences derived from antibodies, see Example 13.

The following are exemplary leader sequences that cause an expressed protein to be secreted from the cell. Any of these sequences or others known to those skilled in the art can be added to the nucleic acid sequence of the variant so that the expressed protein is secreted from the cell. Optionally, the sequence is added to the N-terminus or 5' of the nucleic acid sequence. In this way, the NM23 mutants, deletions, single chain variants and fusion protein chimeras can readily be used *in vitro* and *ex vivo,* as well as *in vivo.*

### Sequence for protein expression in E. coli periplasm

**pelB (pectate lyase B of Erwinia carotovora) leader sequence**
   (DNA)
   aaatatcttcttcctactgctgctgctggtcttcttcttcttgctgctcaacctgctatggct (SEQ ID NO:153) (other DNA sequences are possible depending on the codon used for each amino acid)
   (amino acids)
   KYLLPTAAAGLLLLAAQPAMA (SEQ ID NO:154)
**Sequences for protein secretion in mammalian cells**
**Human serum albumin signal peptide**
   (DNA)
   atgaaatgggttacttttatttctcttctttttcttttttcttctgcttattct (SEQ ID NO:155) (other DNA sequences are possible depending on the codon used for each amino acid)
   (amino acids)
   MKWVTFISLLFLFSSAYS (SEQ ID NO:156)
**Human Kappa light chain signal peptide**
   (DNA)
   atggattttcaagttcaaattttttcttttcttcttatttctgcttctgttattatgtctcgtggt (SEQ ID NO: 157) (other DNA sequences are possible depending on the codon used for each amino acid)
   (amino acids)
   MDFQVQIFSFLLISASVIMSRG (SEQ ID NO: 158)

Cells transfected or transduced to express the NM23 variant that prefers dimerization, can be somatic cell, a stem cell, including hematopoietic stem cells and progenitor cells, or iPS cells. In one embodiment, somatic cells such as fibroblasts or dermablasts are transduced with nucleic acids that encode an NM23 variant that prefers dimer formation. The NM23 variant nucleic acids may be transduced along with one or more genes that cause the host cell to revert to a more immature state. These other genes may include but not limited to Oct4, Sox2, Nanog, Klf4, Lin28 and c-Myc. The cells can be transfected or transduced to express the NM23 variant temporarily, such that it will drive the expansion of that cell type for a limited amount of time, or permanently using methods such as stable transfection or homologous recombination and the like.

The methods of described herein can act to provide a growth factor that is constitutively active or more active than the native growth factor to stimulate the proliferation of a specific population of cells, which may carry a genetic mutation or correction. In one embodiment, one recombinant variant that increases the percentage of a specific multimeric state of a growth factor is carried on the same nucleic acid, plasmid, or expression vector that carries the sequence of the corrected gene or gene to be expressed. In another instance , if it is desired that a gene be down-regulated, a multimer that does not stimulate growth may be used.

In the invention the growth factor is NM23 wherein the dimer form activates growth and higher order multimers such as tetramers and hexamers turn off the NM23 mediated pathway that stimulates growth and induces or maintains pluripotency. For example, NM23 dimers promote stem and progenitor cell growth and pluripotency and also promote the growth of cancer cells. The hexamer or tetramer form of NM23 does not promote stem or cancer cell growth. Therefore, methods that result in variants that prefer dimer formation are used to promote stem cell, progenitor cell and/or cancer cell growth. In the case of stem and progenitor cells, the higher order NM23 multimers can be used to induce differentiation.

Conversely, methods that result in variants that prefer tetramer, hexamer or higher order multimer formation are used to inhibit cancer cell growth. Alternatively, NM23 variants can be further modified to carry a toxin to target the killing of cancer cells. NM23 is a ligand of MUC1* which is present at the surface of many different cancer cells. One could use NM23 to target specifically cancer cells to deliver a drug or toxin to kill the targeted cells. One example is the use of the ribosome-inactivating protein called saporin. By itself, saporin is not able to enter the cell, but when coupled to another protein that binds to the cell surface, the saporin/protein complex can be internalized and is toxic to the cell.

Because NM23 activates the MUC1* growth factor receptor, MUC1 or truncated forms of MUC1, including MUC1* wherein the extra cellular domain includes primarily the sequence of the PSMGFR peptide, can be optionally expressed either *in vitro* or *in vivo* along with the NM23 variant. MUC1 or MUC1 truncations, or MUC1 variants can similarly be expressed from the same expression plasmid as NM23 and optionally a gene desired to be expressed.

In another case, the growth factor receptor itself is engineered to be in a specific multimerization state in order to either promote or inhibit growth. In a preferred embodiment, the growth factor receptor is MUC1* and the preferred multimer is a dimer.

One method of generating proteins that prefer a specific multimerization state is to identify mutants of that protein that prefer the formation of that specific multimerization state. For example, Table 1 lists some of the NM23 mutations that encourage the formation of dimers.

**Table 1. NM23 Example Mutants that Favor Dimer Formation**

| **Name** | **Description** | **Need to be Refolded for Significant Dimer Population** | **Theoretical molecular weight (KDa)** |
|---|---|---|---|
| NM23 S120G *aka S120G* | Serine 120 mutated to glycine | Yes | Monomer: 19.6 |
| | | "R" added to name if refolded | Dimer: 39.2 |
| NM23 P96S/S120G *aka P96S*/*S120G* | Proline 96 mutated to serine and serine 120 mutated to glycine | Yes | Monomer: 18 |
| | | | Dimer: 36 |
| NM23 P96SΔC1 *aka P96S*Δ*C1* | Proline 96 mutated to serine and last C terminal residue deleted | No | Monomer: 18 |
| | | | Dimer: 36 |
| NM23 P96SΔC2 *aka* P96SΔC2 | Proline 96 mutated to serine and the last 2 C terminal residue deleted | No | Monomer: 17.8 |
| | | | Dimer: 35.6 |
| NM23 P96SΔC6 *aka P96S*Δ*C6* | Proline 96 mutated to serine and the last 6 C terminal residues deleted | No | Monomer: 17.2 |
| | | | Dimer: 34.4 |
| NM23 P96SΔC1 /S120G *aka P96S*Δ*C1* /*S120G* | Proline 96 mutated to serine, serine 120 mutated to glycine and last C terminal residue deleted | Yes | Monomer: 18 |
| | | | Dimer: 36 |
| NM23 P96SΔC2 /S120G *aka P96S*Δ*C2* /*S120G* | Proline 96 mutated to serine, serine 120 mutated to glycine and the last 2 C terminal residue deleted | Yes | Monomer: 17.8 |
| | | | Dimer: 35.6 |
| NM23 P96SΔC6 /S120G *aka P96S*Δ*C6* /*S120G* | Proline 96 mutated to serine, serine 120 mutated to glycine and the last 6 C terminal residues deleted | Yes | Monomer: 17.2 |
| | | | Dimer: 34.4 |

Constructs for recombinant proteins are generated such that one or more monomers are already connected. For example single chain proteins wherein two or more monomers are connected either directly or indirectly via for example a linker that may vary in length or sequence to obtain the desired biological activity. Linkers can vary in length and sequence. In a preferred embodiment, the linker is 5-100 amino acids. In a more preferred embodiment the linker is 10-75 amino acids. In a yet more preferred embodiment, the linker is either 10 amino acids or 43 amino acids. Similarly, the sequence of a linker can vary from a flexible (GGGGS₎ₙ type linker to any sequence known or suspected to be flexible in a natural protein, including modifications of native sequences, wherein several mutations intended to increase flexibility or solubility are inserted. Table 2 lists some preferred linker sequences.

**Table 2. Example Linkers**

| **Name** | **Description** | **Single Chain** | **Need to be Refolded for Significant Dimer Population** | **Theoretical molecular weight (kDa)** |
|---|---|---|---|---|
| GSₙ | Monomer-(GGGGS)ₙ-monomer | Yes | No if NM23-S 120G; Depends on monomer sequence and concentration | n=2:∼36 |
| | | | | n=3:∼36.4 |
| | | | | n=6:∼37 |
| IgG1h no C | Modified IgG1 hinge region without cysteins | Yes | No if NM23-S 120G; Depends on monomer sequence and concentration | ∼37.5 |
| IgG2ah no C | modified IgG2a hinge region without cysteins | Yes | Depends on monomer sequence and concentration | ∼37.5 |
| IgG1h no C/IgG2ah no C | modified IgG1 and IgG2a hinge regions without cysteins | Yes | No if NM23-S 120G; Depends on monomer sequence and concentration | ∼39.6 |

| Cysteins are present in linkers below | | | | |
|---|---|---|---|---|
| IgG1Fc | IgG1 Fc region fused at the C-term of NM23 | No | Yes if NM23-S 120G; Depends on monomer sequence and concentration | Monomer: 43.5 |
| | | | | Dimer: 87 |
| IgG1h | IgG1 hinge region fused at the C-term of NM23 | No | Depends on monomer sequence and concentration | Monomer: 19.6 |
| | | | | Dimer: 39.2 |
| IgG2ah | IgG2a hinge region fused at the C-term of NM23 | No | Depends on monomer sequence and concentration | Monomer: 19.8 |
| | | | | Dimer: 39.6 |

Table 3 lists some preferred single chain constructs wherein two monomers are recombinantly connected via a linker.

**Table 3. Examples Of NM23 Variants That Favor Dimer Formation**

| **Name (NM23-X-linker)** | **Description** | **Single Chain** | **Theoretical molecular weight (KDa)** |
|---|---|---|---|
| NM23 S120G GS2 | two NM23 X linked by 2 repeats of (GGGGS) | Yes | -36 |
| NM23 P96S GS2 | | Yes | |
| NM23 P96S/S120G GS2 | | Yes | |
| NM23 P96SΔC1 GS2 | | Yes | |
| NM23 P96SΔC2 GS2 | | Yes | |
| NM23 P96SΔC6 GS2 | | Yes | |
| NM23 P96SAC1 /S120G GS2 | | Yes | |
| NM23 P96SΔC2 /S120G GS2 | | Yes | |
| NM23 P96SΔC6 /S120G GS2 | | Yes | |
| NM23 S120G GS3 | two NM23 X linked by 3 repeats of (GGGGS) | Yes | -36.4 |
| NM23 P96S GS3 | | Yes | |
| NM23 P96S/S120G GS3 | | Yes | |
| NM23 P96SAC1 GS3 | | Yes | |
| NM23 P96SΔC2 GS3 | | Yes | |
| NM23 P96SΔC6 GS3 | | Yes | |
| NM23 P96SAC1 /S120G GS3 | | Yes | |
| NM23 P96SΔC2 /S120G GS3 | | Yes | |
| NM23 P96SΔC6 /S120G GS3 | | Yes | |
| NM23 S120G IgG1h noC | two NM23 X linked by a modified IgG1 hinge region without cysteins | Yes | -37.5 |
| NM23 P96S IgG1h noC | | Yes | |
| NM23 P96S/S120G IgGlh noC | | Yes | |
| NM23 P96SAC1 IgG1h noC | | Yes | |
| NM23 P96SΔC2 IgGlh noC | | Yes | |
| NM23 P96SΔC6 IgGlh noC | | Yes | |
| NM23 P96SAC1 /S120G IgGlh noC | | Yes | |
| NM23 P96SΔC2 /S120G IgGlh noC | | Yes | |
| NM23 P96SΔC6 /S120G IgGlh noC | | Yes | |
| NM23 S120G IgG2ah noC | | Yes | |
| NM23 P96S IgG2ah noC | | Yes | |
| NM23 P96S/S120G IgG2ah noC | | Yes | |
| NM23 P96SAC1 IgG2ah noC | two NM23 X linked by a modified IgG2a hinge region without cysteins | Yes | -37.5 |
| NM23 P96SΔC2 IgG2ah noC | | Yes | |
| NM23 P96SAC6 IgG2ah noC | | Yes | |
| NM23 P96SAC1 /S120G IgG2ah noC | | Yes | |
| NM23 P96SΔC2 /S120G IgG2ah noC | | Yes | |
| NM23 P96SΔC6 /S120G IgG2ah noC | | Yes | |
| NM23 S120G IgG1h/IgG2ah noC | two NM23 X linked by a modified IgG1 and IgG2a hinge regions without cysteins | Yes | -39.6 |
| NM23 P96S IgG1h/IgG2ah noC | | Yes | |
| NM23 P96S/S120G IgG1h/IgG2ah noC | | Yes | |
| NM23 P96SAC1 IgG1h/IgG2ah noC | | Yes | |
| NM23 P96SΔC2 IgG1h/IgG2ah noC | | Yes | |
| NM23 P96SΔC6 IgG1h/IgG2ah noC | | Yes | |
| NM23 P96SΔC1 IS 120G IgG1h/IgG2ah noC | | Yes | |
| NM23 P96SΔC2 /S120G IgG2ah noC | | Yes | |
| NM23 P96SΔC6 /S120G IgG2ah noC | | Yes | |

In some cases, the linker sequence itself tends to form homo or hetero dimers. For example, portions of the Fc region of antibodies dimerize with another Fc region. Chimeric proteins consisting of a portion of the parent protein plus a portion of a protein that naturally dimerizes are variants that prefer dimer formation. Alternatively, chimeras that use Fc sequences from IgM proteins would prefer the formation of higher order multimers such as the characteristic pentamer. Modified hinge regions of antibodies fused to NM23 mutant monomers preferentially form dimers and are described in detail herein.

In another approach, protein dimers are achieved by genetically making the protein of interest a fusion chimera wherein it is fused to a protein or protein fragment that has a dimerization domain. Ligands that are multimeric can be generated by making recombinant chimeras wherein each monomer is connected to a portion of a protein that multimerizes. For example, the proteins Fos and Jun interact so that they could be recombinantly connected to ligands that may be the same or different in order to cause the dimerization of the resultant chimeras. Table 3 lists preferred constructs, some of which are chimeras.

Cysteines can be inserted into the protein of interest such that dimer formation is encouraged via formation of disulfide bonds. The invention also includes inserting cysteines into linkers, whether comprised of natural amino acids or unnatural polymers or small molecules, to facilitate dimerization. Table 4 contains a list of preferred variants, whose dimerization is enhanced by the introduction of cysteines.

**Table 4. Example of dimer formation via disulfide bond formation between the hinge region of antibodies**

| **Name** | **Description** | **Single Chain** | **Theoretical molecular weight (KDa)** |
|---|---|---|---|
| NM23 S120G | IgG1 Fc region fused at the C-term of NM23 | no | Monomer: 43.5 |
| IgGlFc | | | Dimer: 87 |
| NM23 S120G IgGlh | IgG1 hinge region fused at the C-term of NM23 | no | Monomer: 19.6 |
| | | | Dimer: 39.2 |
| NM23 S120G | IgG2a hinge region fused at the C-term of NM23 | no | Monomer: 19.8 |
| IgG2ah | | | Dimer: 39.6 |

Yet another method for making ligand multimers is via chemical coupling of two or more monomeric ligands. For example, a bifunctional linker can be used to chemically couple two protein ligands to make homo or hetero dimers. The linker can be a chemical cross linker or similar that facilitates dimerization via covalent coupling of two monomers to form homo or hetero dimers.

The same can be accomplished by chemically coupling the proteins when they are in dimeric state, for example, immobilizing target protein in a defined geometry, for example on a SAM and chemically coupling the proteins either directly or indirectly via a linker while they are confined in a geometry that mimics dimerization state. Alternatively, dimers can be isolated then a coupling agent is added to directly couple two proteins together either directly or via a linker.

Yet another method for making multimeric ligands is to identify small molecules that bind to the target receptor and then synthesize multimers of the small molecule.

In the invention the multimerization state that is preferred for enhancing a natural biological interaction, such as the activity of a growth factor, is a dimer. NM23 isoforms H1 and H2 are preferred, with H1 especially preferred. In a yet more preferred embodiment, the NM23 is human. Also preferred for use with methods for increasing growth factor activity are mutants that enhance dimerization and optimally resist formation of higher order multimers. For example, the NM23-S120G and NM23-P96S mutants have been reported to prefer dimer formation and to resist the formation of tetramers and hexamers which do not activate the MUC1* growth factor receptor and do not bind to nucleic acids to induce expression of genes involved in pluripotency and cancer. However, for NM23-S120G mutant, we discovered that it is preferred that the protein be denatured and refolded to obtain significant populations of dimer. Similarly, the fraction of the P96S mutant that existed as a dimer was increased by denaturation and refolding. C-terminal deletions disrupt regions that participate in the formation of the higher order multimers and thus increase the portion of the protein that is in dimeric form. Thus C-terminal deletions of NM23 wild type or mutants is preferred for increasing the percentage and stability of dimer populations. C-terminal deletion of mutants such as S120G and/or P96S, that already prefer dimer formation are especially preferred. Deletion of 1-6 amino acids from the C-terminus of NM23 is especially preferred.

Although the S120G and P96S mutations are naturally occurring mutations identified in human cancer and in developmental abnormalities of the fruit fly, respectively, the invention also includes purposely introducing mutations and identifying those that either increase or decrease the propensity to form dimers. For example, to enhance growth factor activity wherein the growth factor is active when in dimeric form, mutations that prefer dimer formation are identified and preferred. For inhibiting growth factor activity, mutations that resist formation of dimers or prefer formation of tetramers or hexamers are preferred. Either site directed or random mutagenesis can be used wherein those that favor dimer formation are identified by a variety of methods including but not limited to structural analysis such as crystal structure, ability to support, induce or maintain pluripotency in stem cells, ability to bind to MUC1* peptide that includes essentially the PSMGFR peptide sequence. In addition, NM23 variants that favor dimer formation can be identified for example by using phage display and standard random mutagenesis wherein the desired mutants are identified by their ability to bind to stem cells or the MUC1* peptide.

Similarly, NM23 variants and similar multimer-specific variants of the invention can be further modified with sequences that increase the variant's ability to penetrate the cell membrane.

As will become evident, the ligand monomers that are genetically engineered to prefer dimer formation may be the wild type protein or a mutant or truncation that prefers dimer formation or resists the formation of higher order multimers.

As described herein ligands that are designed to form higher order multimers can interact with the wild type protein to inhibit the ability of the native protein to form dimers. For example, NM23 binds to the MUC1* growth factor receptor and induces dimerization which triggers growth, survival and pluripotency. NM23 can exist as a monomer, dimer, tetramer or hexamer, depending on its sequence and concentration. Recombinant NM23 can be refolded or purified such that populations of dimers can be isolated. Mutations of NM23 that prefer dimer formation and resist the formation of tetramers and hexamers have been isolated from human cancers. Therefore, an approach for the inhibition of cancerous growth would be to identify NM23 mutants that prefer the formation of higher order multimers, which do not induce growth and pluripotency. Especially preferred would be those mutants that are able to recruit wild type NM23 into their multimers so that they would not form the cancer-associated dimers.

It is known that MUC1* growth factor receptor is activated by ligand induced dimerization of its extra cellular domain. Bivalent antibodies raised against the extra cellular domain of MUC1* (PSMGFR sequence: GTINVHDVETQFNQYKTEAASRYNLTISDVSVSDVPFPFSAQSGA (SEQ ID NO:1) or GTINVHDVETQFNQYKTEAASPYNLTISDVSVSDVPFPFSAQSGA (SEQ ID NO:2) dimerize the MUC1* receptor and stimulate growth in a dose dependent manner. The dose response curve is a classic bell-shaped curve that is characteristic of Class I growth factor receptors that are activated by dimerization. The bell-shaped curve is caused when growth increases as dimerization of MUC1* increases but when the NM23 dimers or the bivalent antibodies are in excess, each receptor is bound by one, rather than two growth factors. This actually blocks dimerization of the growth factor receptor and results in a decrease in growth. Consistent with these findings, the addition of the monovalent Fab of the anti-MUC1*_{ecd} antibody blocks receptor dimerization and consequently inhibits growth, see Figure 1. Similarly, NM23 in dimeric form stimulates the growth of MUC1* positive cells.

An NM23 mutant S120G was previously isolated from a human neuroblastoma. It was reported that the mutant NM23 preferred dimer formation and resisted the formation of higher order multimers, specifically the tetramers and hexamers that wild type NM23 is known to form. Other NM23 mutants that were reported to prefer dimerization were the P96S mutation and deletions at the C-terminus of 1-6 amino acids.

However, when NM23-WT (wild type), S120G or P96S mutants are made as recombinant proteins, their multimerization state depends on sequence, how it is expressed, how it is collected and purified, as well as concentration. For example, despite expressing the S120G mutant, reported to prefer dimer formation, many expression/purification methods resulted in populations comprised exclusively of tetramers and hexamers. Other methods of protein expression produced NM23 populations that were comprised of hexamer, tetramer and a small dimer population. One method for expressing and purifying NM23 mutants that results in significant populations of dimer involved denaturing the expressed protein and then refolding it. In an optional step, the dimer population was further purified by size exclusion chromatography (FPLC) as described in Example 4 herein.

Characterization of NM23-WT or three different preparations of mutant S120G was carried out by FPLC (Figure 2). FPLC show that wild type, NM23-WT, was comprised almost exclusively of hexamers, as was one of the preparation of the mutant, "NM23-_{S120G}-hexamer" S120G, wherein the soluble fraction of the expressed protein was used. Another preparation of the S120G mutant, "NM23-_{S120G}-mixed" in which the soluble fraction was also used, shows by FPLC that it is comprised of a mixture of multimers including about 60% dimer. Another preparation of mutant S120G was performed in which the expressed protein was denatured then refolded according to the method described in Example 3b. FPLC showed that preparation, "NM23-_{S120G}-dimer", was comprised only of dimer and monomer with the dimer portion being 80% of the population. Figure 3a shows that on a non-reducing gel, the NM23 dimer runs with an apparent molecular weight of about 40kDa, while both the monomer and hexamer run with an apparent molecular weight of about 20kDa, indicating that the dimer is stabilized by disulfide bonds while the hexamer is not.

Surface Plasmon Resonance (SPR) experiments were carried out to determine if there were differences in binding affinities of the various NM23s multimers (monomers, dimers and hexamers) to the MUC1* peptide. SPR measurements were taken on a Biacore 3000 instrument wherein a histidine-tagged MUC1* peptide (PSMGFR) was immobilized to saturation on an SPR chip that was coated with a self-assembled monolayer coated with 3.8% NTA-Ni in a background of tri-ethylene glycol terminated thiols, see Example 5, Figure 3b and Figure 5. Figure 3b shows an overlay of Surface Plasmon Resonance (SPR) measurements show vast differences in the ability of the four different NM23 preparations to bind to a MUC1* extra cellular domain peptide (PSMGFR) attached to the SPR chip surface. Results show that the amount of binding of NM23 to its cognate receptor, MUC1*, is a function of how much dimer is present in the sample. SPR measures protein mass at the chip-solution interface, so if the hexamer bound to the MUC1* peptide surface, it would yield an SPR signal 3-times greater than if a dimer bound. That means that the amount of NM23-WT or NM23-S120G-hexamer that bound to the MUC1* peptide was about 12-times less than the amount of NM23-S120G-dimer that bound. In control experiments, an irrelevant peptide was immobilized on the same chip and a minimal amount of background binding resulted, which was subtracted from the measurement shown. However, the amount of binding generated by the NM23-WT and NM23-S120G-hexamer (both comprised almost exclusively of hexamer) ~100RUs is often considered to be within the noise of the system.

To further test the ability of the various NM23 multimers to bind to its cognate receptor, we performed a nanoparticle experiment, see Example 6 and Figure 3c. In this experiment, gold nanoparticles are coated with self-assembled monolayers (SAMs) that have NTA-Ni-thiols incorporated into the SAM. The NTA-Ni moiety captures histidine-tagged proteins. If proteins immobilized on the nanoparticles recognize each other and draw the attached nanoparticles close together, an intrinsic property of the nano gold causes the solution to turn from the characteristic pink to blue. The same thing happens if a dimeric protein added in solution binds to particle-immobilized proteins. NM23-S120G-dimer, NM23-WT, or NM23-S120G-hexamer were separately added to nanoparticles bearing MUC1*_{ecd} peptides (PSMGFR). The photograph of Figure 3c shows that only the dimer form of NM23 binds to its cognate receptor MUC1* peptide, making the solution turn from pink to blue/gray. Neither the wild type NM23 nor the NM23-S120G mutant, which expressed as virtually all hexamer, bound at all to the MUC1* peptide and the solution remained pink and indistinguishable from the control "no protein", in which no NM23 protein was added. To ensure specificity of the observed interaction, the Fab of anti-MUC1* antibody, which was raised against the PSMGFR peptide, was added to competitively inhibit binding of ligands to the peptide. As can be seen in the figure, the Fab inhibited the interaction of NM23 dimers and MUC1* peptide, showing that it was specific binding.

All three batches of NM23, -WT, S120G-hexamer and S120G-dimer were tested for their ability to promote undifferentiated stem cell growth. Human embryonic stem cells were cultured in minimal stem cell media that contained 8nM of one of the NM23 preparations. In one of the wells the free MUC1*_{ecd} peptide (PSMGFR) was added to competitively inhibit binding of NM23-S 120G-dimers to the MUC1* receptor which is on all pluripotent stem cells. The results are shown in the photograph of Figure 3(d-g). Differentiating stem cells have a different morphology than pluripotent stem cells and appear as thickened, darkened areas of cells, whereas pluripotent stem cells grow in a single bright layer of cells. As can be seen in the figure, only the dimer preparation of NM23-S120G (d) was able to support the undifferentiated growth of stem cells. Wild type NM23 (f) caused the stem cells to begin differentiating after three days, while NM23-S120G-hexamers had even more differentiation (e). However, the greatest amount of differentiation occurred as the result of competitively inhibiting the interaction between NM23 dimers and the MUC1* receptor (g).

In another part of this experiment, levels of miR-145, which is the microRNA that signals the stem cell's exit from pluripotency, was measured. This experiment showed that the disruption of the interaction between the NM23 dimer and MUC1* caused a spike in miR-145, which further corroborated the finding that disruption of the interaction between NM23 dimers and MUC1* triggers differentiation and conversely the interaction promotes pluripotency. The proteins used in these experiments to determine their ability to support pluripotent stem cell growth were characterized at the time of the experiment by gel electrophoresis using a non-denaturing native gel. The native gel of Figure 4 shows that NM23-WT is comprised mostly of all hexamer, the NM23-S120G-hexamer prep has a small population of dimer but is comprised mostly of hexamer. The NM23-S120G-dimer prep is comprised mostly of dimer but has a small portion of tetramer. The "NM23-S120G-mixed" preparation that was shown by FPLC to be comprised of 60% dimer (see Figure 2) was tested along with NM23-WT comprised mostly of hexamer (Figure 2) to determine their ability to bind to the MUC1*_{ecd} peptide (PSMGFR) using Surface Plasmon Resonance (SPR). In this experiment, each protein, at five different concentrations, was separately flowed over a chip that was coated with the PSMGFR peptide. The overlay of SPR traces shown in Figure 5 shows that roughly 8-times more "NM23-S120G-mixed" protein bound to a MUC1* extra cellular domain peptide surface than NM23-WT. Because the wild type protein is a hexamer, the number of RUs must be divided by 3 to compare to the amount of dimer that bound. Although both wild type and S120G-dimer show a concentration dependence in binding, the amount of wild type hexamer that bound is so small that it may still be within the noise range of the system.

Therefore, NM23 mutants, deletion mutants and engineered variants that prefer dimer formation are ideal for the growth, maintenance and induction of pluripotency or multipotency, for example in somatic cells, as well as for the numerous applications disclosed herein. Nucleic acids encoding these variants can also be transfected into cells to promote the growth of these cells. Exemplary variants of proteins that prefer a specific dimerization state compared to the native protein were made, characterized and tested for their ability to function as the specific multimer. The NM23 variants that have increased populations of stable dimer compared to the wild type protein that were made and tested are listed in Tables 1, 3 and 4. Figure 27 also provides examples of such constructs. Aspects of their construction, expression and purification are described in Examples 2, 3 and 9. All resulted in an increase of stable dimer population compared to the wild type protein. Some of the variants were optionally refolded to increase the dimer population for *in vitro* uses. However, they did not require refolding and as expressed were able to carry out dimer-associated function such as supporting pluripotent stem cell growth and inhibition of differentiation. Figures 11-13, 15-19, and 24 show that without refolding, the NM23 variants mimic the dimer and function as well or better than NM23-S120G refolded dimers, based on the results of typical stem cell growth experiments that demonstrate that the variants carry out the desired function.

Tables 1, 3 and 4 list NM23 mutants that prefer dimer formation, engineered constructs wherein two NM23 wild type or mutant monomers are connected via a linker to form a dimer, and NM23 fusion proteins that preferentially form dimers. Table 3 lists and describes NM23 variants that were generated, expressed, characterized and tested for their ability to mimic the behavior of native NM23 dimers, and particularly to test their ability to mimic the behavior of NM23-S120G-dimers that were 80% or greater in dimer form. The methods used to generate the constructs, express the protein, and refold in some cases are described in Examples 2, 3, 9, and 10. Methods used to test the function of the variants produced are described in Examples 5-7 and 11-12.

Figure 6 shows photographs of non-reducing (a) and reducing (b) gels for a number of the NM23 variants described in Table 3. The reducing gel shows that these single chain constructs run with an apparent molecular weight that is approximately equal to the molecular weight of an NM23 dimer, The non-reducing gel shows that at very high concentrations, some higher order multimers exist but are eliminated by reducing conditions such as within a cell or by the addition of DTT.

Figure 7 shows PAGE analysis of the purification of two more single chain NM23 variants that are dimers. Figures 8 and 9 show gels that confirm expression and purification of NM23-S120G-IgG1 Fc, a variant that is not a single chain construct but preferentially forms dimers and is described in Table 4. Figure 10a shows the FPLC characterization of the NM23-S120G-IgG1 Fc variant and shows that a subpopulation of dimer is formed which can be increased by protein refolding and/or by FPLC purification of the dimer fractions, highlighted in the non-reducing gel of Figure 10b.

Pluripotent human stem cells were cultured in NM23 variants and their ability to promote undifferentiated stem cell growth and to inhibit spontaneous differentiation of the cells was determined. Both human BGO1v/hOG and H9 embryonic stem cells were cultured in minimal stem cell media plus 8nM of either our standard NM23-S120G-RS or an NM23 variant. In all cases, the NM23 variants tested fully supported pluripotent stem cell growth. Cell morphology was typical of undifferentiated stem cells and was devoid of thickened or darkened areas that denote differentiation, see Figures 11-13, 15-19.

In addition to assessing stem cell morphology as proof that the NM23 variants functioned as well as the native dimers or the S120G dimers, the growth rate of stem cells cultured in media containing the NM23 variants was compared to the growth rate of identical cells cultured in NM23-S120G "RS" that had been refolded and then purified by FPLC so that the isolated fractions were essentially 100% dimer, see Example 11c. In these experiments, 200,000 stem cells all drawn from the same source (human ES - BGO1v/hOG) were cultured in either NM23-S120G RS or one of the NM23 variants shown in Figures 20 and 21. Four days post plating, cells were harvested by trypsinization and cells were counted on a hemocytometer. As can be seen in the graphs, in every case, the variants produced more cells than the isolated dimer population of NM23-S120G ("RS").

As another method of assessing the function of the NM23 dimer preferring variants, quantitative PCR was performed to measure expression levels of the pluripotency genes in stem cells cultured in the NM23 variants, see Example 11d. Figure 22 compares expression levels of pluripotency genes Oct4 and Nanog plus MUC1 and NM23 in stem cells that had been cultured in the NM23 variants for at least 4 passages. The graph of Figure 22 shows that, for cells grown in the NM23 variants, the expression levels of these key indicators of pluripotency are the same or better than for cells cultured in NM23-S120G-RS, which is a population of pure dimers.

As a yet further measure of the function of engineered NM23 dimer preferring variants, their migration from cell surface to cell nucleus was tracked and compared to that of NM23-S120G-RS, see Example 11e and Figures 23-24. It is known that NM23 dimers mediate the growth of MUC1*-positive cancer cells and human pluripotent stem cells, which are all MUC1*-positive. When MUC1*-positive cancer cells are incubated in media that contains NM23 in dimer form, the NM23 dimers bind to the MUC1* receptor, become internalized and within 30-60 minutes are translocated to the nucleus where they likely function as transcription factors. Figure 23 shows confocal images of cancer cells incubated in the presence or absence of either 0, 16nM or 128nM NM23-S120G-RS (100% dimer population). Cells were then stained with the nuclear stain DAPI and an anti-NM23 antibody was then added to the cells and a fluorescently labeled secondary antibody. Note that endogenous NM23 is also stained by the antibodies. However, there is only detectable NM23 in the nucleus when it is added exogenously as a dimer. Optimal concentrations for enhancing cell growth and for nuclear localization for NM23-S120G were previously determined to be between 8nM and 64nM. At higher concentrations, each NM23 dimer binds to each MUC1* receptor rather than 1 dimer binding to and dimerizing two MUC1* receptors. (See bell-shaped curve of Figure 1 where bivalent anti-MUC1* antibody in excess inhibits rather than stimulates growth for the same reason.) Figure 23 (b, e) shows NM23 in the nucleus as indicated by the white arrows.

Figure 24 shows confocal images of the same experiment except that the NM23 that has been added exogenously is the single chain "dimer" variant NM23-S120G-IgG1h/IgG2ah noC, which is two NM23-S120G monomers linked by a modified IgG1 and IgG2a hinge regions, without cysteines, see Example 9e. As can be seen in Figure 24, the NM23 single chain variant readily translocates to the nucleus (b, e), as indicated by the white arrows. The corresponding graphs that quantify the amount of NM23 in the nucleus for NM23-S120G RS (Figure 23h) and for the single chain variant, NM23-S120G-IgG1h/IgG2ah noC (Figure 24h) show that the engineered "dimer" translocates to the nucleus better than NM23 monomers that have been isolated as a dimer population.

These experiments show that connecting two monomers with a linker or fusing the protein of interest with a portion of a strong dimerization domain, results in more stable "dimers" that function as dimers over a wide range of concentrations, whereas the multimerization state of native NM23 is highly dependent on concentration and exists as a dimer only at very low nanomolar concentrations. Mutant NM23 proteins that prefer dimerization are an improvement over the wild type protein for promoting growth factor function and pluripotency, but they vary greatly in the amount of dimer produced and in the stability of those dimers depending on sequence, method of expression and purification. NM23 variants that are either single chain constructs or fusion proteins like those listed in Tables 3 and 4 represent an improvement over the state of the art because they increase the portion of dimer formed, increase dimer stability, and importantly can be expressed as a pseudo dimer in a cell or an organism, where expression and refolding methods required to form dimers of mutants that "prefer dimer formation" could not be done.

Any of the mutants, deletions and/or single chain or fusion chimeras of the invention, including those described in Examples 2, 9 and 10 can be made to be secreted by expressing cells for use *in vitro, ex vivo* and/or *in vivo.* Sequences that cause expressed proteins to be secreted are known to those skilled in the art. Particularly, sequences derived from antibodies are added to the N-terminus of the protein or to the 5'end of the gene of interest. In addition to the inclusion of leader sequences, the expression cell type need not be limited to *E. coli* and also includes mammalian cells, mammalian expression cells, yeast, somatic cells, stem cells, iPS cells or cells undergoing induction of pluripotency or induction to a less mature state than the starting cell.

It is not intended that the use of NM23, mutants or variants thereof, be limited to use with human cells or in humans. MUC1* has great sequence homology among mammals as does NM23. Figure 25 shows that mouse embryonic stem cells grow as well in human NM23 dimers as they do in the standard mouse LIF.

Table 2 shows the various linkers that can be used with any ligand but with NM23 variants shown in Table 1 in preferred embodiments. Table 2 also lists the portions of proteins that were genetically fused to ligand monomers listed in Table 1 to form constitutively active forms of the protein, which in an especially preferred embodiment is NM23.

In addition to the S120G mutant that prefers dimer formation and resists the formation of the higher order multimers, there are other mutants and variants that also favor dimer formation or stabilization. For example, another NM23 mutant that is easier to express and maintain as stable dimer is the P96S mutation. Another approach to making variants that form or stabilize dimers is to delete regions of the protein that participate in the formation of the higher order multimers such as the tetramers and hexamers. For example, in NM23-H1, the C-terminus promotes formation of tetramers and hexamers. Deletions of 1-9 amino acids at the C-terminus are preferred for generating NM23 variants that have increased activity, including increased growth factor activity, increased binding to MUC1* growth factor receptor and/ or increased binding to nucleic acids that regulate expression of other pluripotency and multipotency genes.

In a preferred embodiment, 2 amino acids are deleted (CΔ2) from the C-terminus of human NM23-H1. In an especially preferred embodiment, 6 amino acids are deleted (CΔ6). To maximize solubility, expression of dimers or stabilization of dimers, the protein can be made with one or more mutations plus deletions. For example, one NM23 variant that, when expressed as the recombinant protein, has a soluble fraction that is mostly dimer is NM23-P96S-CΔ6. This NM23 is ideal for stimulation of stem or progenitor cell growth because it does not need to be denatured and refolded, has a major portion of the soluble fraction that is a dimer, and dimer populations that can be further purified by size exclusion chromatography remain stable for long periods of time.

Figure 14 c, d, e, and f show gel and FPLC trace of NM23-P96S-CΔ2 and NM23-P96S-CΔ6, respectively. Construct design and protein expression and purification are described in Example 2c. In addition, Figure 15 shows photos of human ES cells that have been propagated using NM23-P96S-CΔ2 and NM23-P96S-CΔ6 in minimal media. Figure 22 shows gene expression of these human stem cells after being propagated with this NM23 variant. Gene expression levels are compared to growth of the same human embryonic cell line in either: a) bFGF and conditioned media from fibroblast feeder cells; b) NM23-S120G refolded and purified as a homogeneous population of dimer; or c) NM23-P96S-CΔ6. These results confirm that stem cells cultured in both forms of NM23 are comparable at the genetic level.

Other NM23 variants listed in Tables 1, 3 and 4 were generated as described in Examples 2 and 9. Figures 11-13 and 15-19 show that they effectively promoted the growth of human stem cells. Because the membrane proximal regions of MUC1, namely the MUC1* or PSMGFR region and the self-aggregation domain (IBR), are highly conserved among mammals, we tested and confirmed that NM23 dimers also promote the growth and maintenance of mouse stem cells. The invention also includes that NM23 dimers and in particular the variants described herein will act to maintain as well as induce pluripotency and multi-potency in human as well as mouse cells.

Sequence alignment between human NM23-H1 and other mammalian homologues, including but not limited to mouse NM23, will elucidate comparable regions of the homologue protein that should be mutated or deleted.

### Growth of cancer cells

The NM23 variants described herein can also be used as a vehicle for drug delivery for the treatment of MUC1*-positive cancers. For example, cytotoxic agents can be chemically coupled to the NM23 or NM23 variant. Toxins may be genetically engineered such that they are attached to the NM23. Alternatively, the NM23 variant can be modified, for example with cysteine or with certain enzyme-specific sequences that facilitate specific coupling of therapeutic agents to the NM23 variants.

NM23 in dimeric form binds to the MUC1* receptor on stem and progenitor cells. Binding to MUC1* facilitates endocytosis of NM23 dimers after which they are translocated to the nucleus, where NM23 binds to DNA as a dimer to regulate transcription of genes involved in the growth and maintaining pluripotency as well as multi-potency of stem and progenitor cells. Therefore, in one aspect, the invention is directed to using the methods described herein to make NM23 variants that prefer dimer formation for use in the growth, maintenance and induction of pluripotency. That is, NM23 variants that prefer dimer formation can be used *in vitro* to promote the growth of stem and progenitor cells and to maintain pluripotency or multipotency. In addition, also described is the administration of these NM23 variants to a patient for the treatment of conditions that would benefit from treatment with immature cells, including stem and progenitor cells. NM23 and NM23 variants can be administered to a patient either systemically or locally.

### EXAMPLES

### Example 1. The MUC1* growth factor receptor is activated by ligand induced dimerization

Class I growth factor receptors are activated by ligand-induced dimerization of their extra cellular domain. To demonstrate that MUC1* is activated by ligand-induced dimerization of its extra cellular domain, we treated MUC1* positive cells, ZR75-30 breast cancer cells with either the bivalent anti-MUC1* antibody or the monovalent Fab of the same antibody. The graph of Figure 1 shows that the bivalent antibody stimulates growth until it is added at an excess concentration, when rather than ever bivalent antibody dimerizing two receptors, there is an antibody bound to each one receptor. This inhibits growth. The addition of the Fab of the same antibody caused inhibition of cell growth and induced cell death. MUC1*-negative HEK-293 cells (K293) were not affected by either the bivalent or the monovalent Fab of the anti-MUC1* antibody.

### Example 2. Generation of Protein Constructs

### Example 2a. NM23-WT

NM23wt was amplified by polymerase chain reaction (PCR) using the following primers:
Forward 5'-atcgatcatatggccaactgtgagcgtacctt-3' (SEQ ID NO:3)
Reverse 5'-gtggtgctcgagttcatagatccagttctga-3' (SEQ ID NO:4)

The fragment was then purified, digested (NdeI, XhoI) and cloned between NdeI and XhoI restriction sites of the expression vector pET21b.

### Example 2b. NM23-S120G

NM23-H1 mutant S120G (serine #120 mutated to a glycine) was made using the GeneTailor^{™} Site-directed mutagenesis system (Life Technologies) following the manufacturer instructions using the following primers: 5'-gcaggaacattatacatggcggtgattctg-3' (SEQ ID NO:5) and 5'-gccatgtataatgttcctgccaacttgtat-3' (SEQ ID NO:6). Figure 2 shows overlay of FPLC traces comparing multimerization state of the wild type protein to the non-refolded S120G mutant and the refolded S120G. Figures 3-5 show that only the dimeric form of the protein binds to MUC1* (not the hexamer) and only the dimer is able to support pluripotent stem cell growth. Figure 16 shows non-reducing SDS-PAGE characterization and corresponding FPLC trace for the expressed and refolded protein as well as photographs of human stem cells, showing the NM23-S 120G ability to support pluripotent stem cell growth.

### Example 2c. NM23 P96S and deletion constructs.

We generated the NM23-H1 mutant P96S (proline #96 mutated to a serine) using the QuickChange site-directed mutagenesis kit (Agilent) following the manufacturer instructions using the following primers: 5'- tcggggagaccaactctgcagactccaag -3' (SEQ ID NO:7) and 5'- cttggagtctgcagagttggtctccccga -3' (SEQ ID NO:8). The template used for the PCR reaction was NM23 wild type cloned between NdeI and XhoI restriction sites. After sequence confirmation, the deletion constructs were generated by PCR. NM23 P96S ΔC1 was amplified using the following primers: 5'- atcgatcatatggccaactgtgagcgtaccttc -3' (SEQ ID NO:9) and 5'-gtggtgaccggtatagatccagttctgagcaca-3' (SEQ ID NO:10). NM23 P96S ΔC2 was amplified using the following primers: 5'-atcgatcatatggccaactgtgagcgtaccttc-3' (SEQ ID NO:11) and 5'-gtggtgaccggtgatccagttctgagcacagct-3' (SEQ ID NO:12). NM23 P96S ΔC6 was amplified using the following primers: 5'-atcgatcatatggccaactgtgagcgtaccttc-3' (SEQ ID NO:13) and 5'-gtggtgaccggtagcacagctcgtgtaatctacca-3' (SEQ ID NO:14). The resulting fragments were purified, digested (NdeI, AgeI) and cloned between NdeI and AgeI restriction sites of the expression vector pET21b. The pET21b was previously modified by replacing the XhoI restriction by AgeI using an overlap PCR method. Optimal dimer formation was observed when NM23-P96S was cloned between NdeI and XhoI. Optimal dimer formation for all deletion mutants was observed when cloned between NdeI and AgeI. Figure 14 shows non-reducing SDS-PAGE characterization and corresponding FPLC traces for these expressed proteins, wherein the proteins were not refolded or FPLC purified. Figure 15 shows their ability to support pluripotent stem cell growth.

### Example 3a. Protein expression and optional refolding/purification.

LB broth (Luria-Bertani broth) was inoculated with 1/10 of an overnight culture and cultured at 37°C until OD600 reached ~0.5. At this point, recombinant protein expression was induced with 0.4mM Isopropyl-β-D-thio-galactoside (IPTG, Gold Biotechnology) and culture was stopped after 5h. After harvesting the cells by centrifugation (6000 rpm for 10 min at 4°C), cell pellet was resuspended with running buffer: PBS pH7.4, 360 mM NaCl and 80 mM imidazole. Then lysozyme (1 mg/mL, Sigma), MgCl₂ (0.5mM) and DNAse (0.5 ug/mL, Sigma) was added. Cell suspension was incubated on a rotating platform (275 rpm) for 30 min at 37°C and sonicated on ice for 5 min. Insoluble cell debris was removed by centrifugation (20000 rpm for 30 min at 4°c). The cleared lysate was then applied to a Ni-NTA column (Qiagen) equilibrated with the running buffer. The column was washed (8CV) before eluting the protein off the column with the running buffer (6CV) supplemented with 420 mM imidazole.

### Example 3b. Optional protein denaturation for subsequent refolding.

For protein denaturation, the elution fractions were pooled and denatured by adding 1vol of 100mM Tris pH 8.0 + 8M urea, the solution was concentrated by half and another vol of 100mM Tris pH 8.0 + 8M urea was added. This cycle was repeated until final urea concentration was ~7M. The protein was then refolded by dialysis.

### Refolding protocol.

Denatured protein was dialysed overnight against 100mM Tris pH 8.0, 4M urea, 0.2mM imidazole, 0.4M L-arginine, 1mM EDTA and 5% glycerol; then dialysed for 24h against 100mM Tris pH 8.0, 2M urea, 0.2mM imidazole, 0.4M L-arginine, 1mM EDTA and 5% glycerol; next, the protein was dialysed for 24h against 100mM Tris pH 8.0, 1M urea, 0.2mM imidazole, 0.4M L-arginine, 1mM EDTA and 5% glycerol; then dialysed for 8h against 100mM Tris pH 8.0, 0.2mM imidazole, 0.4M L-arginine, 1mM EDTA and 5% glycerol; then, the protein was dialysed overnight against 25mM Tris pH 8.0, 0.2mM imidazole, 0.1M L-arginine, 1mM EDTA and 5% glycerol; dialysed 3x3h against PBS pH 7.4, 0.2mM imidazole, 1mM EDTA and 5% glycerol; dialysed overnight against PBS pH 7.4, 0.2mM imidazole, 1mM EDTA and 5% glycerol; finally the refolded protein was centrifuged (18,500 rpm) 30 min at 4°C and supernatant was collected.

### Optional FPLC Purification

Specific multimers can be further purified from a mixed pool using size exclusion chromatography, also called FPLC. The dimer was further purified by size exclusion chromatography (Superdex 200) "FPLC". FPLC fractions that were essentially 100% dimer were collected and pooled and aliquoted and stored at -80°C and are referred to herein as NM23-S120G-RS or S120G-RS. The fractions containing the dimer were pooled,

### Example 4. FPLC characterization of NM23 wild type compared to three different preparations of recombinant NM23-S120G mutant

Typically, 500uL of each sample to be characterized were loaded onto a Superdex 200 10/300 GL column. The molecular weight of each species peak is determined by comparison to an FPLC trace made by a molecular weight standard that is injected before characterizing the sample proteins. Figure 2 shows the overlay of the FPLC traces that characterize the multimer composition of NM23-WT compared to NM23-S120G purified from the soluble fraction of a first preparation of the expressed protein (labeled here as NM23-_{S120G}-hexamer), the soluble fraction of a second preparation (labeled here as NM23-_{S120G}-mixed), or a preparation of S120G that was denatured and refolded (labeled here as NM23-_{S120G}-dimer). 500 ul of each sample was loaded onto a Superdex 200 10/300 GL. NM23-WT was at 0.17 mg/ml, NM23-_{S120G}-hexamer was loaded at 0.19 mg/ml, NM23-_{S120G}-mixed was loaded at 0.10 mg/ml and NM23-_{S120G}-dimer was loaded at 0.15 mg/ml. NM23-WT and NM23-_{S120G}-hexamer have their major peak at 96 kDa, which corresponds to the molecular weight of an NM23 hexamer; NM23-_{S120G}-mixed and NM23-_{S120G}-dimer both have their major peak at 29 KDa, which corresponds to the dimer, however, the relative proportion of dimer in the refolded preparation, NM23-_{S120G}-dimer, is far greater than the fraction isolated from the soluble portion.

### Example 5. Surface Plasmon Resonance (SPR) to test the ability of different NM23 multimers to bind to a MUC1* extra cellular domain peptide (PSMGFR) that was immobilized on an SPR chip.

Three different preparations of recombinant NM23-S120G plus NM23-WT (wild type) were tested for their ability to bind to a MUC1* extra cellular domain peptide (PSMGFR sequence) using techniques of surface plasmon resonance. The different preparations were first analyzed by FPLC to characterize them according to which multimers they formed. FPLC analysis of the NM23 species that were tested is shown in Figure 2 and described above in Example 4. The samples were also analyzed by gel electrophoresis on a non-reducing gel that does not disrupt disulfide binds, see Figure 3a. NM23-S120G dimers show up on a non-reducing gel at approximately 40 KDa. Conversely, monomers, tetramers and hexamers run with an apparent molecular weight of about 20KDa, presumably because the higher order multimers do not depend on disulfide formation. Note that the same preparation that ran at 96KDa by FPLC (hexamer), in Figure 2, ran at 20 KDa on the non-reducing gel.

Surface Plasmon Resonance (SPR) experiments were carried out using a Biacore 3000 instrument. Bare gold Biacore chips were coated with self-assembled monolayers (SAMs) according to methods of Bamdad, C. The use of variable density self-assembled monolayers to probe the structure of a target molecule. Biophys J. 1998 Oct;75(4):1989-96. NTA-Ni-tri-ethylene glycol SAMs were formed to present 3.8% NTA-Ni, which binds to and captures histidine-tagged proteins. Histidine tagged PSMGFR peptide (MUC1*_{ecd} peptide) was flowed over the chip surface and immobilized to saturation. Next, each of four different preparations of NM23-S120G were injected over a stable peptide surface.

The overlay of Surface Plasmon Resonance (SPR) measurements of Figure 3b show vast differences in the ability of the four different NM23 preparations to bind to a MUC1* extra cellular domain peptide (PSMGFR) attached to the SPR chip surface. Results show that the amount of binding of NM23 to its cognate receptor, MUC1*, is a function of how much dimer is present in the sample. SPR measures protein mass at the chip-solution interface, so if the hexamer bound to the MUC1* peptide surface, it would yield an SPR signal 3-times greater than if a dimer bound. That means that the amount of NM23-WT or NM23-S120G-hexamer that bound to the MUC1* peptide was about 12-times less than the amount of NM23-S120G-dimer that bound. In control experiments, an irrelevant peptide was immobilized on the same chip and a minimal amount of background binding resulted, which was subtracted from the measurement shown. However, the amount of binding generated by the NM23-WT and NM23-S120G-hexamer (both comprised almost exclusively of hexamer) ~100RUs is often considered to be within the noise of the system.

A similar SPR experiment was performed and results are shown in Figure 5. The "NM23-S120G-mixed" preparation that was shown by FPLC to be comprised of 60% dimer (see Figure 2) was tested along with NM23-WT comprised of essentially all hexamer (Figure 2) to determine their ability to bind to the MUC1*_{ecd} peptide (PSMGFR sequence) using Surface Plasmon Resonance (SPR). In this experiment, each protein, at five different concentrations, was separately flowed over a chip that was coated with the PSMGFR peptide. The overlay of SPR traces shown in Figure 5 shows that roughly 8-times more "NM23-S120G-mixed" protein bound to a MUC1* extra cellular domain peptide surface than NM23-WT. Because the wild type protein is a hexamer, the number of RUs must be divided by 3 to compare to the amount of dimer that bound. Although both wild type and S120G-dimer show concentration dependence in binding, the amount of wild type hexamer that bound is so small that it may still be within the noise range of the system. Taken together, these SPR experiments indicate that the hexamer form of NM23 does not bind to the MUC1* receptor.

### Example 6. Nanoparticle experiment is performed to further test the ability of NM23 dimers versus NM23 hexamers to bind to the MUC1*_{ecd} peptide (PSMGFR).

NTA-Ni SAMs were formed on gold nanoparticles according to the methods of Thompson et al, dx.doi.org/10.1021/am200459a | ACS Appl. Mater. Interfaces 2011, 3, 2979-2987. In this method, gold nanoparticles are coated with self-assembled monolayers (SAMs) that have NTA-Ni-thiols incorporated into the SAM. The NTA-Ni moiety captures histidine-tagged proteins. If proteins immobilized on the nanoparticles recognize each other and draw the attached nanoparticles close together, an intrinsic property of the nano gold causes the solution to turn from the characteristic pink to blue. The same thing happens if a dimeric protein added in solution binds to particle-immobilized proteins. NM23-S120G-dimer, NM23-WT, or NM23-S120G-hexamer were separately added to nanoparticles bearing MUC1*_{ecd} peptides (PSMGFR).

The photograph of Figure 3c shows that only the dimer form of NM23 binds to its cognate receptor MUC1* peptide, making the solution turn from pink to blue/gray. NM23-S120G-RS (refolded and purified) and NM23-P96S-ΔC2 (not refolded) bound to the particle-immobilized MUC1* peptide. Neither the wild type NM23 nor the NM23-S120G mutant, which expressed as virtually all hexamer, bound at all to the MUC1* peptide and the solution remained pink and indistinguishable from the control "no protein", in which no NM23 protein was added. To ensure specificity of the observed interaction, the Fab of anti-MUC1* antibody, which was raised against the PSMGFR peptide, was added to competitively inhibit binding of ligands to the peptide. As can be seen in the figure, the Fab inhibited the interaction of NM23 dimers and MUC1* peptide, showing that it was specific binding.

### Example 7. Functional test of the ability of NM23 dimers versus hexamers to promote pluripotent stem cell growth (reference example)

Three batches of NM23, -WT, S120G-hexamer and S120G-dimer were tested for their ability to promote undifferentiated stem cell growth. Human embryonic stem cells (H9) were cultured in minimal stem cell media (this media described in Example 8 below) that contained 8nM of one of the NM23 preparations. In one of the wells the free MUC1*_{ecd} peptide (PSMGFR) was added to competitively inhibit binding of NM23-S120G-dimers to the MUC1* receptor which is on all pluripotent stem cells. The cells were plated at 200,000 cells per well of a 6-well plate that had been coated with an anti-MUC1* monoclonal antibody (MN-C3) to make the stem cells adhere. Media was changed every 48 hours as is typical. Photographs were taken on Day 4.Olympus IX 71 inverted microscope The results are shown in the photograph of Figure 3(d-g). Differentiating stem cells have a different morphology than pluripotent stem cells and appear as thickened, darkened areas of cells, whereas pluripotent stem cells grow in a single bright layer of cells. As can be seen in the figure, only the dimer preparation of NM23-S120G (d) was able to support the undifferentiated growth of stem cells. Wild type NM23 (f) caused the stem cells to begin differentiating after three days, while NM23-S120G-hexamers had even more differentiation (e). However, the greatest amount of differentiation occurred as the result of competitively inhibiting the interaction between NM23 dimers and the MUC1* receptor (g). In control wells, the H9 stem cells were cultured in the standard 4 ng/mL bFGF plus 50% MEF condition media added to minimal stem cell media, wherein the stem cells were plated over a layer of Matrigel. The NM23-S120G-RS as well as the NM23 variants performed as well or better than these controls.

In another part of this experiment, levels of miR-145, which is the microRNA that signals the stem cell's exit from pluripotency, was measured. That experiment showed that the disruption of the interaction between the NM23 dimer and MUC1* caused a spike in miR-145, which further corroborated the finding that disruption of the interaction between NM23 dimers and MUC1* triggers differentiation and conversely the interaction promotes pluripotency. The proteins used in these experiments to determine their ability to support pluripotent stem cell growth were characterized at the time of the experiment by gel electrophoresis using a non-denaturing native gel. The native gel of Figure 4 shows that NM23-WT is comprised essentially of all hexamer, the NM23-S120G-hexamer prep has a small population of dimer but is comprised mostly of hexamer. The NM23-S120G-dimer prep is comprised mostly of dimer but has a small portion of tetramer.

### Example 8. Minimal Stem Cell Media (also, "Minimal Media")

400 ml DME/F12/GlutaMAX I (Invitrogen #10565-018)
100 ml Knockout Serum Replacement (Invitrogen# 10828-028)
5ml 100x MEM Non-essential Amino Acid Solution (Invitrogen# 11140-050)
0.9 ml (0.1mM) beta-mercaptoethanol (55mM stock, Invitrogen# 21985-023)
2.5 ml PSA (penicillin, streptomycin, amphotericin) MP Biochem (#1674049)

### Example 9. NM23 Variants - Construct generation

### Single chain NM23 constructs

NM23 with or without the mutations such as S120G, P96S, or C-terminal deletions can be engineered to prefer dimer formation by making a construct that links two protein monomers. NM23-S120G or other mutation that makes the protein resist formation of tetramers and hexamers is preferred. Table 2 gives the DNA sequence followed by the encoded amino acid sequence. Figure 6 shows reducing and non-reducing SDS-PAGE characterization of single chain variants that had been refolded. The gels confirm that each runs with the apparent molecular weight of the monomer-linker-monomer. The non-reducing gel shows minor populations of higher order multimers dependent on disulfide formation which was eliminated by addition of DTT, which like the cytoplasm produces a reducing environment.

### Example 9a. NM23 S120G GS2 linker

A (GGGGS)x2 linker was introduced in frame of NM23 S120G (3') by PCR using the following primers:
Forward 5'-atcgatcatatggccaactgtgagcgtacctt-3' (SEQ ID NO:15)
Reverse 5'-atcgatgctagcggatccgccaccgccggatccgccaccgccttcatagatccagttctgagcacagctcg-3' (SEQ ID NO: 16)

The resulting fragment was purified, digested (Ndel, NheI) and cloned between NdeI and NheI restriction sites of the expression vector pET21b.

Another NM23 S120G fragment was amplified by polymerase chain reaction (PCR) using the following primers:
Forward 5'-atcgatgctagcatggccaactgtgagcgtaccttc-3' (SEQ ID NO:17)
Reverse 5'-gtggtgctcgagttcatagatccagttctga-3' (SEQ ID NO:18)

The fragment was then purified, digested (NheI/XhoI) and cloned in frame, between the NheI and XhoI restrictions sites of the previously cloned NM23 S120G containing the (GGGGS)x2 linker. The expressed protein can be purified and/or refolded, for *in vitro* applications, using the optional refolding protocol of Example 3b, with optional addition of 1-5mM DTT. Figure 11 shows FPLC analysis (a), SDS-PAGE characterization of the refolded protein (b) and the ability of the non-refolded, non-purified protein to support pluripotent stem cell growth (c-e). Figure 17 shows the ability of this variant to support pluripotent stem cell growth without having to be refolded or purified. Figure 26 shows photographs of non-reducing SDS-PAGE characterization of this variant, which has not been refolded, which shows that the major population is the dimer and it is essentially devoid of hexamer and other higher order multimers.

### Example 9b. NM23 S120G GS3 linker

A (GGGGS)x3 linker was introduced in frame of NM23 S120G (3') by PCR using the following primers:
Forward 5'-atcgatcatatggccaactgtgagcgtacctt-3' (SEQ ID NO:19)
Reverse 5'-atcgatgctagcggatccgccaccgccggatccgccaccgccggatccgccaccgcttcatagatccagttctgagc acagctcg-3' (SEQ ID NO:20)

The resulting fragment was purified, digested (NdeI, NheI) and cloned between NdeI and NheI restriction sites of the expression vector pET21b.

Another NM23 S120G fragment was amplified by polymerase chain reaction (PCR) using the following primers:
Forward 5'-atcgatgctagcatggccaactgtgagcgtaccttc-3' (SEQ ID NO:21)
Reverse 5'-gtggtgctcgagttcatagatccagttctga-3' (SEQ ID NO:22)

The fragment was then purified, digested (NheI/XhoI) and cloned in frame, between the NheI and XhoI restrictions sites of the previously cloned NM23 S120G containing the (GGGGS)x3 linker. The expressed protein can be purified and/or refolded, for *in vitro* applications, using the optional refolding protocol of Example 3b, with optional addition of 1-5mM DTT.

### Example 9c. NM23-S120G-IgG1h noC (modified hinge region of IgG1 without cysteines)

A modified hinge region of IgG1 without cysteine was introduced in frame of NM23 S120G (3') by 2 successive PCR reactions. First the following couple of primers were used:
Forward 5'-atcgatcatatggccaactgtgagcgtacctt-3' (SEQ ID NO:23)
Reverse IgG 1 # 1 tccggcgccggttttggcggtttagtatgggttttatcttcatagatccagttctgagcacagctcg (SEQ ID NO:24)

The PCR fragments was purified and used as template in a second PCR reaction using the following primers:
Forward 5'-atcgatcatatggccaactgtgagcgtacctt-3' (SEQ ID NO:25)
Reverse IgG 1 #2 atcgatgctagcaccggtaccaggaccacccagcagttccggcgccggttttggcgtttagtatg (SEQ ID NO:26)

The resulting fragment was purified, digested (NdeI, NheI) and cloned between NdeI and NheI restriction sites of the expression vector pET21b.

Another NM23 S120G fragment was amplified by polymerase chain reaction (PCR) using the following primers:
Forward 5'-atcgatgctagcatggccaactgtgagcgtaccttc-3' (SEQ ID NO:27)
Reverse 5'-gtggtgctcgagttcatagatccagttctga-3' (SEQ ID NO:28)

The fragment was then purified, digested (NheI/XhoI) and cloned in frame, between the NheI and XhoI restrictions sites of the previously cloned NM23 S120G containing the modified hinge region linker. The expressed protein can be purified and/or refolded, for in vitro applications, using the optional refolding protocol of Example 3b, with optional addition of 1-5mM DTT. Figures 7 shows purification of the expressed protein over a nickel column. Figure 12 shows FPLC analysis (a), SDS-PAGE characterization of the refolded protein (b) and the ability of the non-refolded, non-purified protein to support pluripotent stem cell growth (c-e). Figure 18 shows the ability of this variant to support pluripotent stem cell growth without having to be refolded or purified. Figure 26 shows photographs of non-reducing SDS-PAGE characterization of this variant, which has not been refolded, which shows that the major population is the dimer and it is essentially devoid of hexamer and other higher order multimers.

### Example 9d. NM23 S120G IgG2a noC (modified hinge region of IgG2a without cysteines)

A modified hinge region of IgG2a without cysteine was introduced in frame of NM23 S120G (3') by 2 successive PCR reactions. First the following couple of primers were used:
Forward 5'-atcgatcatatggccaactgtgagcgtacctt-3' (SEQ ID NO:29)
Reverse IgG2a#1
   tcggaggtttcggaggtttaatagtcggaccaccagtttcatagatccagttctgagcacagctcg (SEQ ID NO:30)

The PCR fragments was purified and used as template in a second PCR reaction using the following primers:
Forward 5'-atcgatcatatggccaactgtgagcgtacctt-3' (SEQ ID NO:31)
Reverse IgG2a#2
   atcgatgctagccggaccacccagcaggttcggagcaggtttcggaggtttcggaggtttaatagtcg (SEQ ID NO:32)

The resulting fragment was purified, digested (NdeI, NheI) and cloned between NdeI and NheI restriction sites of the expression vector pET21b.

Another NM23 S120G fragment was amplified by polymerase chain reaction (PCR) using the following primers:
Forward 5'-atcgatgctagcatggccaactgtgagcgtaccttc-3' (SEQ ID NO:33)
Reverse 5'-gtggtgctcgagttcatagatccagttctga-3' (SEQ ID NO:34)

The fragment was then purified, digested (NheI/XhoI) and cloned in frame, between the NheI and XhoI restrictions sites of the previously cloned NM23 S120G containing the modified hinge region linker. The expressed protein can be purified and/or refolded, for *in vitro* applications, using the optional refolding protocol of Example 3b, with optional addition of 1-5mM DTT. Figures 7 shows purification of the expressed protein over a nickel column. Figure 12 shows FPLC analysis (a), SDS-PAGE characterization of the refolded protein (b) and the ability of the non-refolded, non-purified protein to support pluripotent stem cell growth (c-e).

### Example 9e. NM23 S120G IgG1h/IgG2ah noC (modified hinge region of IgG1 and IgG2a without cysteines)

A modified hinge region of **IgG1** and IgG2a without cysteine was introduced in frame of NM23 S120G (3') by 4 successive PCR reactions. First the following couple of primers were used:
Forward 5'-atcgatcatatggccaactgtgagcgtacctt-3' (SEQ ID NO:35)
Reverse #1 tccggcgccggttttggcggtttagtatgggttttatcttcatagatccagttctgagcacagctcg (SEQ ID NO:36)

The PCR fragment was purified and used as template in a second PCR reaction using the following primers:
Forward 5'-atcgatcatatggccaactgtgagcgtacctt-3' (SEQ ID NO:37)
Reverse #2 tcggaccaccagtaccggtaccaggaccacccagcagttccggcgccggttttggcggtttagtatg (SEQ ID NO:38)

The PCR fragment was purified and used as template in a third PCR reaction using the following primers:
Forward 5'-atcgatcatatggccaactgtgagcgtacctt-3' (SEQ ID NO:39)
Reverse #3 tcggagcaggtttcggaggtttcggaggtttaatagtcggaccaccagtaccggtaccaggaccac (SEQ ID NO:40)

The PCR fragment was purified and used as template in a fourth PCR reaction using the following primers:
Forward 5'-atcgatcatatggccaactgtgagcgtacctt-3' (SEQ ID NO:41)
Reverse #4 atcgatgctagccggaccacccagcaggttcggagcaggtttcggaggttt cggag (SEQ ID NO:42)

The resulting fragment was purified, digested (NdeI, NheI) and cloned between NdeI and NheI restriction sites of the expression vector pET21b.

Another NM23 S120G fragment was amplified by polymerase chain reaction (PCR) using the following primers:
Forward 5'-atcgatgctagcatggccaactgtgagcgtaccttc-3' (SEQ ID NO:43)
Reverse 5'-gtggtgctcgagttcatagatccagttctga-3' (SEQ ID NO:44)

The fragment was then purified, digested (NheI/XhoI) and cloned in frame, between the NheI and XhoI restrictions sites of the previously cloned NM23 S120G containing both IgG1 and IgG2a modified hinge region linker. The expressed protein can be purified and/or refolded, for *in vitro* applications, using the optional refolding protocol of Example 3b, with optional addition of 1-5mM DTT. Figure 13 shows FPLC analysis (a), SDS-PAGE characterization of the refolded protein (b) and the ability of the non-refolded, non-purified protein to support pluripotent stem cell growth (c-e). Figure 18 shows the ability of this variant to support pluripotent stem cell growth without having to be refolded or purified. Figure 26 shows photographs of non-reducing SDS-PAGE characterization of this variant, which has not been refolded, which shows that the major population is the dimer and it is essentially devoid of hexamer and other higher order multimers.

### Example 10. Dimerizing NM23 Chimeras

A fusion protein in which NM23-wt or preferably S120G mutant is genetically fused to a protein that naturally dimerizes is another method of producing an NM23 variant that prefers dimer formation and if the S120G mutation is included the resultant dimers will resist forming the higher order multimers such as tetramers and hexamers. One way to do this is to fuse NM23, or a portion of NM23 that binds to the MUC1* peptide, to the Fc portion of an antibody. The Fc portion of antibodies homo-dimerizes via disulfide bonds between cysteines. This construct consists of the NM23-S120G protein connected to the Fc region of an IgG antibody. Inclusion of a histidine tag enables purification over an NTA-Ni affinity column. Alternatively, the fusion protein can be purified over a protein A or Protein G column. Cleavage with Pepsin cleaves the Fc region and releases the portion just below the Cysteines.

### Example 10a. NM23 S120G-IgG1 Fc (NM23 fused to the Fc region antibody)

NM23 S120G was amplified by polymerase chain reaction (PCR) using the following primers:
Forward 5'-atcgatcatatggccaactgtgagcgtacctt-3' (SEQ ID NO:45)
Reverse 5'-gtggtgctcgagttcatagatccagttctga-3' (SEQ ID NO:46)

The fragment was then purified, digested (NdeI, XhoI) and cloned between NdeI and XhoI restriction sites of the expression vector pET21b.

The Fc region of IgG1 was amplified by PCR using the following primers:
Forward 5'-attgtgctcgagggttgtaagccttgcatatgtacagtcccag-3' (SEQ ID NO:47)
Reverse 5'-gcactactcgagtttaccaggagagtgggagaggctcttctcag-3' (SEQ ID NO:48)

The fragment was then purified, digested (XhoI) and cloned in frame (at the XhoI restriction site) of the previously cloned NM23 S120G. The expressed protein can be purified and/or refolded, for *in vitro* applications. If protein is in inclusion bodies: after harvesting the cells by centrifugation (6000 rpm for 10 min at 4°C), cell pellet was resuspended with running buffer: 100mM NaH₂PO₄, 10mM Tris pH 8.0, 10 mM imidazole and 8M urea. The solution was incubated on a rotating platform (275 rpm) for 30min at 37°C and sonicated on ice for 5 min. Insoluble cell debris was removed by centrifugation (20000 rpm for 30 min at 4°C). The cleared lysate was then applied to a Ni-NTA column (Qiagen) equilibrated with the running buffer. The column was washed (8CV) before eluting the protein off the column with the running buffer (6CV) supplemented with 420 mM imidazole . Before refolding, NTA-Ni elution fractions were pooled and 5mM reduced glutathione (GSSH) and 0.5mM oxidized glutathione (GSSG) was added and incubated over night at 4°C with stirring. Then the protein was refolded using the optional refolding protocol of Example 3b. To avoid protein being sequestered in inclusion bodies, necessitating denaturation and refolding, the following is performed: protein expression can be directed to the periplasm of the expressing cell which has been shown to favor disulfide bond formation and allowing the protein to be folded correctly and soluble. Figures 8-10 show the purification, SDS-PAGE and FPLC characterization of this variant.

### Example 10b. NM23 S120G IgGlh (NM23 fused to the hinge region of IgG1)

The IgG1 hinge region was fused 3' to NM23 S120G by polymerase chain reaction (PCR) using the following primers:
Forward 5'-atcgatcatatggccaactgtgagcgtacctt-3' (SEQ ID NO:49)
Reverse 5' atcgatctcgagaccaacacaaatacacggtttacaaccagaatcacgtggcaccggttcatagatccagttctgagcacagctcg - 3' (SEQ ID NO:50)

The fragment was then purified, digested (NdeI, XhoI) and cloned between NdeI and XhoI restriction sites of the expression vector pET21b. The expressed protein can be purified and/or refolded, for in vitro applications as described in Example 10a.

### Example 10c. NM23 S120G IgG2ah( NM23 fused to the hinge region of IgG2a)

The IgG2a hinge region was fused 3' to NM23 S120G by polymerase chain reaction (PCR) using the following primers:
Forward 5'-atcgatcatatggccaactgtgagcgtacctt-3' (SEQ ID NO:51)
Reverse 5' atcgatctcgagacctggacatttacacggtggacacggtttaatggtcggaccacgcggttcatagatccagttctgagcacagctcg -3' (SEQ ID NO:52)

The fragment was then purified, digested (NdeI, XhoI) and cloned between NdeI and XhoI restriction sites of the expression vector pET21b. The expressed protein can be purified and/or refolded, for in vitro applications as described in Example 10a.

### Example 11. Functional analysis of NM23 Variants

### Example 11a. Ability to form stable dimers

NM23 variants that were generated were first tested for their ability to form dimers. The single chain constructs should migrate through a reducing SDS-PAGE gel with the molecular weight of the monomer-linker-monomer. On a non-reducing gel, the single chain variants migrate with the apparent molecular weight of the dimer, while the higher order multimers generally run with the apparent molecular weight of the monomer. The characteristic tetramers and hexamers do not depend on disulfide bonds to multimerize, while dimers of native NM23, NM23-S120G and NM23-P96S and other variants do depend on disulfide bonds. Native gels and FPLC were also used to determine the multimerization state of the variants. Reducing and non-reducing SDS-PAGE gels showing formation of the dimer are shown in Figures 3, 6, 9, 10-14, 16 and 26. FPLC traces showing dimer formation are shown in Figures 2, 10-14 and 16. Figure 4 shows a Native SDS-PAGE gel.

### Example 11b. Ability to promote pluripotent stem cell growth (reference example)

Either H9 or BGO1v/hOG embryonic stem cells were plated onto either Matrigel coated or anti-MUCl * (MN-C3) antibody coated 6-well cell culture plates at a density of 200,000 cells per well. The cells were cultured in minimal stem cell media (see Example 8) to which was added 8nM NM23-S120G-RS or an NM23 variant of the invention. Media was changed every 24 hours for the BGO1v/hOG cells and every 48 hours for the H9 cells. A Rho kinase inhibitor was added at each media change for the BGO1v/hOG cells but only for the first 48 hours for the H9 cells. Cells were cultured for four days and then photographed under magnification. Figures 11-13 and 15-19 show the results. The cell morphology indicates that the cells are undifferentiated in that they are a single layer devoid of clumping or darkening.

### Example 11c. Growth rate of stem cells cultured in NM23 variants (reference example)

In addition to assessing stem cell morphology as proof that the NM23 variants functioned as the native dimers or the S120G dimers, the growth rate of stem cells cultured in media containing the NM23 variants was compared to the growth rate of identical cells cultured in NM23-S120G "RS" that had been refolded and then purified by FPLC so that the isolated fractions were essentially 100% dimer. In these experiments, 200,000 stem cells all drawn from the same source (human ES - BGO1v/hOG) were cultured in either NM23-S120G RS or one of the NM23 variants shown in Figures 20 and 21. Four days post plating, cells were harvested by trypsinization and cells were counted on a hemocytometer. As can be seen in the graphs, in every case, the variants produced more cells than the isolated dimer population of NM23-S120G ("RS").

### Example 11d. RT-PCR analysis of expression levels of pluripotency genes in stem cells cultured in NM23 variants

As another method of assessing the function of the NM23 dimer preferring variants, quantitative PCR was performed to measure expression levels of the pluripotency genes in stem cells cultured in the NM23 variants. Figure 22 compares expression levels of pluripotency genes Oct4 and Nanog plus MUC1 and NM23 in stem cells that had been cultured in the NM23 variants for at least 4 passages. The graph of Figure 22 shows that, for cells grown in the NM23 variants, the expression levels of these key indicators of pluripotency are the same or better than for cells cultured in NM23-S120G RS, which is a population of pure dimers.

Experimental details: Stem cells grown in different NM23 variants were collected. The cells were pelleted and frozen at -70°C. Total RNA was extracted from the samples using TRIzol^{®} Reagent. Quantification of NANOG, OCT4, MUC1, NM23 and GAPDH in the RNA samples was performed using TaqMan^{®} One Step RT-PCR Master Mix Reagents. The real-time PCR data were analyzed using the comparative Cₜ method. The relative amount of each transcript in each sample was obtained by computing the difference between the target Cₜ and the corresponding GAPDH (ΔCₜ). A second normalization was performed by subtracting the RS sample ΔCₜ from all the others in the data set (ΔΔCₜ).

### Example 11e. Ability of NM23 variants to penetrate cell membrane and translocate to the nucleus

As a yet further measure of the function of engineered NM23 dimer preferring variants, their migration from cell surface to cell nucleus was tracked and compared to that of NM23-S120G RS. It is known that NM23 dimers mediate the growth of MUC1*-positive cancer cells and human pluripotent stem cells, which are all MUC1*-positive. When MUC1*-positive cancer cells are incubated in media that contains NM23 in dimer form, the NM23 dimers bind to the MUC1* receptor, become internalized and within 30-60 minutes are translocated to the nucleus where they likely function as transcription factors. Figure 23 shows confocal images of cancer cells incubated in the presence or absence of either 0, 16nM or 128nM NM23-S120G RS (100% dimer population). Cells were then stained with the nuclear stain DAPI and an anti-NM23 antibody was then added to the cells and a fluorescently labeled secondary antibody. Note that endogenous NM23 is also stained by the antibodies. However, there is only detectable NM23 in the nucleus when it is added exogenously as a dimer. Optimal concentrations for enhancing cell growth and for nuclear localization for NM23-S120G were previously determined to be between 8nM and 64nM. At higher concentrations, each NM23 dimer binds to each MUC1* receptor rather than 1 dimer binding to and dimerizing two MUC1* receptors. (See bell-shaped curve of Figure 1 where bivalent anti-MUC1* antibody in excess inhibits rather than stimulates growth for the same reason.) Figure 23 (b, e) shows NM23 in the nucleus as indicated by the white arrows. Figure 24 shows confocal images of the same experiment except that the NM23 that has been added exogenously is the single chain "dimer" variant NM23-S120G-IgG1h/IgG2ah noC, which is two NM23-S120G monomers linked by a modified IgG1 and IgG2a hinge regions, without cysteines, see Example 9e. As can be seen in Figure 24, the NM23 single chain variant readily translocates to the nucleus (b, e), as indicated by the white arrows. The corresponding graphs that quantify the amount of NM23 in the nucleus for NM23-S120G-RS (Figure 23h) and for the single chain variant, NM23-S120G-IgG1h/IgG2ah noC (Figure 24h) show that the engineered "dimer" translocates to the nucleus better than NM23 monomers that have been isolated as a dimer population.

Experimental details: T47D breast cancer cells were initially plated onto collagen-coated 8-well chambers containing 10% FBS RPMI media for 24 hrs followed by serum-starvation (1% FBS RPMI) for 24 hrs, at 37°C, 5 % CO₂. Subsequently T47D cells were incubated with 16 nM or 128 nM NM23_{S120GRS} or NM23_{IgG1/IgG2a} in 10 % FBS RPMI for 30 minutes. T47D cells were then fixed in 4% paraformaldehyde. Cells were blocked for one hour in PBS + 1% BSA + 5% normal goat serum + 0.01% Triton-x ("blocking buffer") and then incubated in blocking buffer containing primary antibody for one hour at room temperature. Cells were then washed with PBS followed by incubation with the appropriate secondary antibody (Alexa-Fluor, Invitrogen) for one hour at room temperature (kept in the dark). Following washing with PBS, cells were mounted with Prolong Gold + DAPI (Invitrogen) and coverslip. T47D cells were visualized on a Zeiss LSM 510 laser scanning confocal microscope.

### Example 12. Cross species function

NM23 supports proliferation of mouse ES cells with pluripotent colony morphology.

Mouse ES cells (129/S6, EMD Millipore, Billerica, MA) were cultured on inactivated MEF feeder cell layers for two days in mouse ES cell minimal medium (mESC-MM) supplemented with either 1,000 U/mL recombinant mLIF (a, c) (EMD Millipore) or 16 nM NM23-S120G-RS (b, d), and photographed at low magnification under phase-contrast illumination. Size bars indicate 500 microns. In both cases, single cells and colonies consisting of just a few cells on day 1 give rise to larger multicellular oval colonies with bright, defined edges typical of pluripotent mouse ES cells. mESC-MM consists of KnockOut D-MEM basal medium, 15% KnockOut Serum Replacement, 1X GlutaMax I, 1X OptiMEM non-essential amino acids, 0.1 mM B-ME (Life Technologies, Carlsbad, CA), and 1X Penicillin/Streptomycin (Lonza, Allendale, NJ).

Results are shown in Figure 25 and demonstrate that mouse stem cells grow equally well in NM23 (human) as they do in mouse stem cell media with mouse LIF as the growth factor. Therefore, NM23 variants described herein can be used in mouse cell systems and mouse NM23 and NM23 variants can be used in human cell systems.

### Example 13. Generation of constructs that are secreted by the expressing cell

Any of the mutants, deletions and/or single chain or fusion chimeras of the invention, including those described in Examples 2, 9 and 10 can be made to be secreted by the expressing cells for use *in vitro, ex vivo* and/or *in vivo.* Sequences that cause expressed proteins to be secreted are known to those skilled in the art. Particularly, sequences derived from antibodies are added to the N-terminus of the protein or to the 5'end of the gene of interest. In addition to the inclusion of leader sequences, the expression cell type need not be limited to *E. coli* and also includes mammalian cells, mammalian expression cells, yeast, somatic cells, stem cells, iPS cells or cells undergoing induction of pluripotency or induction to a less mature state than the starting cell.

### Example 14. Comparison of recombinant NM23-WT, S120G and variants of the invention without refolding

One of the benefits of NM23 variants of the invention is that they are designed to spontaneously form stable dimers without the necessity of denaturation and refolding which cannot be done in an *in vivo* setting. To demonstrate the advantage of single chain and fusion chimeras that naturally dimerize, NM23-WT, NM23-S120G and variants NM23-S120G-GS2, IgG1h-noC, IgG1h/IgG2ah-noC were expressed and without denaturation or refolding were characterized by non-reducing SDS-PAGE. The gel of Figure 26 shows that only the protein variants migrate as dimer. The wild type NM23 and the NM23-S120G (non refolded) run with the apparent molecular weight of the monomer on a non-reducing gel. As previously discussed herein, the NM23 hexamer runs as a monomer on non-reducing gels because its multimerization does not depend on disulfide bonds but has been shown by FPLC to actually be the hexamer (see Figures 2,3a, and 4 for comparison).

### Example 15. Identification of mutants that prefer dimerization and resist formation of higher order multimers.

Mutations that promote cancer or stem cell growth can be identified by sequencing NM23 from several cancers. The S120G mutant was isolated from a neuroblastoma. Alternatively, one can randomly mutate NM23 encoding DNA then test the resultant proteins to screen for the mutants that promote cancer or stem cell growth. The limiting factor is the amount of time to screen the many possible mutations in a cell-based assay. A convenient method for testing mutants for their ability to form dimers and also resist formation of higher order multimers is to test the mutants for their ability to bind to the MUC1* peptide. As we have shown, NM23 tetramers and hexamers do not bind to MUC1* peptide. An assay that simultaneously identifies mutants that form dimers but not tetramers and hexamers is a nanoparticle assay in which the MUC1* peptide is loaded onto gold nanoparticles. In multi-well plates each mutant is added to the peptide-bearing nanoparticles. If the mutant readily forms dimers, the dimeric NM23 binds to the particle-immobilized MUC1* peptides and draws the particles close together which causes the particle solution to change from pink to blue. If the mutants are added at high concentration, the mutants that are still able to form tetramers and hexamers will do so, which will not bind to the peptides and the solution will remain pink. Therefore, mutants that prefer dimer formation and do not form the inactive hexamers are readily identified because they will turn the nanoparticle solution blue even when added at high concentrations of for example more than 500nM.

### Example 16. (reference example)

Human BGO1v/hOG embryonic stem cells were plated on to Vita plates coated with 12.5ug per well of a monoclonal anti-MUC1* antibody (MN-C3) at a cell density of 100,000 cells per well of a 6-well cell culture plate. The cells were cultured for 2 days in NM23 variants that had been refolded according to the optional refolding protocol of Example 3. The NM23 variants used at 8nM in minimal stem cell media (see Example 8) were NM23-S120G-RS (a,e), NM23-S120G-GS2 ("R" in figure denotes refolded) (b,f), NM23-S120G-IgG1h noC (c,g), and NM23-S120G-IgG1h/IgG2ah noC (d,h). As can be seen from the cell morphology, all the stem cells grew as pluripotent stem cells, devoid of differentiating, fibroblast like cells and also devoid of thickening and darkening which are also indicative of differentiation.

## Claims

1. A method for:
(a) proliferating cells *in vitro* or *ex vivo;*
(b) inducing pluripotency in a somatic cell *in vitro* or *ex vivo;* or
(c) promoting the growth of stem and progenitor cells, *in vitro* or *ex vivo,*
comprising transfecting or transducing the cells with an expression vector comprising an isolated nucleic acid sequence encoding a recombinantly made protein construct that preferentially forms a specific multimer,
wherein the specific multimer is formed by recombinantly connecting a protein monomer to a second monomer,
wherein the protein monomer is an NM23 monomer,
wherein the multimerization state is a dimer, comprising two monomers or fragments of the monomers,
wherein the two monomers or fragments of the monomers are linked together through a linker peptide, thus forming a monomer-linker-monomer construct, and
wherein the NM23 is Hi1 or H2.

2. A method for proliferating cells as claimed in claim 1(a), wherein the cell is a stem or progenitor cell.

3. A method according to claim 1(c), wherein a nucleic acid sequence of one or more of the genes in the vector native to the cell have been modified.

4. The method according to claim 1, wherein the multimerization state of the protein is its biologically active state.

5. The method according to claim 1, wherein the dimer is a homodimer or a heterodimer.

6. The method according to claim 1, wherein the protein is mammalian protein, preferably, wherein the protein is:
(a) human protein; or
(b) mouse protein.

7. The method according to any one of claims 1 or 4-6, wherein the linker includes GS, GS2, GS3, IgG1 hinge region, or IgG2a hinge region or combination thereof.

8. The method according to claim 1, wherein the monomers are covalently linked together by a disulfide bond.

9. The method of any one of claims 1 or 4-8, wherein the linker is IgG1 hinge or IgG2a hinge.

10. The method according to claim 1, wherein cysteines are inserted into the protein to promote multimer formation via disulfide bonds.

11. The method according to any one of claims 1 or 4-10, wherein the protein construct comprises an amino acid sequence that facilitates:
(a) entrance into a cell or into the nucleus of the cell; or
(b) secretion of the protein construct from its expressing host cell.

12. The method according to claim 1, wherein the protein construct is selected from the following proteins constructs as defined in table 3:
NM23 S120G GS2,
NM23 P96S GS2,
NM23 P96S/S120G GS2,
NM23 P96SΔC1 GS2,
NM23 P96SΔC2 GS2,
NM23 P96SΔC6 GS2,
NM23 P96SΔC1/S120G GS2,
NM23 P96SΔC2 /S120G GS2,
NM23 P96SΔC6 /S120G GS2,
NM23 S120G GS3,
NM23 P96S GS3,
NM23 P96S/S120G GS3,
NM23 P96SΔC1 GS3,
NM23 P96SΔC2 GS3,
NM23 P96SΔC6 GS3,
NM23 P96SAC1/S120G GS3,
NM23 P96SΔC2 /S120G GS3,
NM23 P96SΔC6 /S120G GS3,
NM23 S120G IgGih noC,
NM23 P96S IgGih noC,
NM23 P96S/S120G IgGih noC,
NM23 P96SΔC1 IgGih noC,
NM23 P96SΔC2 IgGih noC,
NM23 P96SΔC6 IgGih noC,
NM23 P96SΔC1/S120G IgGih noC,
NM23 P96SΔC2 /S120G IgGih noC,
NM23 P96SΔC6 /S120G IgGih noC,
NM23 S120G IgG2ah noC,
NM23 P96S IgG2ah noC,
NM23 P96S/S120G IgG2ah noC,
NM23 P96SΔC1 IgG2ah noC,
NM23 P96SΔC2 IgG2ah noC,
NM23 P96SΔC6 IgG2ah noC,
NM23 P96SΔC1/S120G IgG2ah noC,
NM23 P96SΔC2 /S120G IgG2ah noC,
NM23 P96SΔC6 /S120G IgG2ah noC,
NM23 S120G IgG1h/IgG2ah noC,
NM23 P96S IgG1h/IgG2ah noC,
NM23 P96S/S120G IgG1h/IgG2ah noC,
NM23 P96SΔC1 IgG1h/IgG2ah noC,
NM23 P96SΔC2 IgG1h/IgG2ah noC,
NM23 P96SΔC6 IgG1h/IgG2ah noC,
NM23 P96SΔC1/S120G IgG1h/IgG2ah noC,
NM23 P96SΔC2 /S120G IgG2ah noC, or
NM23 P96SΔC6 /S120G IgG2ah noC.

13. The method according to claim 1, wherein the nucleic acid:
(a) further comprises a sequence that encodes amino acid sequence that facilitates entrance into a cell or into the nucleus of the cell;
(b) further comprises a sequence that encodes amino acid sequence that facilitates secretion of the protein from its expressing host cell; or
(c) comprises nucleic acid encoding NM23 or a mutant thereof that favors dimer formation.

14. The method according to claim 1, wherein the vector:
(a) is a plasmid, or
(b) is a virus.

## Patentansprüche

1. Verfahren zur:
(a) Proliferation von Zellen in vitro oder ex vivo;
(b) Induktion von Pluripotenz in einer somatischen Zelle in vitro oder ex vivo; oder
(c) Förderung des Wachstums von Stamm- und Vorläuferzellen in vitro oder ex vivo,
umfassend das Transfizieren oder Transduzieren der Zellen mit einem Expressionsvektor, der eine isolierte Nucleinsäuresequenz umfasst, die für ein rekombinant hergestelltes Proteinkonstrukt kodiert, das bevorzugt ein spezifisches Multimer bildet,
wobei das spezifische Multimer durch rekombinantes Verbinden eines Proteinmonomers mit einem zweiten Monomer gebildet wird,
wobei das Proteinmonomer ein NM23-Monomer ist,
wobei der Multimerisierungszustand ein Dimer ist, das zwei Monomere oder Fragmente der Monomere umfasst,
wobei die beiden Monomere oder Fragmente der Monomere durch ein Linkerpeptid miteinander verbunden sind, wodurch ein Monomer-Linker-Monomer-Konstrukt gebildet wird, und
wobei das NM23 H1 oder H2 ist.

2. Verfahren zur Proliferation von Zellen nach Anspruch 1(a), wobei die Zelle eine Stamm- oder Vorläuferzelle ist.

3. Verfahren nach Anspruch 1(c), wobei eine Nucleinsäuresequenz eines oder mehrerer der Gene im Vektor, die nativ zu der Zelle ist, modifiziert wurde.

4. Verfahren nach Anspruch 1, wobei der Multimerisierungszustand des Proteins ein biologisch aktiver Zustand ist.

5. Verfahren nach Anspruch 1, wobei das Dimer ein Homodimer oder ein Heterodimer ist.

6. Verfahren nach Anspruch 1, wobei das Protein ein Säugetierprotein ist, wobei das Protein vorzugsweise Folgendes ist:
(a) ein menschliches Protein oder
(b) ein Mausprotein.

7. Verfahren nach einem der Ansprüche 1 oder 4 bis 6, wobei der Linker GS, GS2, GS3, eine IgG1-Gelenkregion oder eine IgG2a-Gelenkregion oder eine Kombination davon umfasst.

8. Verfahren nach Anspruch 1, wobei die Monomere durch eine Disulfidbindung kovalent miteinander verbunden sind.

9. Verfahren nach einem der Ansprüche 1 oder 4 bis 8, wobei der Linker ein IgG1-Gelenk oder IgG2a-Gelenk ist.

10. Verfahren nach Anspruch 1, wobei Cysteine in das Protein eingeführt werden, um eine Multimerbildung mittels Disulfidbindungen zu fördern.

11. Verfahren nach einem der Ansprüche 1 oder 4 bis 10, wobei das Proteinkonstrukt eine Aminosäuresequenz umfasst, die Folgendes begünstigt:
(a) den Eintritt in eine Zelle oder in den Kern der Zelle; oder
(b) die Sekretion des Proteinkonstrukts aus ihrer exprimierenden Wirtszelle.

12. Verfahren nach Anspruch 1, wobei das Proteinkonstrukt aus folgenden Proteinkonstrukten wie in Tabelle 3 definiert ausgewählt ist:
NM23 S120G GS2,
NM23 P96S GS2,
NM23 P96S/S120G GS2,
NM23 P96SΔC1 GS2,
NM23 P96SΔC2 GS2,
NM23 P96SΔC6 GS2,
NM23 P96SΔC1 /Sr20G GS2,
NM23 P96SΔC2 /S120G GS2,
NM23 P96SΔC6 /S120G GS2,
NM23 S120G GS3,
NM23 P96S GS3,
NM23 P96S/S120G GS3,
NM23 P96SΔC1 GS3,
NM23 P96SΔC2 GS3,
NM23 P96SΔC6 GS3,
NM23 P96SAC1/S120G GS3,
NM23 P96SΔC2 /S120G GS3,
NM23 P96SΔC6 /S120G GS3,
NM23 S120G IgG1h noC,
NM23 P96S IgGih noC,
NM23 P96S/S120G IgG1h noC,
NM23 P96SΔC1 IgGih noC,
NM23 P96SΔC2 IgG1h noC,
NM23 P96SΔC6 IgG1h noC,
NM23 P96SΔC1 /S120G IgGih noC,
NM23 P96SΔC2 /S120G IgG1h noC,
NM23 P96SΔC6 /S120G IgG1h noC,
NM23 S120G IgG2ah noC,
NM23 P96S IgG2ah noC,
NM23 P96S/S120G Ig2ah noC,
NM23 P96SΔC1 IgG2ah noC,
NM23 P96SΔC2 IgG2ah noC,
NM23 P96SΔC6 IgG2ah noC,
NM23 P96SΔC1 /S120G IgGaah noC,
NM23 P96SΔC2 /S120G IgGaah noC,
NM23 P96SΔC6 /S120G IgG2ah noC,
NM23 S120G IgG1h/IgG2ah noC,
NM23 P96S IgG1h/IgG2ah noC,
NM23 P96S/S120G IgG1h/IgG2ah noC,
NM23 P96SΔC1 IgG1h/IgGaah noC,
NM23 P96SΔC2 IgG1h/IgG2ah noC,
NM23 P96SΔC6 IgG1h/IgG2ah noC,
NM23 P96SΔC1 /S120G IgG1h/IgG2ah noC,
NM23 P96SΔC2 /S120G IgG2ah noC und
NM23 P96SΔC6 /S120G IgG2ah noC.

13. Verfahren nach Anspruch 1, wobei die Nucleinsäure:
(a) weiters eine Sequenz umfasst, die für eine Aminosäuresequenz kodiert, die den Eintritt in eine Zelle oder in den Kern der Zelle begünstigt;
(b) weiters eine Sequenz umfasst, die für eine Aminosäuresequenz kodiert, welche die Sekretion des Proteins aus ihrer exprimierenden Wirtszelle begünstigt; oder
(c) eine Nucleinsäure umfasst, die für NM23 oder eine Mutante davon kodiert, welche eine Dimerbildung begünstigt.

14. Verfahren nach Anspruch 1, wobei der Vektor:
(a) ein Plasmid ist oder
(b) ein Virus ist.

## Revendications

1. Procédé pour :
(a) faire proliférer des cellules *in vitro* ou ex *vivo* ;
(b) induire une pluripotence dans une cellule somatique *in vitro* ou ex *vivo* ; ou
(c) promouvoir la croissance des cellules souches et progénitrices, *in vitro* ou ex *vivo,*
comprenant une transfection ou une transduction des cellules avec un vecteur d'expression comprenant une séquence d'acide nucléique isolée codant pour une construction protéique fabriquée par recombinaison qui forme de préférence un multimère spécifique,
dans lequel le multimère spécifique est formé en reliant par recombinaison un monomère protéique à un second monomère,
dans lequel le monomère protéique est un monomère NM23,
dans lequel l'état de multimérisation est un dimère, comprenant deux monomères ou fragments des monomères,
dans lequel les deux monomères ou fragments des monomères sont liés entre eux par l'intermédiaire d'un peptide lieur, en formant ainsi une construction monomère-lieur-monomère, et
dans lequel le NM23 est H1 ou H2.

2. Procédé de prolifération de cellules selon la revendication 1(a), dans lequel la cellule est une cellule souche ou progénitrice.

3. Procédé selon la revendication 1(c), dans lequel une séquence d'acide nucléique d'un ou plusieurs des gènes dans le vecteur native de la cellule a été modifiée.

4. Procédé selon la revendication 1, dans lequel l'état de multimérisation de la protéine est son état biologiquement actif.

5. Procédé selon la revendication 1, dans lequel le dimère est un homodimère ou un hétérodimère.

6. Procédé selon la revendication 1, dans lequel la protéine est une protéine de mammifère, de préférence dans lequel la protéine est :
(a) une protéine humaine ; ou
(b) une protéine de souris.

7. Procédé selon l'une quelconque des revendications 1 ou 4 à 6, dans lequel le lieur inclut une région de charnière GS, GS2, GS3, IgG1 ou IgG2a, ou une combinaison de celles-ci.

8. Procédé selon la revendication 1, dans lequel les monomères sont liés ensemble de manière covalente par une liaison disulfure.

9. Procédé selon l'une quelconque des revendications 1 ou 4 à 8, dans lequel le lieur est une charnière IgG1 ou une charnière IgG2a.

10. Procédé selon la revendication 1, dans lequel des cystéines sont insérées dans la protéine pour favoriser la formation de multimères via des liaisons disulfure.

11. Procédé selon l'une quelconque des revendications 1 ou 4 à 10, dans lequel la construction protéique comprend une séquence d'acides aminés qui facilite :
(a) une entrée dans une cellule ou dans le noyau de la cellule ; ou
(b) une sécrétion de la construction protéique à partir de sa cellule hôte d'expression.

12. Procédé selon la revendication 1, dans lequel la construction protéique est choisie parmi les constructions protéiques suivantes telles que définies dans le tableau 3 :
NM23 S120G GS2,
NM23 P96S GS2,
NM23 P96S/S120G GS2,
NM23 P96SΔC1 GS2,
NM23 P96SΔC2 GS2,
NM23 P96SΔC6 GS2,
NM23 P96SAC1/S120G GS2,
NM23 P96SΔC2/S120G GS2,
NM23 P96SΔC6/S120G GS2,
NM23 S120G GS3,
NM23 P96S GS3,
NM23 P96S/S120G GS3,
NM23 P96SΔC1 GS3,
NM23 P96SΔC2 GS3,
NM23 P96SΔC6 GS3,
NM23 P96SAC1/S120G GS3,
NM23 P96SΔC2/S120G GS3,
NM23 P96SΔC6/S120G GS3,
NM23 S120G IgGlh n0C,
NM23 P96S IgGlh n0C,
NM23 P96S/S120G IgGlh noC,
NM23 P96SΔC1 IgGlh noC,
NM23 P96SΔC2 IgGlh noC,
NM23 P96SΔC6 IgGlh noC,
NM23 P96SAC1/S120G IgGlh noC,
NM23 P96SΔC2/S120G IgGlh noC,
NM23 P96SΔC6/S120G IgGlh noC,
NM23 S120G IgG2ah noC,
NM23 P96S IgG2ah noC,
NM23 P96S/S120G IgG2ah noC,
NM23 P96SΔC1 IgG2ah noC,
NM23 P96SΔC2 IgG2ah noC,
NM23 P96SΔC6 IgG2ah noC,
NM23 P96SΔC1/S20G IgG2ah noC,
NM23 P96SΔC2/S120G IgG2ah noC,
NM23 P96SΔC6/S120G IgG2ah noC,
NM23 S120G IgG1h/IgG2ah noC,
NM23 P96S IgG1h/IgG2ah noC,
NM23 P96S/S120G IgG1h/IgG2ah noC,
NM23 P96SΔC1 IgG1h/IgG2ah noC,
NM23 P96SΔC2 IgG1h/IgG2ah noC,
NM23 P96SΔC6 IgGih/IgG2ah noC,
NM23 P96SΔCi/S120G IgG1h/IgG2ah noC,
NM23 P96SΔC2/S120G IgG2ah noC, ou
NM23 P96SΔC6/S120G IgG2ah noC.

13. Procédé selon la revendication 1, dans lequel l'acide nucléique :
(a) comprend en outre une séquence qui code pour une séquence d'acides aminés qui facilite une entrée dans une cellule ou dans le noyau de la cellule ;
(b) comprend en outre une séquence qui code pour une séquence d'acides aminés qui facilite une sécrétion de la protéine à partir de sa cellule hôte d'expression ; ou
(c) comprend un acide nucléique codant pour NM23 ou un mutant de celui-ci qui favorise la formation de dimères.

14. Procédé selon la revendication 1, dans lequel le vecteur :
(a) est un plasmide, ou
(b) est un virus.
